(19) Europäisches Patentamt
European Patent Office
Office européen des brevets

(11) EP 3 696 269 A1

(12) **EUROPEAN PATENT APPLICATION**
published in accordance with Art. 153(4) EPC

(43) Date of publication:
**19.08.2020 Bulletin 2020/34**

(21) Application number: **18865423.0**

(22) Date of filing: **12.10.2018**

(51) Int Cl.:
***C12N 15/113*** *(2010.01)* ***A61K 31/7088*** *(2006.01)*
***A61K 48/00*** *(2006.01)*

(86) International application number:
**PCT/JP2018/038174**

(87) International publication number:
**WO 2019/074110 (18.04.2019 Gazette 2019/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.10.2017 JP 2017199945**

(71) Applicant: **Bonac Corporation**
**Kurume-shi, Fukuoka 839-0861 (JP)**

(72) Inventors:
- **OHGI, Tadaaki**
  **Kurume-shi**
  **Fukuoka 839-0861 (JP)**
- **EMURA, Chisato**
  **Kurume-shi**
  **Fukuoka 839-0861 (JP)**
- **KINOSHITA, Takashi**
  **Kurume-shi**
  **Fukuoka 839-0861 (JP)**

(74) Representative: **J A Kemp LLP**
**14 South Square**
**Gray's Inn**
**London WC1R 5JJ (GB)**

# (54) SINGLE-STRANDED NUCLEIC ACID MOLECULE, AND PRODUCTION METHOD THEREFOR

(57) The present invention provides a new single-stranded nucleic acid molecule and a production method thereof.

The present invention relates to a production method of single-stranded nucleic acid molecule represented by the general formula (I):

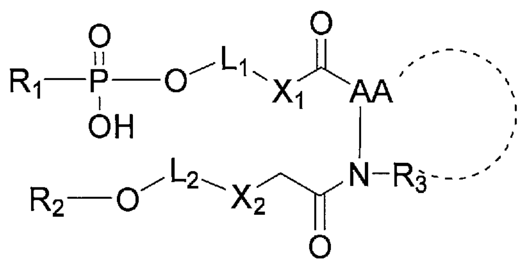

(I)

wherein each symbol is as described in the description.



Printed by Jouve, 75001 PARIS (FR)

SEQUENCE LISTING

<110>  BONAC CORPORATION

<120>  A single-stranded nucleic acid molecule and a production method thereof

<130>  092801

<150>  JP 2017-199945
<151>  2017-10-13

<160>  9

<170>  PatentIn version 3.5

<210>  1
<211>  19
<212>  RNA
<213>  Artificial Sequence

<220>
<223>  sequence targeting TGF-B1

<400>  1
uaugcugugu guacucugc                                                    19

<210>  2
<211>  21
<212>  RNA
<213>  Artificial Sequence

<220>
<223>  sequence targeting prorenin receptor

<400>  2
uuauaugcaa gguuauaggg a                                                 21

<210>  3
<211>  23
<212>  RNA
<213>  Artificial Sequence

<220>
<223>  sequence targeting miR-29b

<400>  3
uagcaccauu ugaaaucagu guu                                               23

<210>  4
<211>  25
<212>  RNA
<213>  Artificial Sequence

<220>
<223>  sequence targeting TGF-B1

<400>  4
gguauaugcu gugugua cuc ugcuu                                            25

```
<210>  5
<211>  23
<212>  RNA
<213>  Homo sapiens

<400>  5
gcagaguaca cacagcauau acc                                    23


<210>  6
<211>  22
<212>  DNA
<213>  Artificial Sequence

<220>
<223>  PCR Primer

<400>  6
ttgtgcggca gtggttgagc cg                                     22


<210>  7
<211>  24
<212>  DNA
<213>  Artificial Sequence

<220>
<223>  PCR Primer

<400>  7
gaagcaggaa aggccggttc atgc                                   24


<210>  8
<211>  23
<212>  DNA
<213>  Artificial Sequence

<220>
<223>  PCR Primer

<400>  8
gccacggctg cttccagctc ctc                                    23


<210>  9
<211>  25
<212>  DNA
<213>  Artificial Sequence

<220>
<223>  PCR Primer

<400>  9
aggtctttgc ggatgtccac gtcac                                  25
```

## Description

### Technical Field

The present invention relates to a single-stranded nucleic acid molecule and a production method thereof.

### Background Art

As single-stranded nucleic acid molecules, for example, those described in Patent Documents 1 and 2 are known.

### Document List

### Patent Document

Patent Document 1: WO 2012/005368
Patent Document 2: WO 2012/017919

### Summary of the Invention

### Problems to be Solved by the Invention

General long-chain oligomer synthesis has technical and productive problems, such as reduction in reaction yield, difficulty in removal of by-product, restrictive use of amidite monomers, higher costs and the like. The development of a new, easy, general-purpose and practicable production method which can solve these problems are desired.

### Means of Solving the Problems

The present inventors have conducted intensive studies in an attempt to solve the above-mentioned problems and found that a method of reacting a nucleic acid represented by the formula (II) with a compound represented by the formula (IV), and then reacting the resulting compound with a nucleic acid represented by the formula (III) can markedly avoid contamination of short-chain oligomers (e.g., N-1, 2, 3 and the like), which often becomes problems in long-chain oligomer synthesis, and the reaction product obtained thereby can be easyly purified, which resulted in the completion of the present invention.

Accordingly, the present invention provides the following.

[1] A method of producing a single-stranded nucleic acid molecule represented by the following general formula (I):

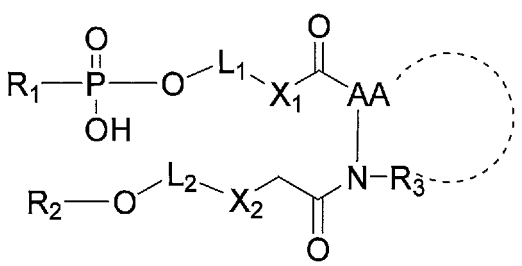

(I)

wherein

$R_1$ is a region comprising an oligonucleotide or a polynucleotide, the 3'-terminal nucleotide residue of which is bonded to -P(=O)(OH)-O-;
$R_2$ is a region comprising an oligonucleotide or a polynucleotide, the 5'-terminal nucleotide residue of which is bonded to -O-;
$X_1$ and $X_2$ are each independently NH, S or O;
$L_1$ and $L_2$ are each independently a $C_{1-30}$ alkylene chain, or-$(CH_2)_1$-(O-$(CH_2)_m$-$)_n$- wherein 1 is an integer of 1

to 3, m is an integer of 1 to 3, and n is an integer of 1 to 10; -C(=O)-AA-$NR_3$- is an amino acid residue; and $R_3$ is a hydrogen atom or a $C_{1-6}$ alkyl group, or $R_3$ is bonded to AA to form a nitrogen-containing heterocycle (hereinafter sometimes to be referred to as single-stranded nucleic acid molecule (I)),
which comprises

(Step 1) a step of reacting a nucleic acid represented by the following general formula (II):

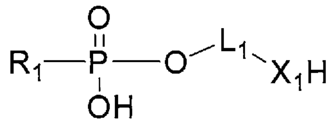
(II)

wherein each symbol is as defined above (hereinafter sometimes to be referred to as nucleic acid (II)) with a compound represented by the following general formula (IV) :

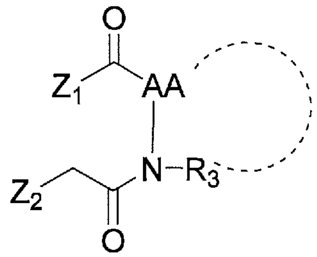
(IV)

wherein

$Z_1$ is a succinimidyloxy group, a benzotriazolyloxy group, a pentafluorophenoxy group or a halogen atom;
$Z_2$ is a halogen atom; and
the other symbols are as defined above (hereinafter sometimes to be referred to as compound (IV)) to obtain a nucleic acid represented by the following general formula (V):

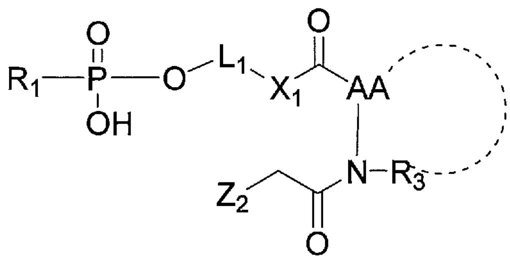
(V)

wherein each symbol is as defined above (hereinafter sometimes to be referred to as nucleic acid (V)); and
(Step 2) a step of reacting the nucleic acid represented by the general formula (V) with a nucleic acid represented by the following general formula (III):

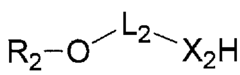
(III)

wherein each symbol is as defined above (hereinafter sometimes to be referred to as nucleic acid (III)) to obtain the single-stranded nucleic acid molecule represented by the general formula (I).

[2] The method of the aforementioned [1], wherein the amino acid residue is a proline residue, a glycine residue, or a lysine residue with the side chain amino group being optionally protected.
[3] The method of the aforementioned [1], wherein $X_2$ is S.
[4] The method of the aforementioned [1], wherein $X_1$ is NH.

[5] The method of the aforementioned [1], wherein $L_1$ is a $C_{2-11}$ alkylene chain.
[6] The method of the aforementioned [1], wherein $L_1$ is - $CH_2CH_2(OCH_2CH_2)_n$- wherein n is an integer of 1 to 5.
[7] The method of the aforementioned [1], wherein $L_2$ is a $C_{2-11}$ alkylene chain.
[8] The method of the aforementioned [1], wherein $L_2$ is-$CH_2CH_2(OCH_2CH_2)_n$- wherein n is an integer of 1 to 5.
[9] The method of the aforementioned [1], wherein $Z_1$ is a succinimidyloxy group.
[10] The method of any of the aforementioned [1] to [9], wherein the single-stranded nucleic acid molecule comprises a sequence that inhibits expression of a target gene.
[11] A single-stranded nucleic acid molecule represented by the following general formula (Ia):

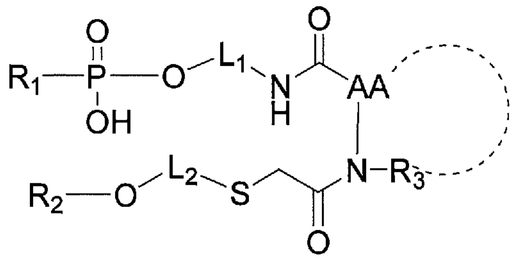

(Ia)

wherein

$R_1$ is a region comprising an oligonucleotide or a polynucleotide, the 3'-terminal nucleotide residue of which is bonded to -P(=O)(OH)-O-;
$R_2$ is a region comprising an oligonucleotide or a polynucleotide, the 5'-terminal nucleotide residue of which is bonded to -O-;
$L_1$ and $L_2$ are each independently a $C_{1-30}$ alkylene chain, or-$(CH_2)_1$-(O-$(CH_2)_m$-$)_n$- wherein 1 is an integer of 1 to 3, m is an integer of 1 to 3, and n is an integer of 1 to 10; -C(=O)-AA-$NR_3$- is an amino acid residue; and $R_3$ is a hydrogen atom or a $C_{1-6}$ alkyl group, or $R_3$ is bonded to AA to form a nitrogen-containing heterocycle (hereinafter sometimes to be referred to as single-stranded nucleic acid molecule (Ia)).

[12] The single-stranded nucleic acid molecule of the aforementioned [11], wherein the amino acid residue is a proline residue, a glycine residue, or a lysine residue with the side chain amino group being optionally protected.
[13] The single-stranded nucleic acid molecule of the aforementioned [11], wherein $L_1$ is a $C_{2-11}$ alkylene chain.
[14] The single-stranded nucleic acid molecule of the aforementioned [11], wherein $L_1$ is -$CH_2CH_2(OCH_2CH_2)_n$- wherein n is an integer of 1 to 5.
[15] The single-stranded nucleic acid molecule of the aforementioned [11], wherein $L_2$ is a $C_{2-11}$ alkylene chain.
[16] The single-stranded nucleic acid molecule of the aforementioned [11], wherein $L_2$ is -$CH_2CH_2(OCH_2CH_2)_n$- wherein n is an integer of 1 to 5.
[17] The single-stranded nucleic acid molecule of any of the aforementioned [11] to [16], which comprises a sequence that inhibits expression of a target gene.
[18] A pharmaceutical composition comprising the single-stranded nucleic acid molecule of any of the aforementioned [11] to [17].
[19] An inhibitor of expression of a target gene, comprising the single-stranded nucleic acid molecule of the aforementioned [17] .
[20] A compound represented by the following general formula (IVa):

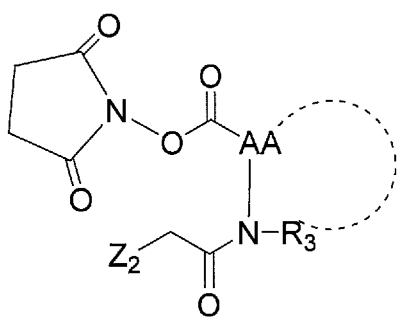

(IVa)

wherein

$Z_2$ is a halogen atom; -C(=O)-AA-$NR_3$- is an amino acid residue; and
$R_3$ is a hydrogen atom or a $C_{1-6}$ alkyl group, or $R_3$ is bonded to AA to form a nitrogen-containing heterocycle (hereinafter sometimes to be referred to as compound (IVa)).

[21] The compound of the aforementioned [20], wherein the amino acid residue is a proline residue, a glycine residue, or a lysine residue with the side chain amino group being optionally protected.
[22] The compound of the aforementioned [20], wherein $Z_2$ is a chlorine atom or a bromine atom.

**Effect of the Invention**

According to the new production method of single-stranded nucleic acid molecules of the present invention, long-chain oligomer synthesis with difficulty can be carried out by employing short-chain oligomer techniques, and new single-stranded nucleic acid molecules produced by the method can be provided.

**Brief Description of the Drawings**

[Fig.1] Fig.1 is a HPLC chart of the sense strand + P synthesized in Example 2.
[Fig.2] Fig.2 is a HPLC chart of the antisense strand (SH form) synthesized in Example 2.
[Fig.3] Fig.3 is a HPLC chart of the single-stranded nucleic acid molecule PS-0001-C3 synthesized in Example 2.
[Fig.4] Fig.4 is a HPLC chart of the single-stranded nucleic acid molecule KS-0001 synthesized in Example 4.
[Fig.5] Fig.5 is a HPLC chart of the single-stranded nucleic acid molecule AS-0001 synthesized in Reference Example.
[Fig.6] Fig.6 is a graph showing the relative expression level of the TGF-β1 gene in Experimental Example 1.
[Fig.7] Fig.7 shows schematic views illustrating an example of the single-stranded nucleic acid molecule of the present invention.
[Fig.8] Fig.8 shows schematic views illustrating another example of the single-stranded nucleic acid molecule of the present invention.
[Fig.9] Fig.9 shows schematic views illustrating other examples of the single-stranded nucleic acid molecule of the present invention.

**Description of Embodiments**

The present invention is explained in detail below. First, the definition of each symbol in the formulas is explained in detail below.
$R_1$ is a region comprising an oligonucleotide or a polynucleotide, the 3'-terminal nucleotide residue of which is bonded to -P(=O)(OH)-O-, and $R_2$ is a region comprising an oligonucleotide or a polynucleotide, the 5'-terminal nucleotide residue of which is bonded to -O-.
The term "nucleotide residue" means a bivalent group obtained by removal of the OH from the phosphoric acid group at the 5'-position of the nucleotide (in the nucleotide, the phosphoric acid is bonded to the hydroxyl group at the 5'-position of the sugur moiety of nucleocide), and removal of the H from the hydroxyl group at the 3'-position. In the "3'-terminal nucleotide residue" of $R_1$, the 3'-position is bonded to

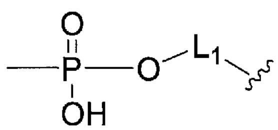

in the formula (I), and, in the "5'-terminal nucleotide residue" of $R_2$, the 5'-position is bonded to

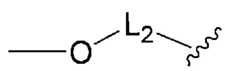

in the formula (I).
$R_1$ may be one of a region (X) and a region (Xc), each comprising an oligonucleotide or a polynucleotide containing a deoxyribonucleotide (DNA) and/or a ribonucleotide (RNA) as a building block, in the first single-stranded nucleic acid molecule of the below-mentioned present invention, and $R_2$ may be the other. Alternatively, $R_1$ may be

either of regions (X)/(Y) and regions (Xc)/(Yc), each comprising an oligonucleotide or a polynucleotide containing a deoxyribonucleotide (DNA) and/or a ribonucleotide (RNA) as a building block, in the second single-stranded nucleic acid molecule of the below-mentioned present invention, and $R_2$ may be the other. Furthermore, the above-mentioned region (Yc) and the above-mentioned region (Y) may be, for example, directly or indirectly linked to each other, as mentioned below.

$X_1$ and $X_2$ are each independently NH, S or O.
$X_1$ is preferably NH.
$X_2$ is preferably S.

$L_1$ and $L_2$ are each independently a $C_{1-30}$ alkylene chain, or - $(CH_2)_1$-(O-$(CH_2)_m$-$)_n$- wherein 1 is an integer of 1 to 3, m is an integer of 1 to 3, and n is an integer of 1 to 10.

Examples of the "$C_{1-30}$ alkylene chain" represented by $L_1$ or $L_2$ include $-CH_2-$, $-(CH_2)_2-$, $-(CH_2)_3-$, $-(CH_2)_4-$, $-CH(CH_3)-$,$-C(CH_3)_2-$, $-CH(C_2H_5)-$, $-CH(C_3H_7)-$, $-(CH(CH_3))_2-$, $-CH_2-CH(CH_3)$ -,$-CH(CH_3)-CH_2-$, $-(CH_2)_4-$, $-(CH_2)_5-$, $-(CH_2)_6-$, $-(CH_2)_7-$, $-(CH_2)_8-$,$-(CH_2)_9-$, $-(CH_2)_{10}-$, $-(CH_2)_{11}-$, $-(CH_2)_{12}-$ and the like. The "$C_{1-30}$ alkylene chain" is preferably a $C_{2-11}$ alkylene chain, more preferably a $C_{3-6}$ alkylene chain. Particularly preferably, $L_1$ is a $C_{4-6}$ alkylene chain, and $L_2$ is a $C_3$ alkylene chain.

In the "$-(CH_2)_1-(O-(CH_2)_m-)_n-$" represented by $L_1$ or $L_2$, 1 is preferably an integer of 1 to 2, m is preferably an integer of 1 to 2, and n is preferably an integer of 1 to 5, more preferably an integer of 1 to 2.

Examples of the "$-(CH_2)_1-(O-(CH_2)_m-)_n-$" represented by $L_1$ or $L_2$ include $-CH_2(OCH_2)_n-$, $-CH_2CH_2(OCH_2CH_2)_n-$ and-$CH_2CH_2CH_2(OCH_2CH_2CH_2)$n- wherein n is an integer of 1 to 5, preferred are $-CH_2(OCH_2)_n-$ and $-CH_2CH_2(OCH_2CH_2)_n-$ wherein n is an integer of 1 to 5,
more preferred is $-CH_2CH_2(OCH_2CH_2)_n-$ wherein n is an integer of 1 to 5, and
particularly preferred is $-CH_2CH_2(OCH_2CH_2)_n-$ wherein n is an integer of 1 to 2.

$L_1$ and $L_2$ are preferably each independently a $C_{2-11}$ alkylene chain or $-CH_2CH_2(OCH_2CH_2)$n- wherein n is an integer of 1 to 5.

$L_1$ and $L_2$ are more preferably each independently a $C_{3-6}$ alkylene chain or $-CH_2CH_2(OCH_2CH_2)_n-$ wherein n is an integer of 1 to 2.

Particularly preferably, $L_1$ is a $C_{4-6}$ alkylene chain, and $L_2$ is a $C_3$ alkylene chain.

In the formulas (I), (Ia), (IV), (IVa) and (V), -C(=O)-AA-$NR_3$- is an amino acid residue, and $R_3$ is a hydrogen atom or a $C_{1-6}$ alkyl group, or $R_3$ is bonded to AA to form a nitrogen-containing heterocycle.

Examples of the "$C_{1-6}$ alkyl group" represented by $R_3$ include methyl, ethyl, propyl, isopropyl, butyl, isobutyl, sec-butyl, tert-butyl, pentyl, isopentyl, neo-pentyl, 1-ethylpropyl, hexyl, isohexyl, 1,1-dimethylbutyl, 2,2-dimethylbutyl, 3,3-dimethylbutyl and 2-ethylbutyl.

Examples of the "nitrogen-containing heterocycle" formed by $R_3$ bonded to AA include 3- to 8-membered monocyclic nitrogen-containing non-aromatic heterocycles such as pyrroline, pyrrolidine, imidazoline, imidazolidine, oxazoline, oxazolidine, pyrazoline, pyrazolidine, thiazoline, thiazolidine, piperidine, piperazine, tetrahydropyridine, dihydropyridine, tetrahydropyrimidine, tetrahydropyridazine, morpholine, thiomorpholine, azepane, diazepane, azepine and the like. Among them, preferred is pyrrolidine.

$R_3$ is preferably a hydrogen atom, or $R_3$ is bonded to AA to form pyrrolidine.

The term "amino acid residue" means a bivalent group obtained by removal of the OH from the carboxy group of the amino acid, and removal of one hydrogen atom from the amino group of the amino acid. When the amino acid contains a carboxy group or an amino group in the side chain, the "amino acid residue" include a bivalent group obtained by removal of the OH or hydrogen atom from these group.

The "amino acid residue" is not particularly limited, and examples thereof include residues derived from all known amino acids, such as residues derived from $\alpha$-amino acids, residues derived from $\beta$-amino acids, residues derived from $\gamma$-amino acids, and the like.

$R_3$ is optionally bonded to AA to form a nitrogen-containing heterocycle, and examples of such "amino acid residue" include cyclic amino acid residues such as a proline residue and the like.

When the amino acid residue has a side chain functional group (e.g., an amino group, a carboxy group, a hydroxy group, a sulfanyl group (SH), an amido group ($CONH_2$) and the like), the side chain functional group may be protected by a known suitable protecting group. Examples of the protecting group for an amino group include a tert-butoxycarbonyl (Boc) group, a benzyloxycarbonyl (Z) group, a 9-fluorenylmethyloxycarbonyl (Fmoc) group and the like. Examples of the protecting group for a carboxy group include a methyl group, an ethyl group, a benzyl group and the like. Examples of the protecting group for a hydroxy group or a sulfanyl group (SH) include a trityl (triphenylmethyl) group and the like.

The side chain functional group of the amino acid residue may be modified by a sugar, a lipid, a peptide and the like.

Preferable examples of the "amino acid residue" include a proline residue, a glycine residue, a lysine residue, a phenylalanine residue, a beta-alanine residue and the like. The side chain amino group of a lysine residue may be

protected by a tert-butoxycarbonyl group, or modified by a sugar, a lipid, a peptide and the like.

The "amino acid residue" is preferably a proline residue, a glycine residue or a lysine residue with the side chain amino group being optionally protected (e.g., the side chain amino group of the lysine residue may be protected by a tert-butoxycarbonyl group), more preferably a proline residue, or a lysine residue with the side chain amino group being optionally protected (e.g., the side chain amino group of the lysine residue may be protected by a tert-butoxycarbonyl group).

$Z_1$ is a succinimidyloxy group, a benzotriazolyloxy group, a pentafluorophenoxy group or a halogen atom.

Examples of the "halogen atom" represented by $Z_1$ include a fluorine atom, a chlorine atom, a bromine atom and an iodine atom. Among them, preferred are a chlorine atom and a bromine atom.

$Z_1$ is preferably a succinimidyloxy group .

$Z_2$ is a halogen atom.

Examples of the "halogen atom" represented by $Z_2$ include a fluorine atom, a chlorine atom, a bromine atom and an iodine atom. Among them, preferred are a chlorine atom and a bromine atom.

$Z_3$ is a halogen atom.

Examples of the "halogen atom" represented by $Z_3$ include a fluorine atom, a chlorine atom, a bromine atom and an iodine atom. Among them, preferred are a chlorine atom and a bromine atom.

The production method of single-stranded nucleic acid molecule (I) is explained below. Single-stranded nucleic acid molecule (I) can be produced, for example, according to the following Production Method 1.

**Production Method 1**

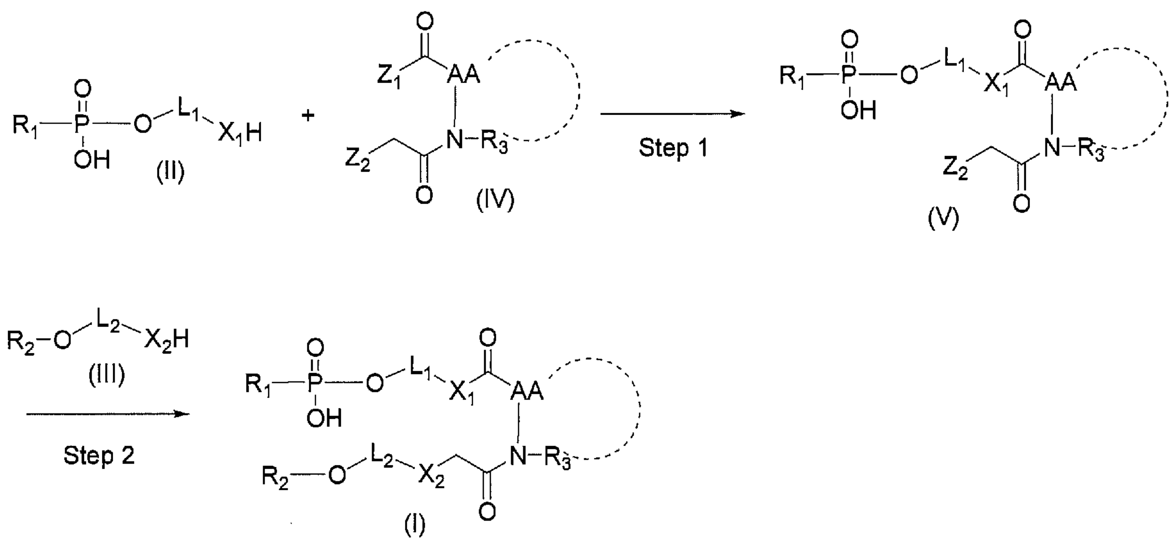

(II) (IV) (V) (III) (I)

wherein each symbol is as defined above.

(Step 1)

Nucleic acid (V) is produced by reacting nucleic acid (II) with compound (IV).

The reaction is generally carried out under a basic condition (preferably pH 8 to 9) (for example, in a phosphate buffer solution or a carbonate buffer solution, or in the presence of a base such as diisopropylethylamine, triethylamine and the like).

The reaction is generally carried out in a solvent, at 0°C - room temperature. Examples of the solvent include amide solvents such as N,N-dimethylformamide, N,N-dimethylacetamide and the like; ether solvents such as tetrahydrofuran (THF), dioxane and the like; polar solvents such as dimethyl sulfoxide, acetonitrile and the like; water and the like. Among them, preferred are a mixed solvent of dimethylformamide and water, and water.

After the completion of the reaction, low molecular compound is removed from the reaction mixture by simple gel filtration column and the like to give nucleic acid (V).

(Step 2)

Single-stranded nucleic acid molecule (I) is produced by reacting nucleic acid (V) with nucleic acid (III).
The reaction is generally carried out under a basic condition (preferably pH 8 to 9) (for example, in a phosphate buffer solution or a carbonate buffer solution).
The reaction is generally carried out in a solvent at room temperature.
After the completion of the reaction, the reaction mixture is purified by HPLC and the like to give single-stranded nucleic acid molecule (I).
Single-stranded nucleic acid molecule (I) wherein -C(=O)-AA-$NR_3$- is an amino acid residue having a protected side chain functional group, can be produced from compound (IV) wherein - C(=O)-AA-$NR_3$- is an amino acid residue having a protected side chain functional group, according to Step 1 and Step 2.
Single-stranded nucleic acid molecule (I) wherein -C(=O)-AA-$NR_3$- is an amino acid residue having a protected side chain functional group, can be converted to single-stranded nucleic acid molecule (I) wherein -C(=O)-AA-$NR_3$- is an amino acid residue having a non-protected side chain functional group, by deprotection known per se. For example, single-stranded nucleic acid molecule (I) wherein -C(=O)-AA-$NR_3$- is a lysine residue with the side chain amino group being protected by a tert-butoxycarbonyl group, can be converted to single-stranded nucleic acid molecule (I) wherein -C(=O)-AA-$NR_3$- is a lysine residue with the side chain amino group being not protected, by a treatment with an acid such as hydrogen chloride, trifluoroacetic acid and the like.
Single-stranded nucleic acid molecule (I) wherein -C(=O)-AA-$NR_3$- is an amino acid residue having a modified side chain functional group, can be produced from compound (IV) wherein - C(=O)-AA-$NR_3$- is an amino acid residue having a modified side chain functional group, according to Step 1 and Step 2.
Alternatively, single-stranded nucleic acid molecule (I) wherein -C(=O)-AA-$NR_3$- is an amino acid residue having a non-protected side chain functional group, can be converted to single-stranded nucleic acid molecule (I) wherein -C(=O)-AA-$NR_3$- is an amino acid residue having a modified side chain functional group, by introducing sugar, lipid, peptide and the like to the non-protected functional group, with employing amidation reaction, esterification reaction, etherication reaction and the like.
Compound (IV) used in Production Method 1 can be produced, for example, according to the following Production Method 2.

**Production Method 2**

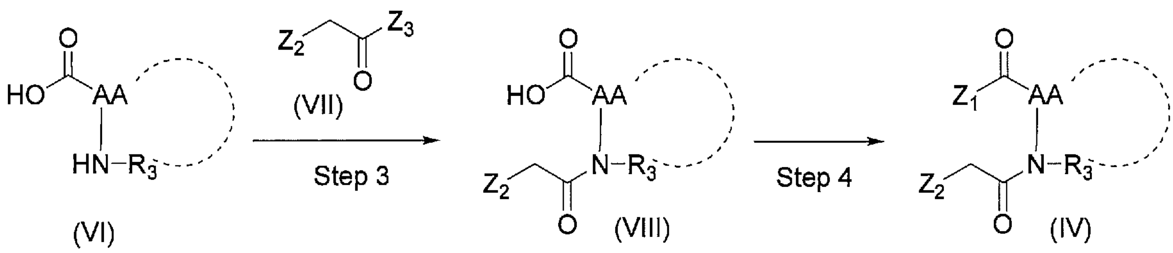


wherein $Z_3$ is a halogen atom, and the other symbols are as defined above.

(Step 3)

Compound (VIII) is produced by reacting the amino acid, compound (VI) with compound (VII).
When $Z_3$ is a chlorine atom, the reaction is generally carried out in a solvent, at room temperature to under heating. Examples of the solvent include ether solvents such as tetrahydrofuran (THF), dioxane and the like; polar solvents such as acetonitrile, dimethylformamide and the like, and the like. Among them, preferred is tetrahydrofuran.
When $Z_3$ is a bromine atom, the reaction is generally carried out in a solvent, in the presence of a base, under cooling to at room temperature. Examples of the solvent include water; ether solvents such as tetrahydrofuran (THF), dioxane and the like; polar solvents such as acetonitrile, dimethylformamide and the like, and the like. Among them, preferred is a mixed solvent of water and tetrahydrofuran. Examples of the base include sodium hydroxide and the like.
After the completion of the reaction, the reaction mixture is worked up by a known method to give compound (VIII).

(Step 4)

Compound (IV) is produced by converting the carboxyl group of compound (VIII) to -C(=O)$Z_1$.

When $Z_1$ is a succinimidyloxy group, the reaction is carried out by reacting compound (VIII) with N-hydroxysuccinimide in the presence of a condensing agent such as 1-ethyl-3-(3-dimethylaminopropyl)carbodiimide hydrochloride and the like.

The reaction is generally carried out in a solvent at room temperature. Examples of the solvent include halogen solvents such as dichloromethane, chloroform and the like; ether solvents such as tetrahydrofuran (THF), dioxane and the like, and the like. Among them, preferred is dichloromethane.

Alternatively, the reaction is carried out by reacting compound (VIII) with di(N-succinimidylcarbonate) in the presence of a base.

Examples of the base include N,N-dimethylaminopyridine and the like.

The reaction is generally carried out in a solvent at room temperature. Examples of the solvent include polar solvents such as acetonitrile, dimethylformamide and the like, and the like. Among them, preferred is acetonitrile.

When $Z_1$ is a benzotriazolyloxy group, the reaction is carried out by reacting compound (VIII) with 1-hydroxybenzotriazole. This reaction is carried out in the same manner as in the case where $Z_1$ is a succinimidyloxy group.

When $Z_1$ is a pentafluorophenoxy group, the reaction is carried out by reacting compound (VIII) with pentafluorophenol. This reaction is carried out in the same manner as in the case where $Z_1$ is a succinimidyloxy group.

When $Z_1$ is a chlorine atom, the reaction is carried out by reacting compound (VIII) with a chlorinating agent such as thionyl chloride and the like.

Compound (IV) wherein $Z_1$ is a succinimidyloxy group, i.e., a compound represented by the the following general formula (IVa):

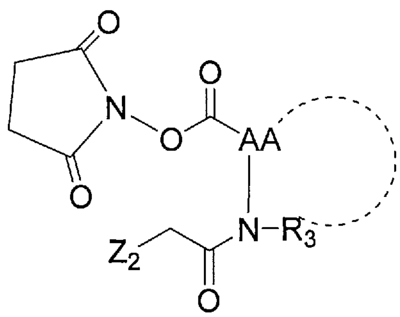

(IVa)

wherein each symbol is as defined above,
is particularly preferably used.

Nucleic acid (II) used in Production Method 1 can be synthesized by removing the DMTr group from a carrier containing

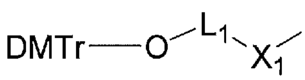

wherein DMTr is a 4,4'-dimethoxytrityl group, and the other symbols are as defined above,
and then starting solid-phase synthesis by phosphoramidite method, from the resulting carrier.

Nucleic acid (III) used in Production Method 1 can be synthesized by removing the protecting group for 5'-terminal hydroxyl group in the final step of solid-phase synthesis, and then introducing the corresponding reagent into the hydroxyl group by phosphoramidite method, and then subjecting the resulting compound to suitable treatment. For example, when nucleic acid (III) is a nucleic acid represented by the formula

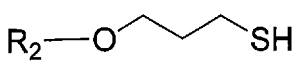,

the compound can be synthesized by removing the protecting group for 5'-terminal hydroxyl group, and then introducing

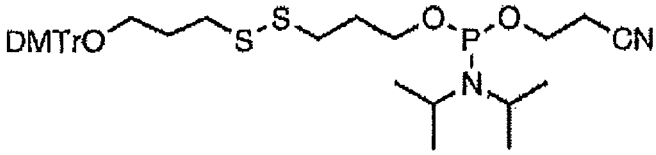

wherein DMTr is as defined above,
into the hydroxyl group by phosphoramidite method, and then releasing the resulting compound from the solid-phase and deprotecting according to a conventional method, and then treating the resulting compound with dithiothreitol.

Among of thus-produced single-stranded nucleic acid molecule (I), single-stranded nucleic acid molecule represented by the following general formula (Ia):

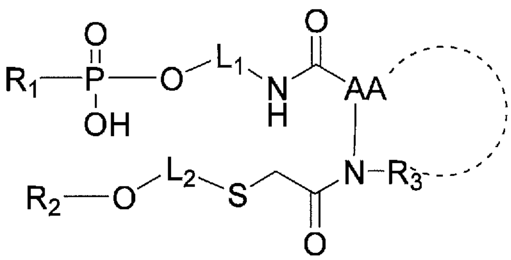

(Ia)

wherein each symbol is as defined above (hereinafter sometimes to be referred to as single-stranded nucleic acid molecule (Ia)) is a new compound.

Preferable examples of single-stranded nucleic acid molecule (Ia) include single-stranded nucleic acid molecules represented by the following formulas.

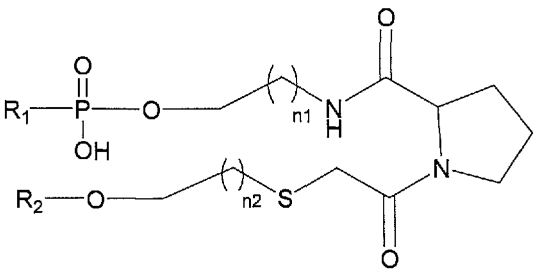

(I-a-Pro)

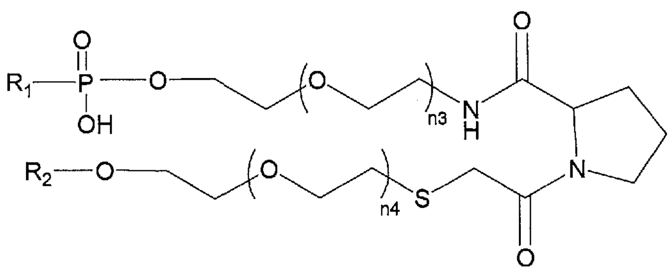

(I-b-Pro)

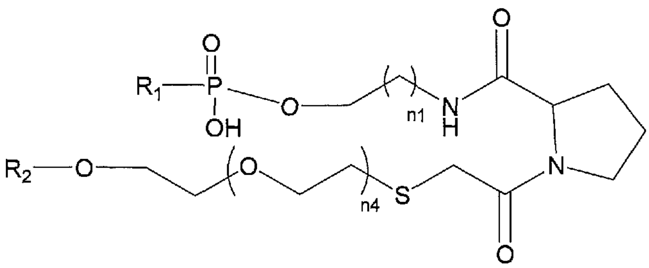

(I-c-Pro)

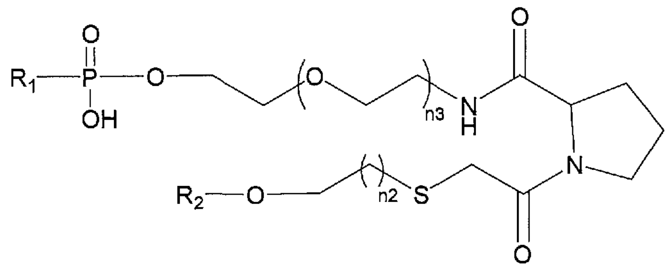

(I-d-Pro)

wherein n1 and n2 are each independently an integer of 1 to 10, n3 and n4 are each independently an integer of 1 to 5, and the other symbols are as defined above,

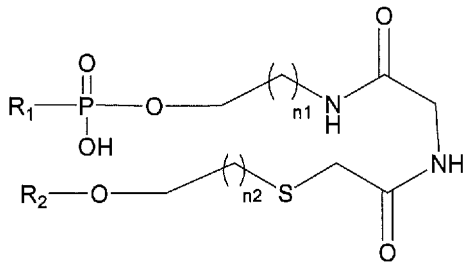

(I-a-Gly)

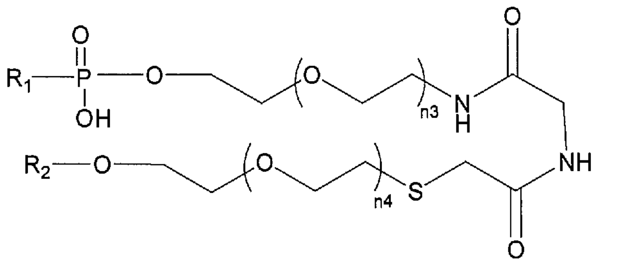

(I-b-Gly)

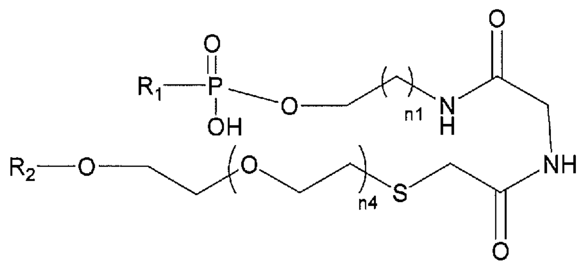

(I-c-Gly)

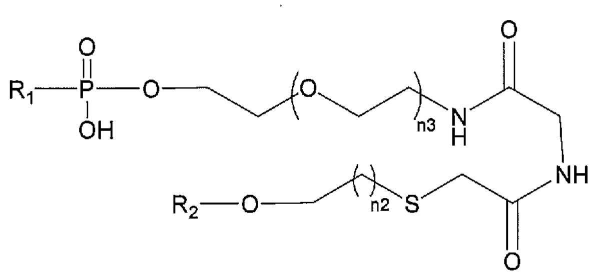

(I-d-Gly)

wherein each symbol is as defined above,

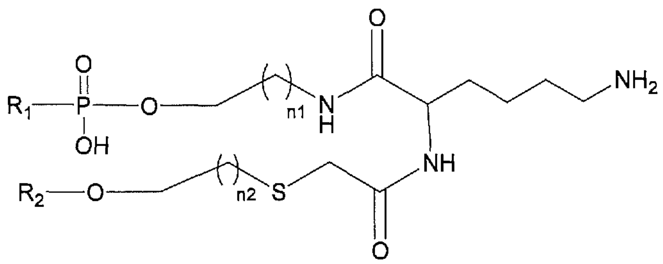

(I-a-Lys)

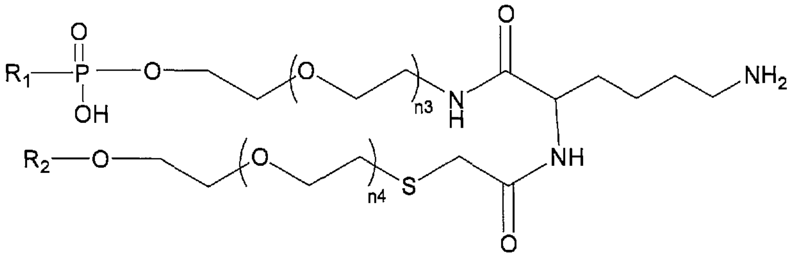

(I-b-Lys)

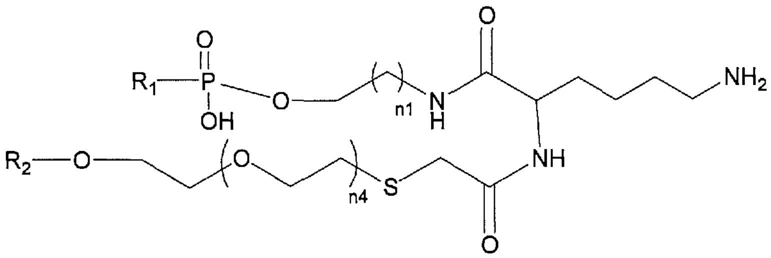

(I-c-Lys)

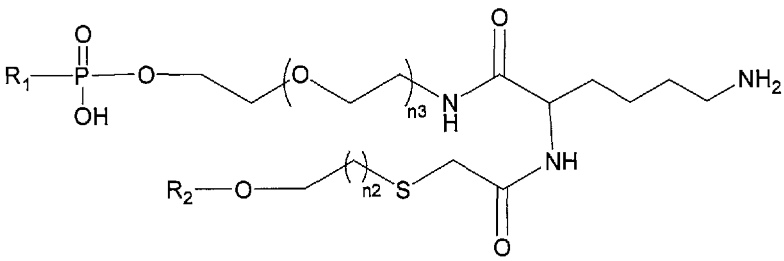

(I-d-Lys)

wherein each symbol is as defined above,

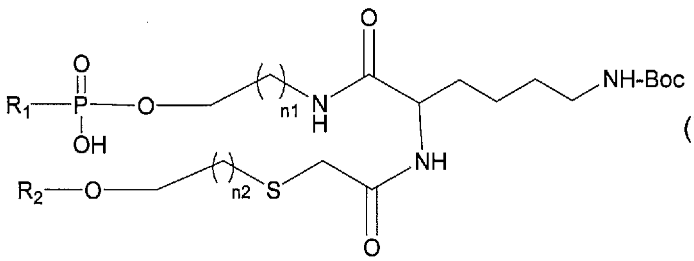

(I-a-Lys(Boc))

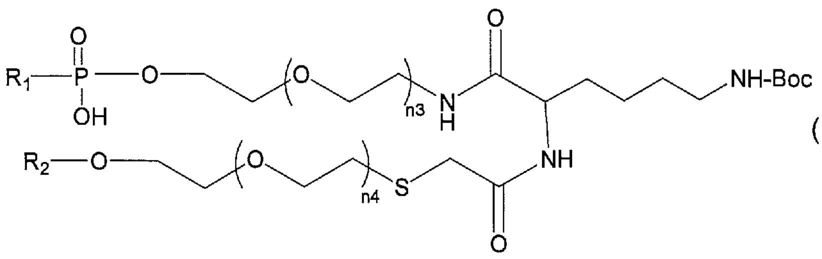

(I-b-Lys(Boc))

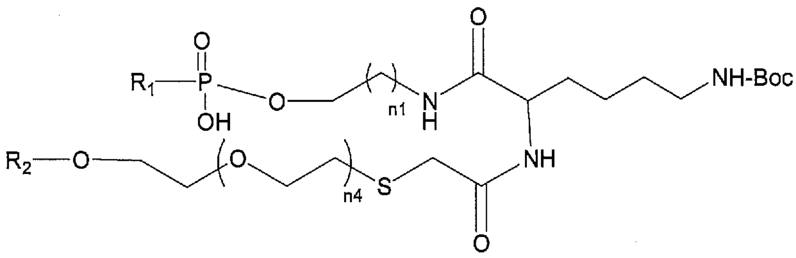

(I-c-Lys(Boc))

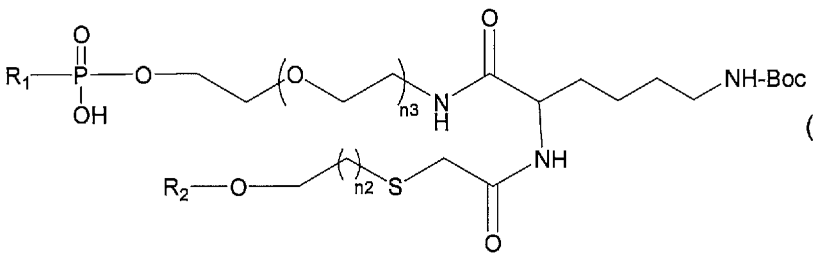

(I-d-Lys(Boc))

wherein Boc is a tert-butoxycarbonyl group, and the other symbols are as defined above.

In the aforementioned formulas,

n1 is preferably an integer of 3 to 5,

n2 is preferably 2,

n3 is preferably an integer of 1 to 2, and

n4 is preferably an integer of 1 to 2.

More preferable examples of single-stranded nucleic acid molecule (Ia) include single-stranded nucleic acid molecules represented by the the following formulas.

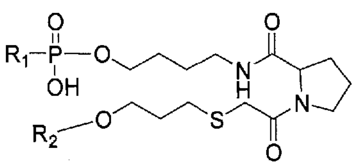

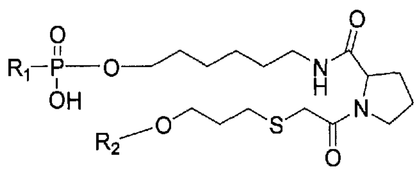

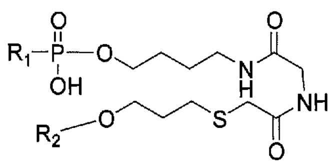

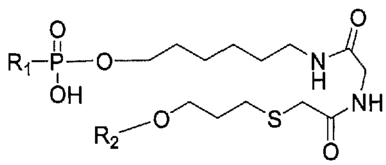

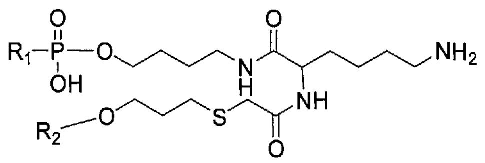

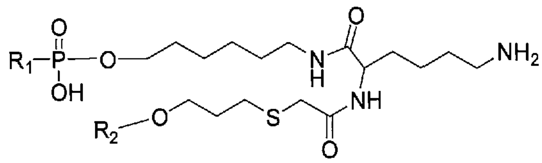

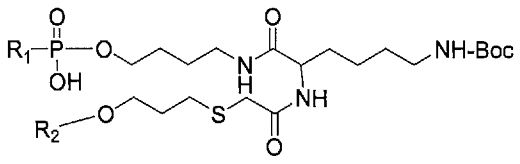

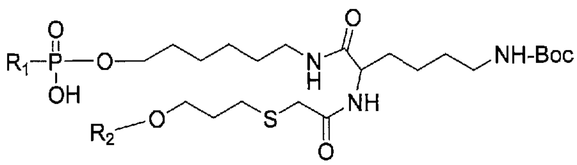

wherein Boc is a tert-butoxycarbonyl group, and the other symbols are as defined above.

Still more preferable examples of single-stranded nucleic acid molecule (Ia) include the following molecules.

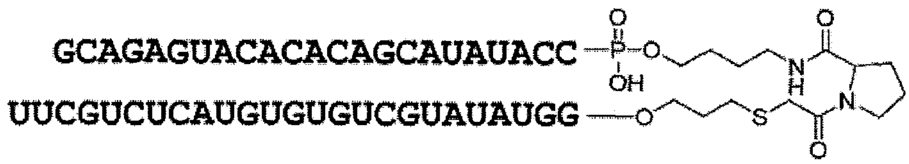

SEQ ID NO: 5

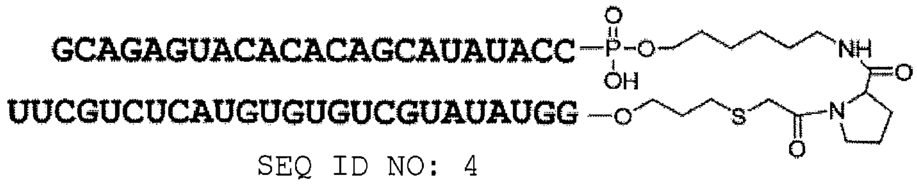

SEQ ID NO: 4

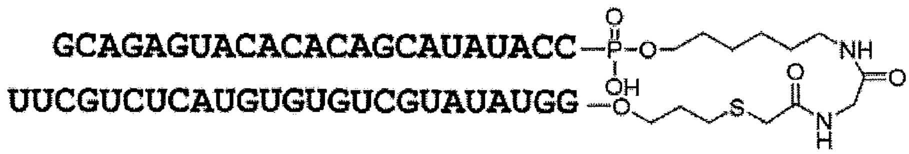

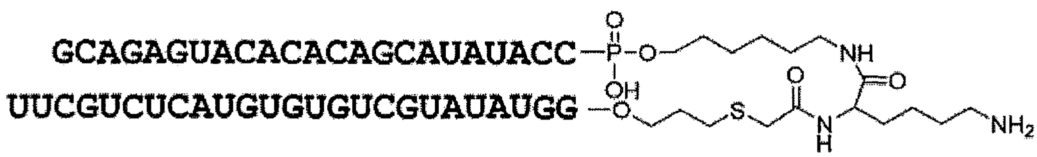

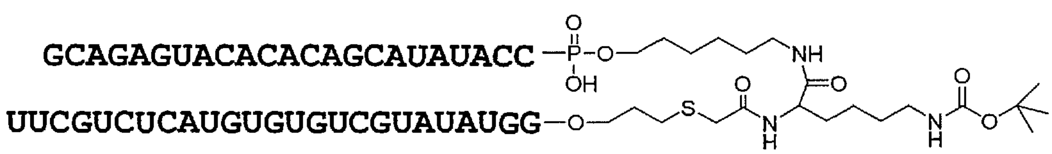

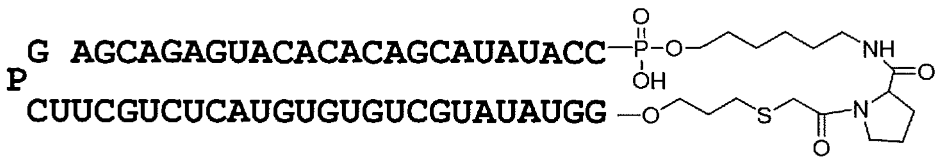

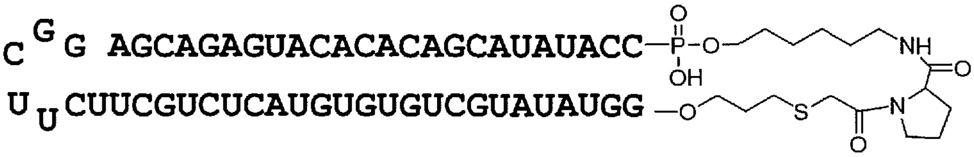

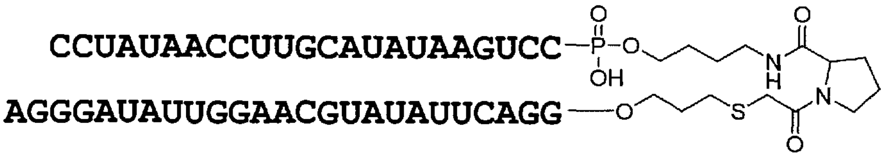

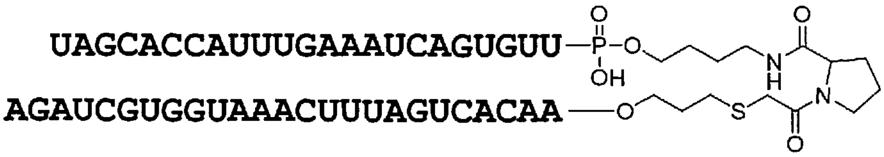

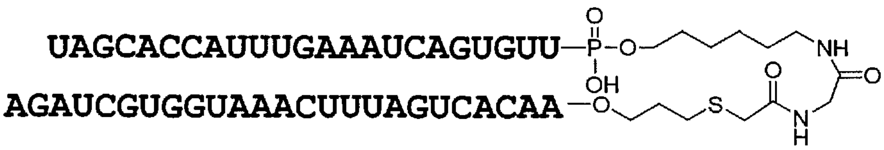

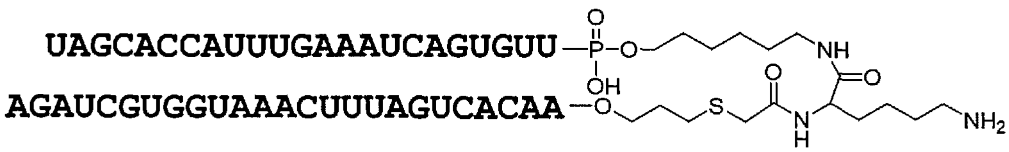

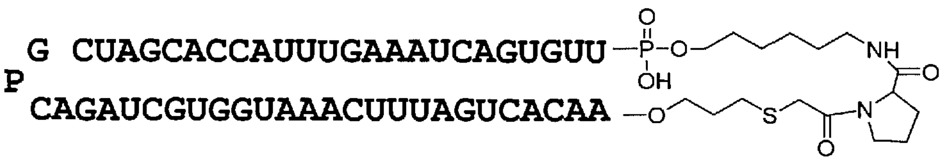

The reaction can also be carried out using compounds (IV'b) - (IV'g) instead of compound (IV), according to the above-mentioned production method. The structures of compounds (IV'b) - (IV'g) and examples thereof are shown below.

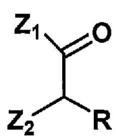

**(IV'b)**

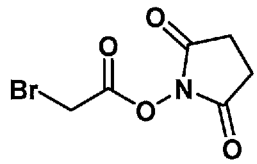

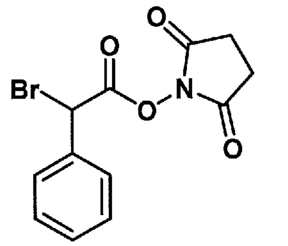

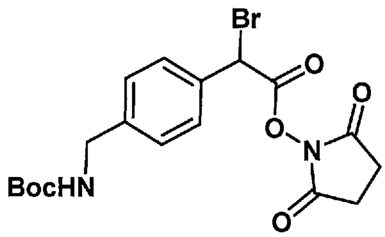

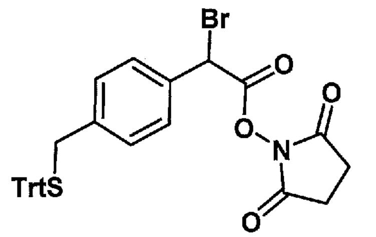

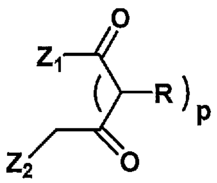

(IV'c)

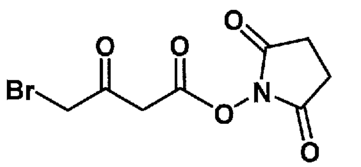

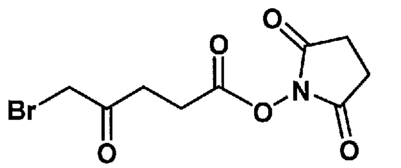

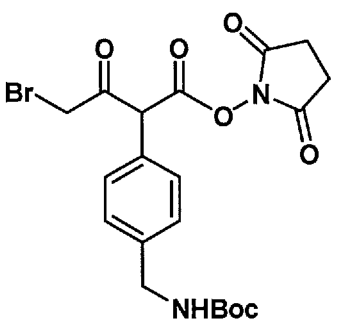

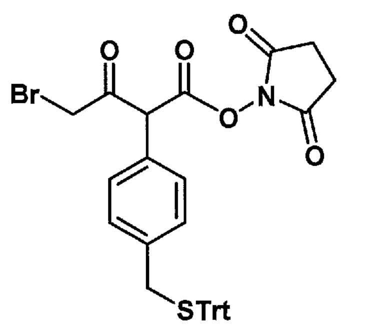

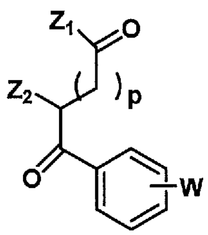

(IV'd)

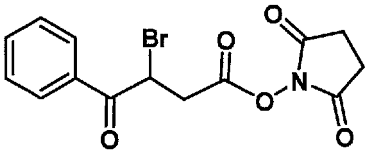
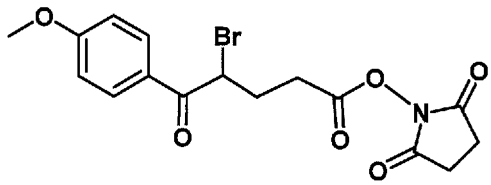

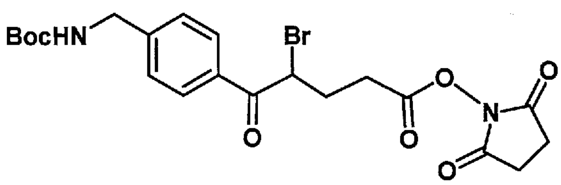
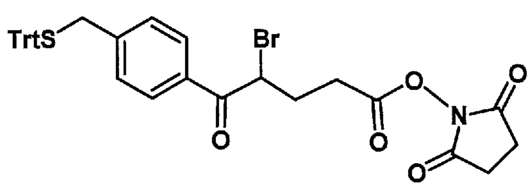

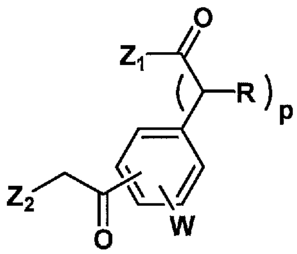
(IV'e)

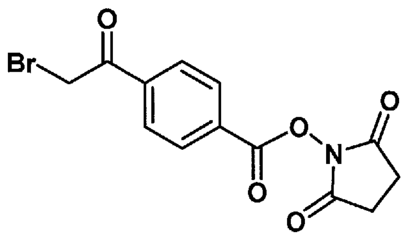
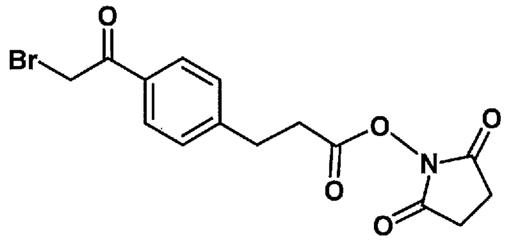

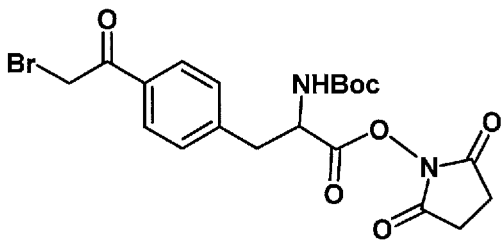
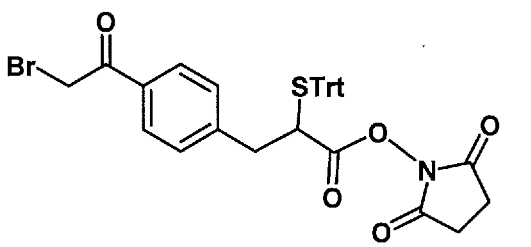

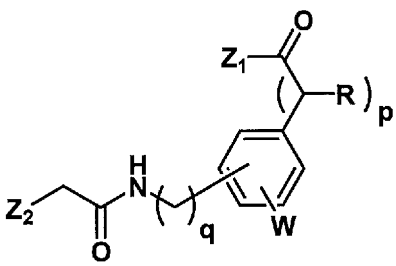
(IV'f)

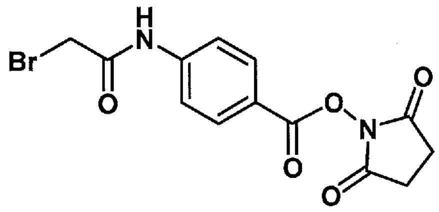

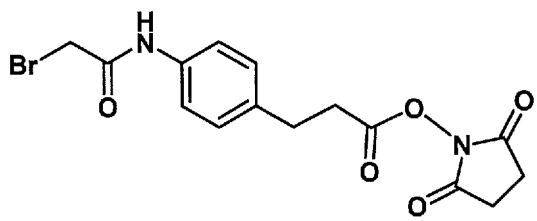

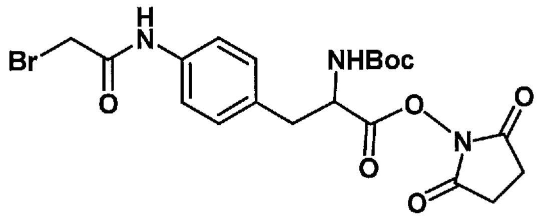

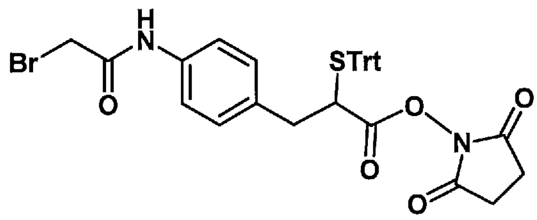

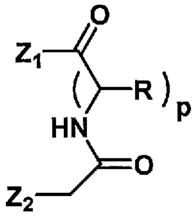
(IV'g)

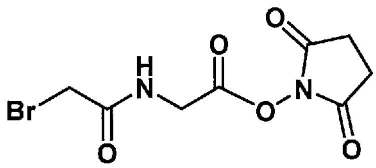

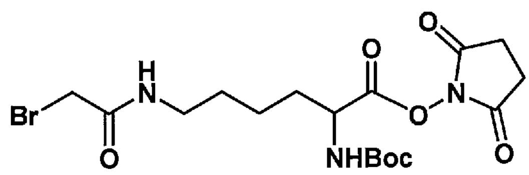

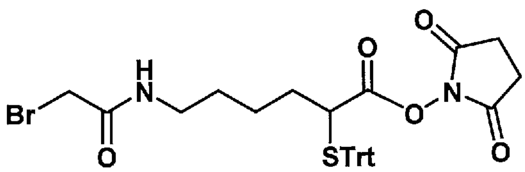

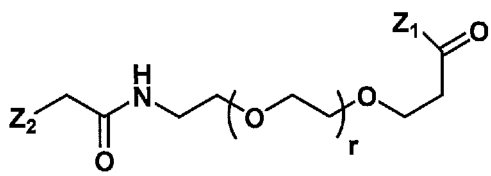
(IV'h)

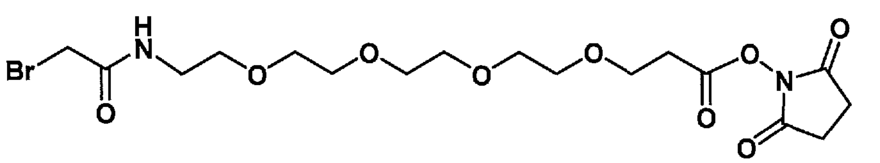

In each formula,
R is a hydrogen atom, an optionally substituted $C_{6-10}$ aryl group,
a protected amino group or a protected sulfanyl group (SH);
W is an optionally substituted $C_{1-6}$ alkyl group, or a $C_{1-6}$ alkoxy group;
p is an integer of 0 to 6;
q is an integer of 0 to 6;

r is an integer of 0 to 6;
Trt is a trityl group; and
the other symbols are as defined above.

Examples of the "$C_{6-10}$ aryl group" include phenyl, 1-naphthyl and 2-naphthyl.

Examples of the substituent of the "optionally substituted $C_{6-10}$ aryl group" include an optionally substituted $C_{1-6}$ alkyl group.

Examples of the "$C_{1-6}$ alkyl group" include methyl, ethyl, propyl, isopropyl, butyl, isobutyl, sec-butyl, tert-butyl, pentyl, isopentyl, neo-pentyl, 1-ethylpropyl, hexyl, isohexyl, 1,1-dimethylbutyl, 2,2-dimethylbutyl, 3,3-dimethylbutyl and 2-ethylbutyl.

Examples of the "optionally substituted $C_{1-6}$ alkyl group" include a $C_{1-6}$ alkyl group; and a $C_{1-6}$ alkyl group substituted by substituent(s) selected from a protected amino group and a protected sulfanyl group (SH).

Examples of the "protected amino group" include an amino group protected by a protecting group such as tert-butoxycarbonyl group, benzyloxycarbonyl group and the like.

Examples of the "protected sulfanyl group" include a sulfanyl group protected by a protecting group such as trityl(triphenylmethyl) group and the like.

Preferable examples of the "optionally substituted $C_{1-6}$ alkyl group" include a $C_{1-6}$ alkyl group (e.g., methyl) having an amino group protected by a tert-butoxycarbonyl group, a $C_{1-6}$ alkyl group (e.g., methyl) having a sulfanyl group protected by a trityl(triphenylmethyl) group, and the like.

Examples of the "$C_{1-6}$ alkoxy group" include methoxy, ethoxy, propoxy, isopropoxy, butoxy, isobutoxy, sec-butoxy, tert-butoxy, pentyloxy and hexyloxy.

The following nucleic acids (I'b) - (I'g) can be produced by using the above-mentioned compounds (IV'b) - (IV'g). The structures of nucleic acids (I'b) - (I'g) and examples thereof are shown below.

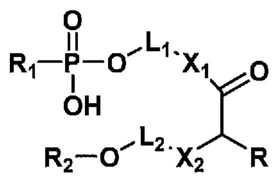

(I'b)

Examples of nucleic acid (I'b) include the following nucleic acids.

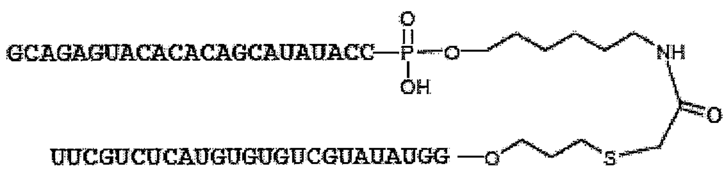

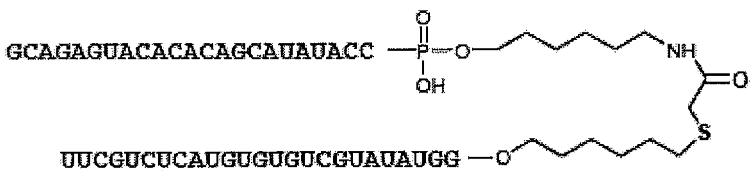

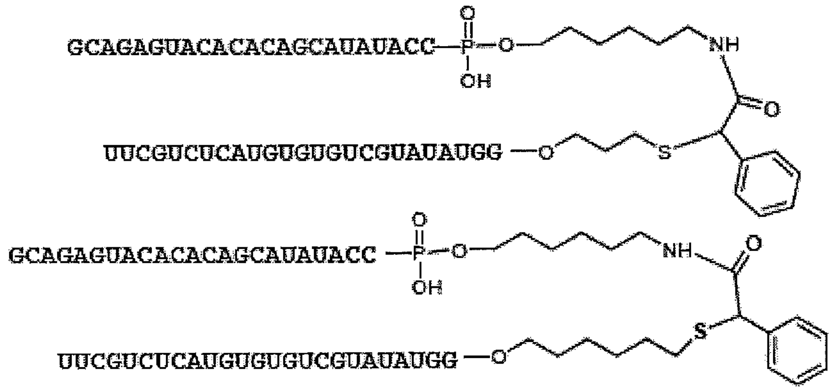

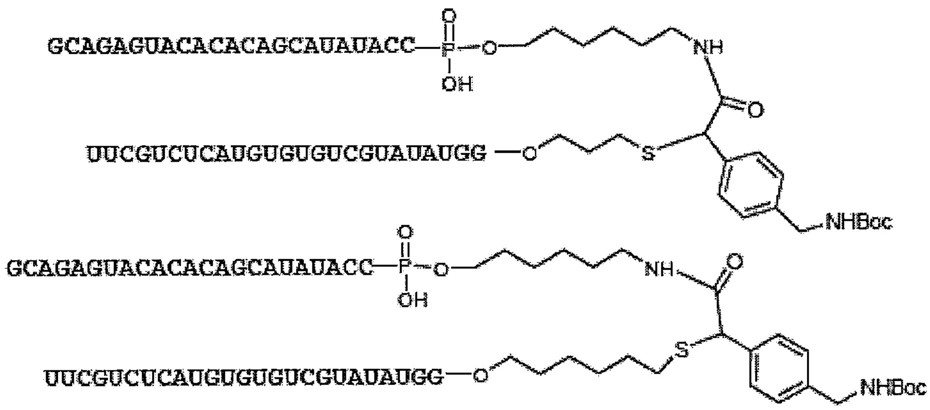

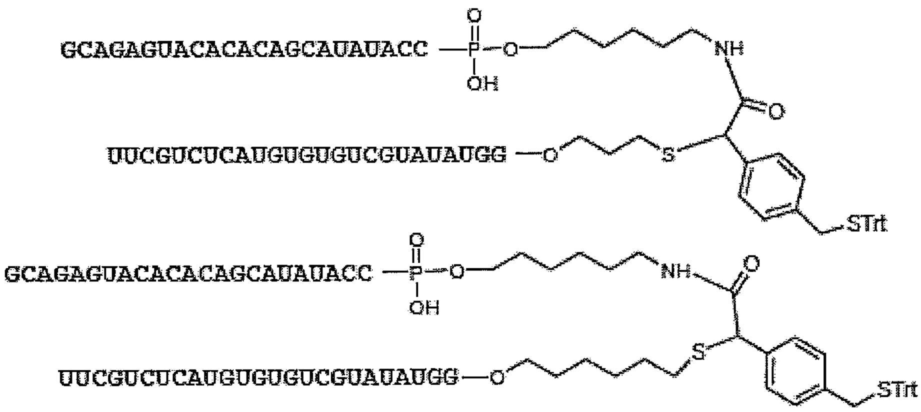

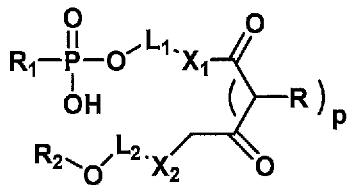 (I'c)

Examples of nucleic acid (I'c) include the following nucleic acids.

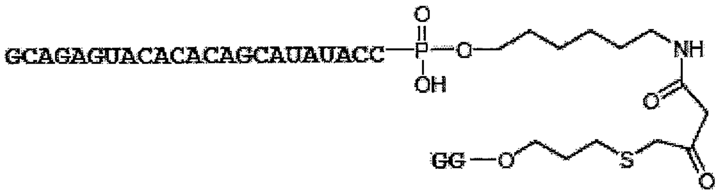

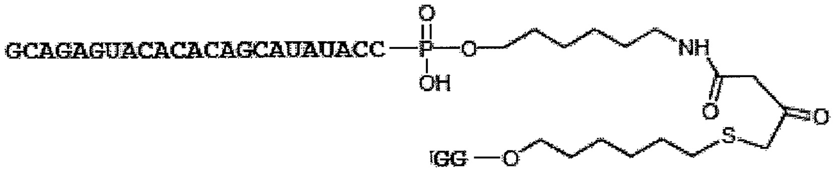

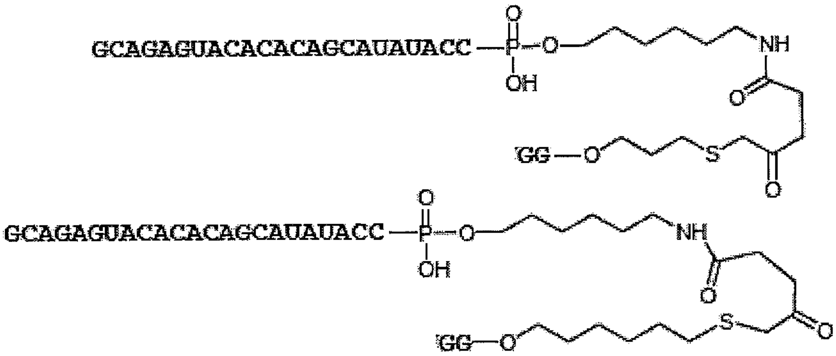

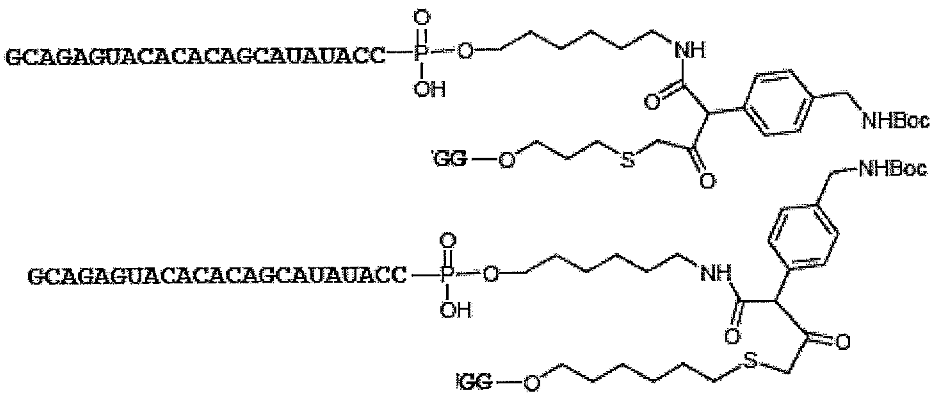

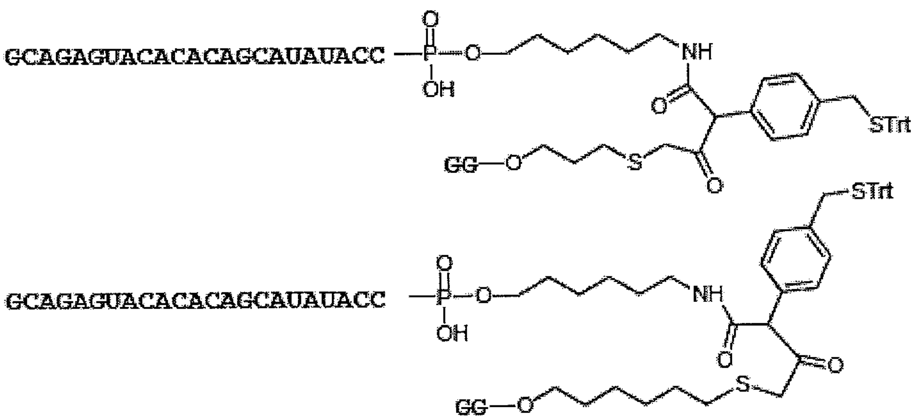

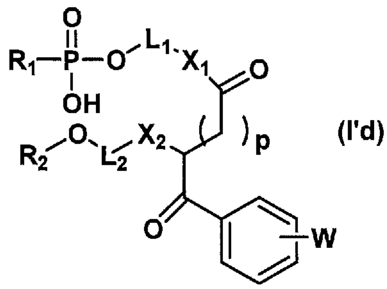

(I'd)

Examples of nucleic acid (I'd) include the following nucleic acids.

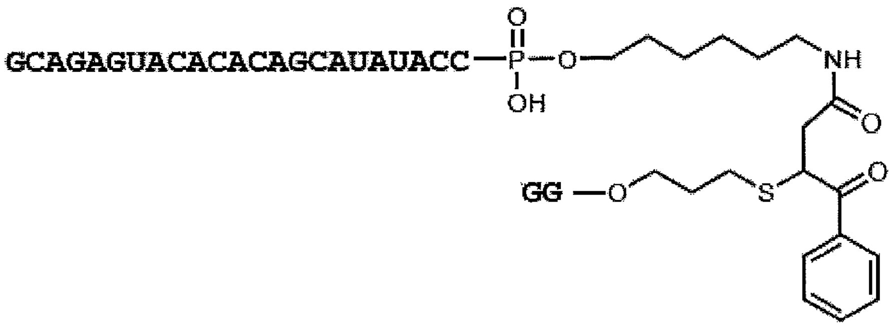

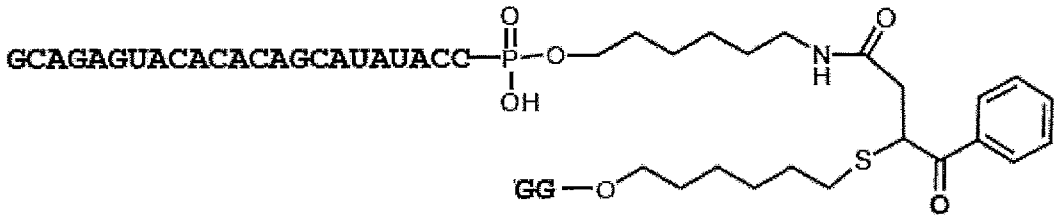

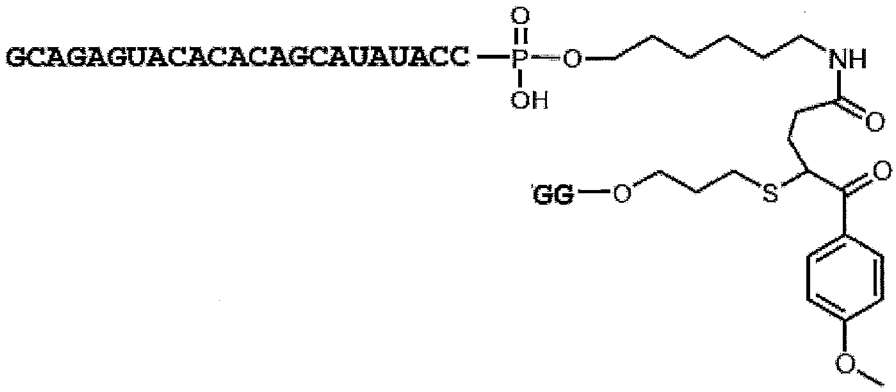

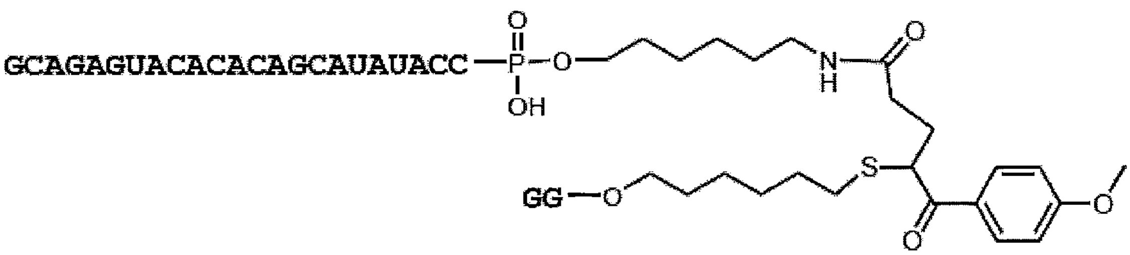

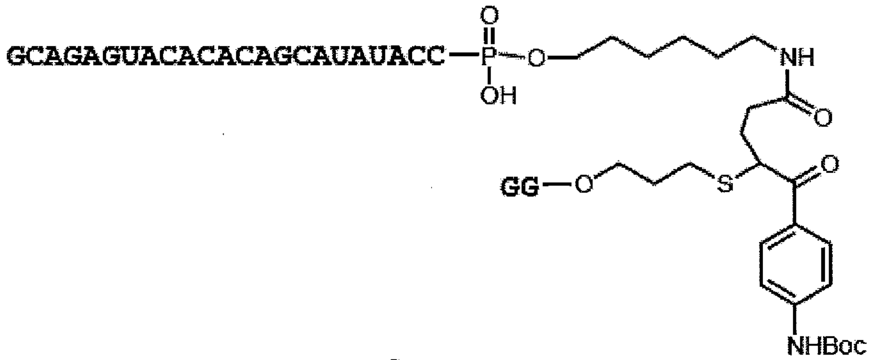

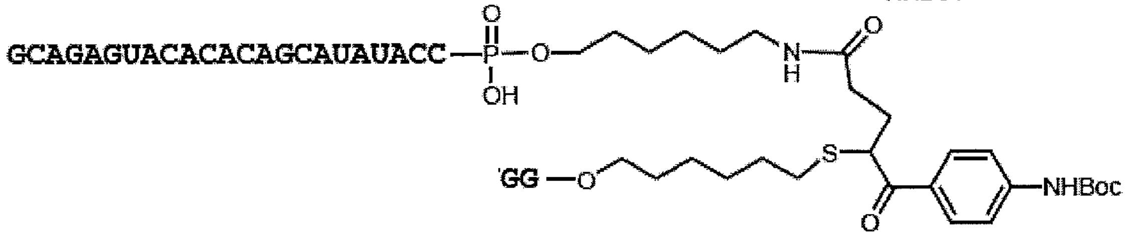

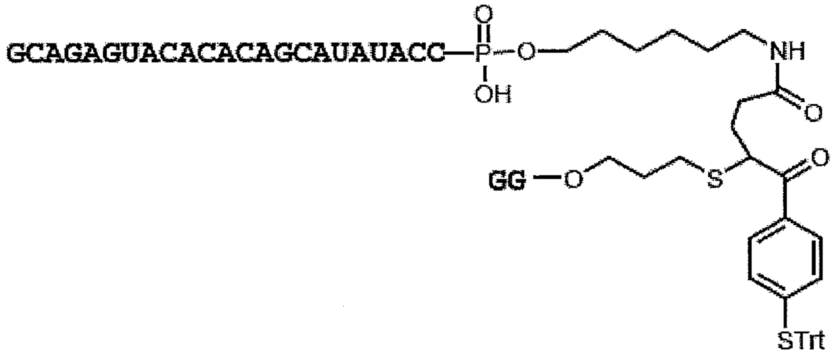

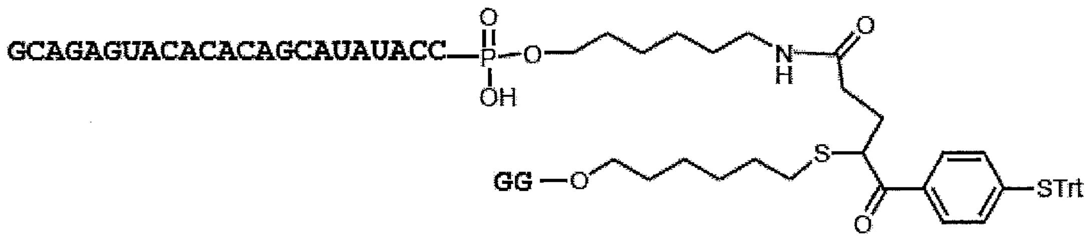

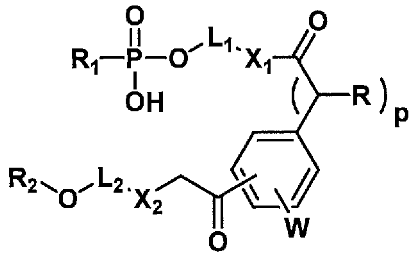

(I'e)

Examples of nucleic acid (I'e) include the following nucleic acids.

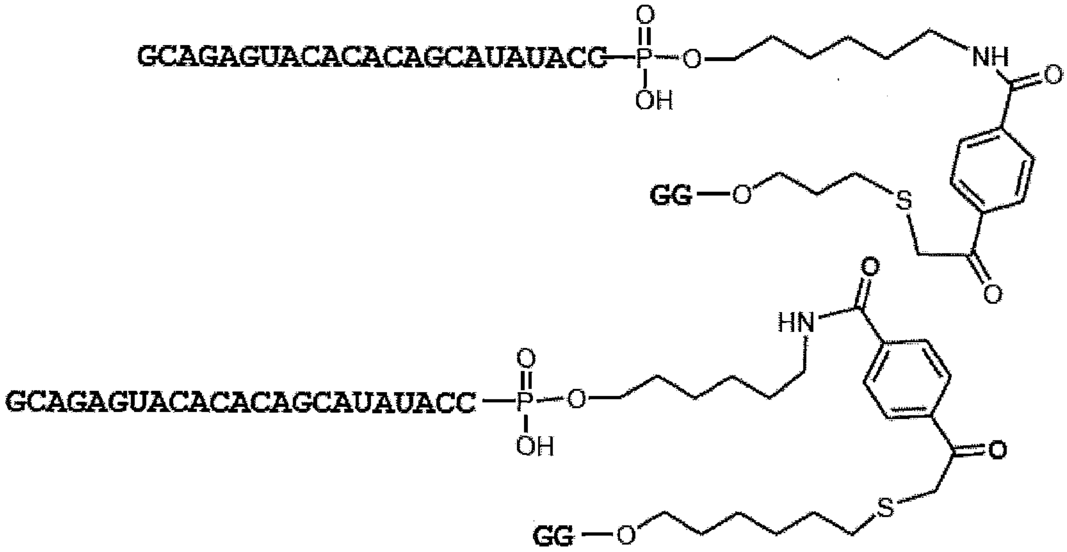

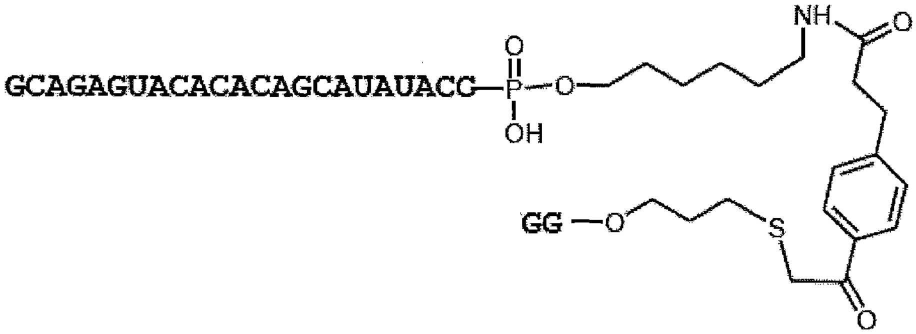

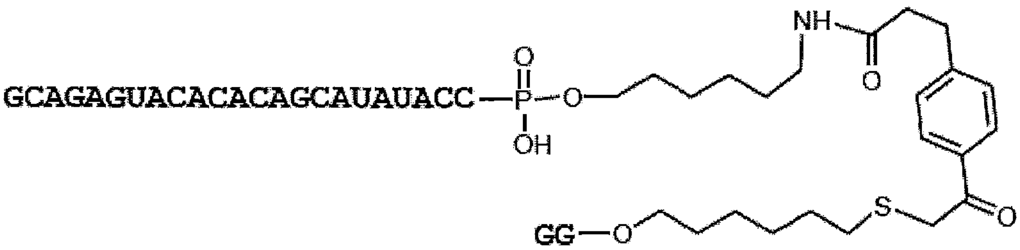

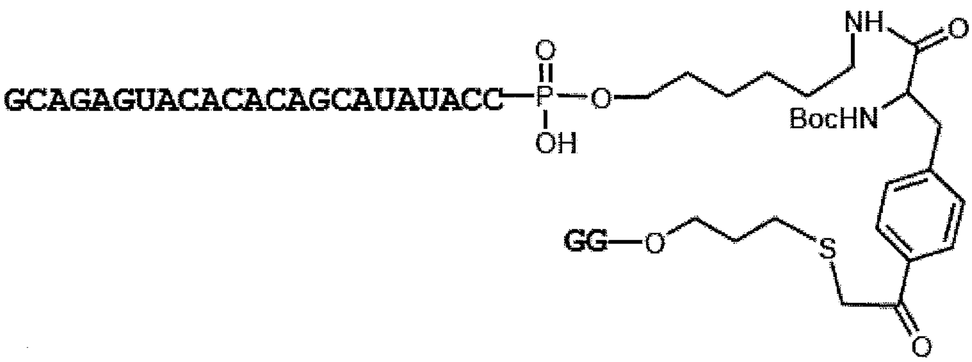

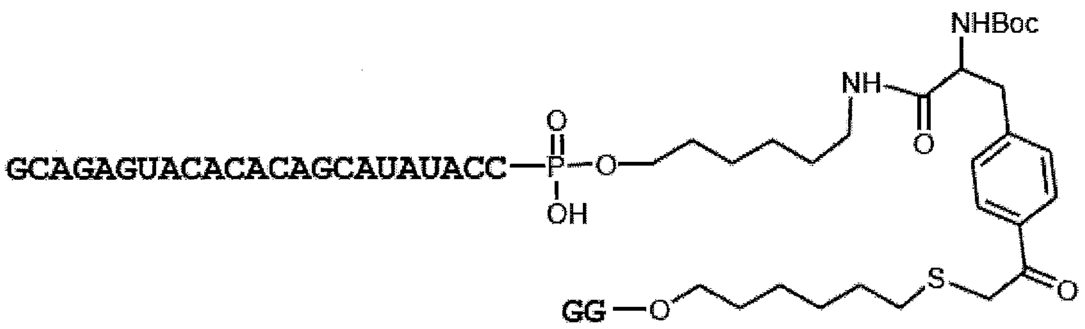

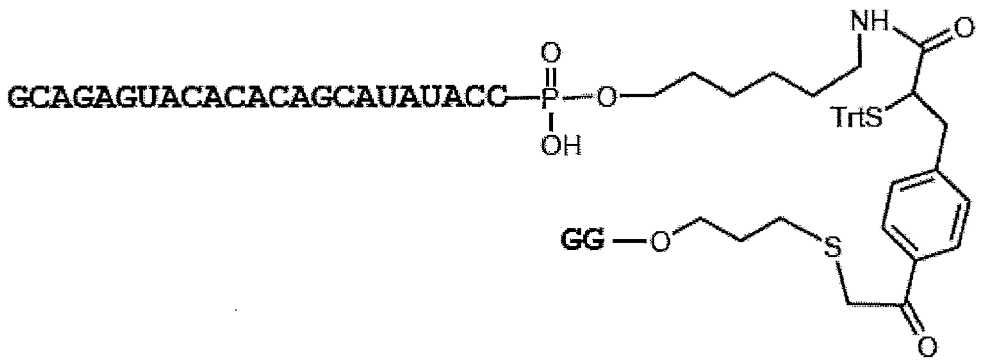

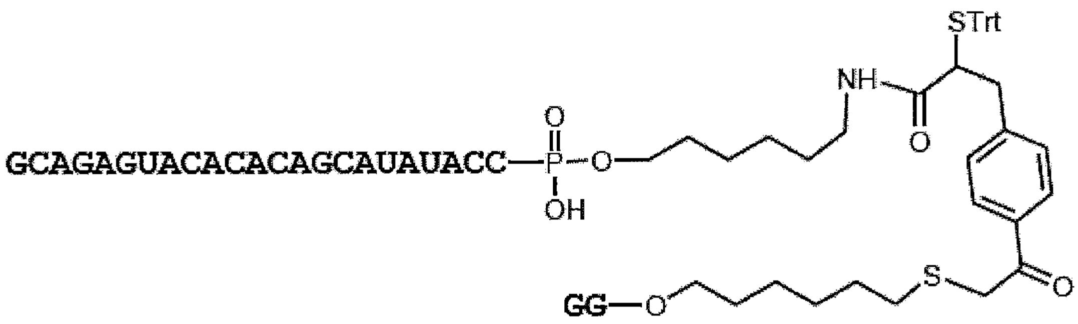

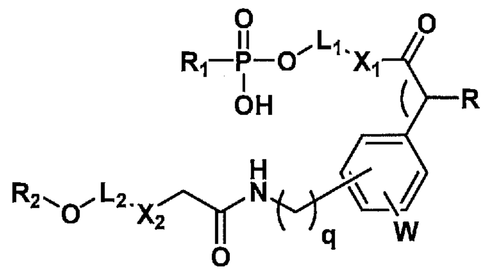

(I'f)

Examples of nucleic acid (I'f) include the following nucleic acids.

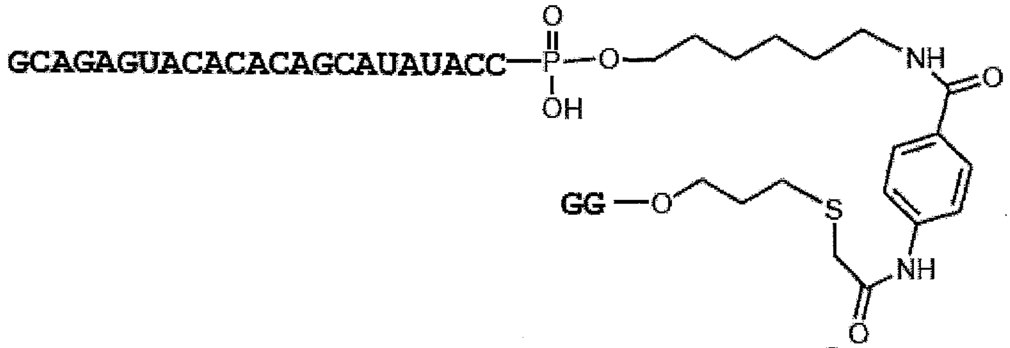

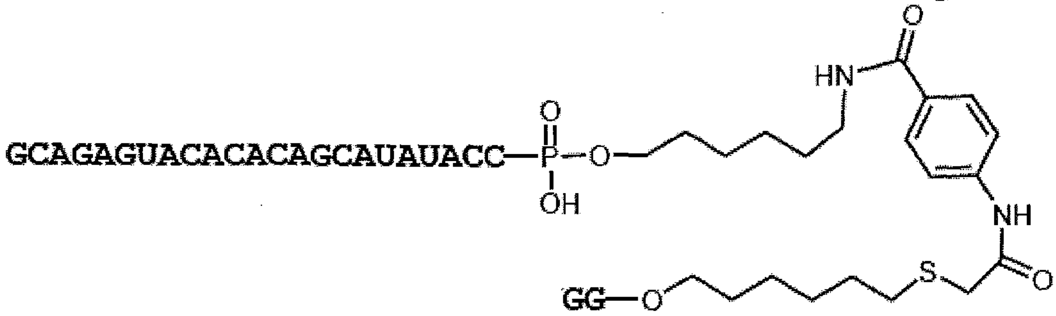

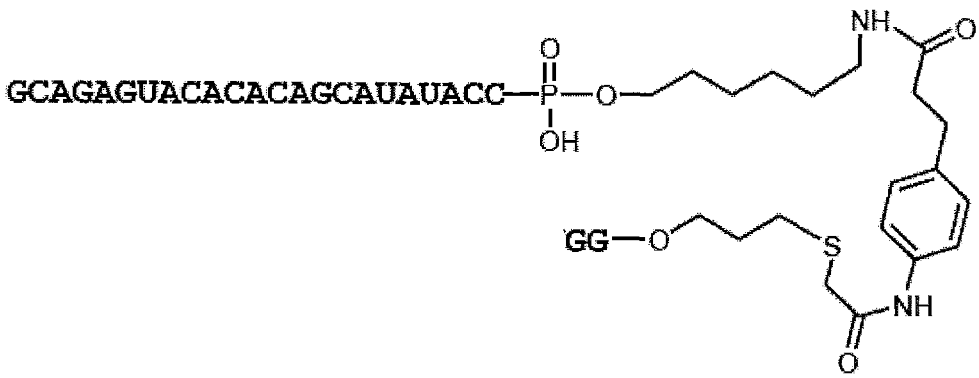

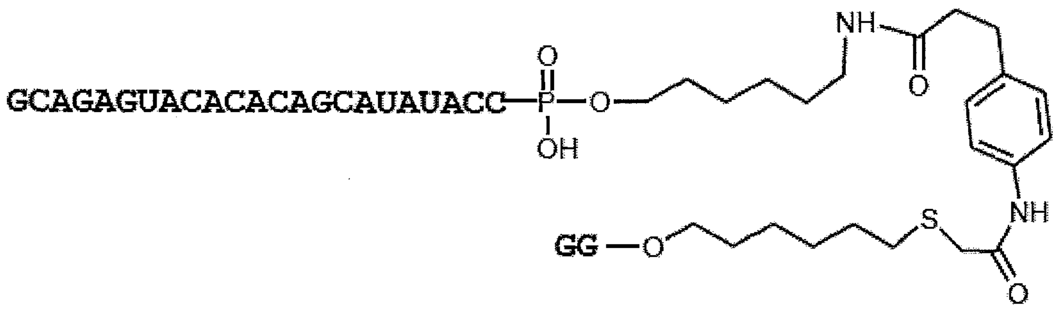

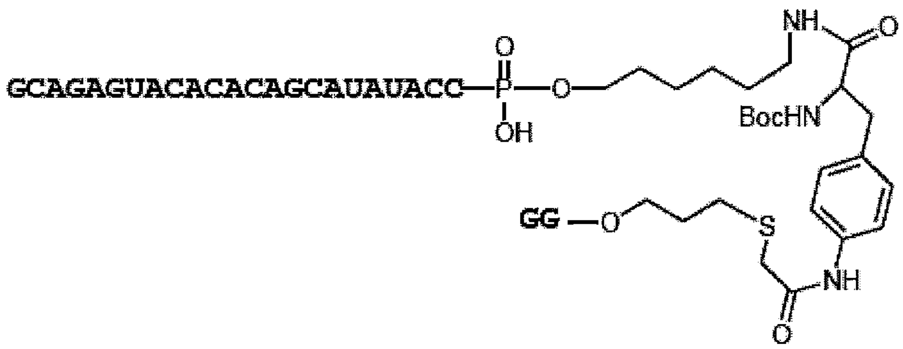

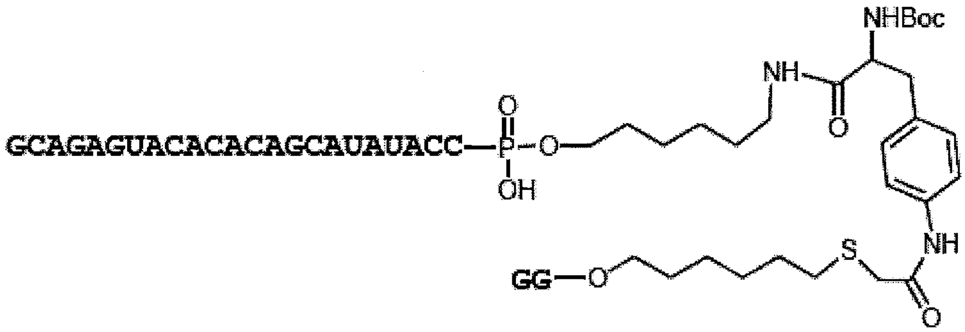

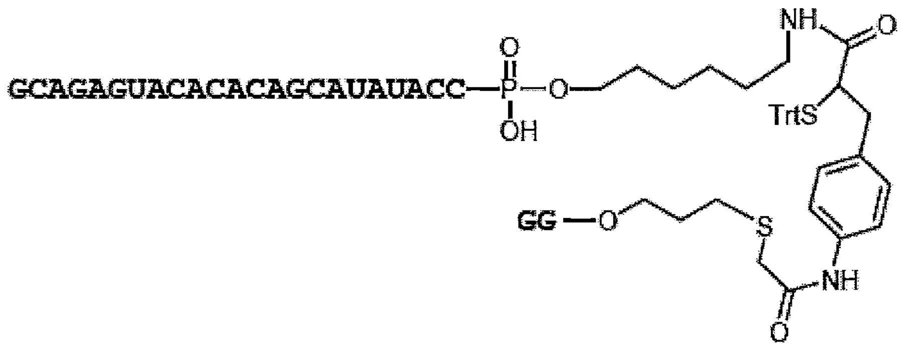

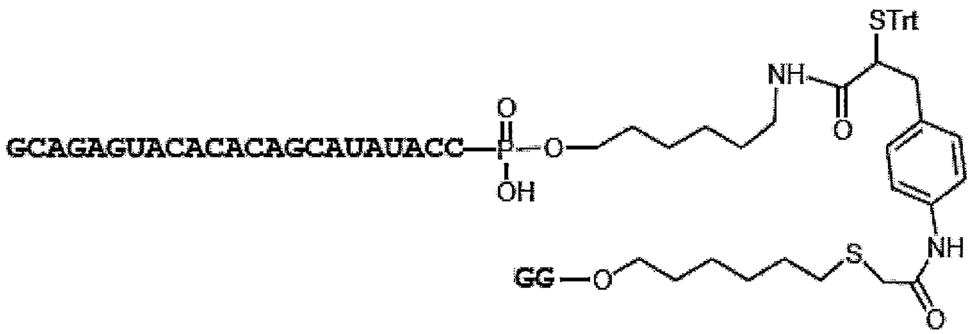

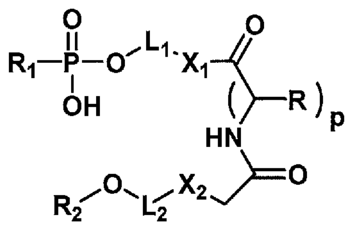

(I'g)

Examples of nucleic acid (I'g) include the following nucleic acids.

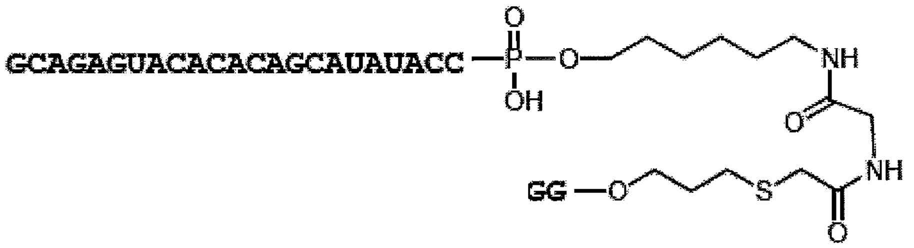

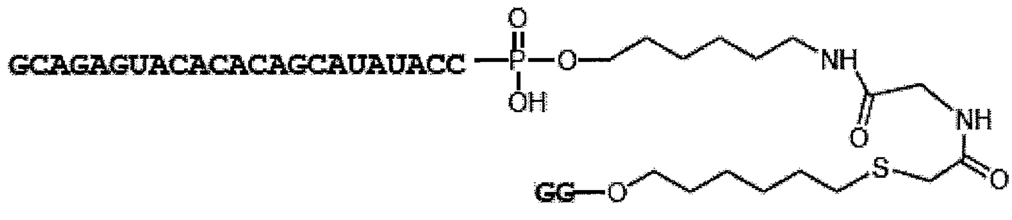

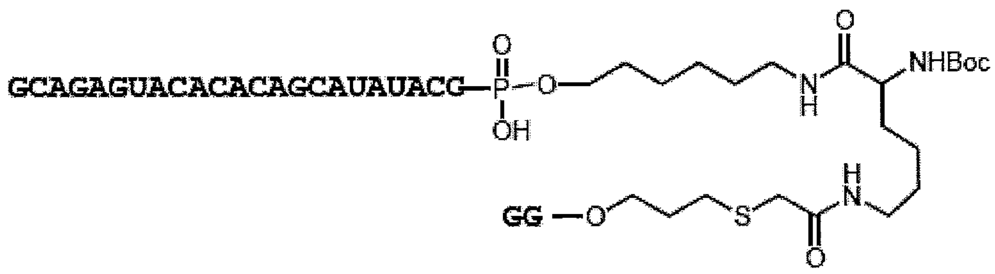

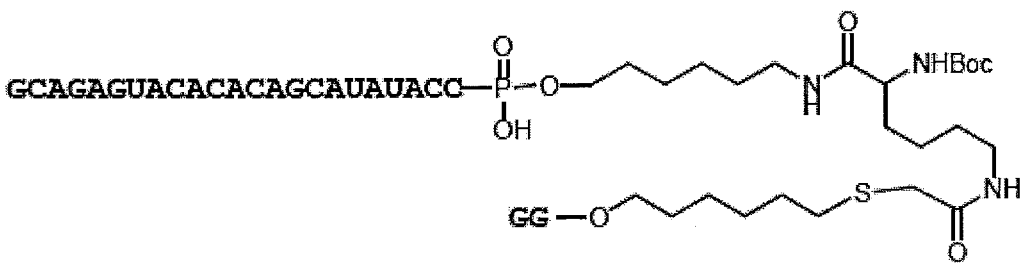

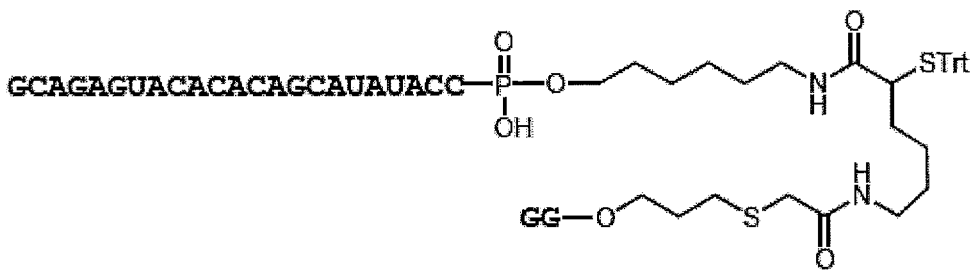

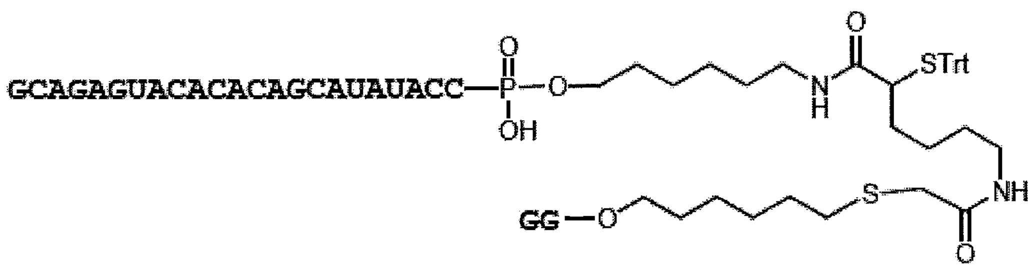

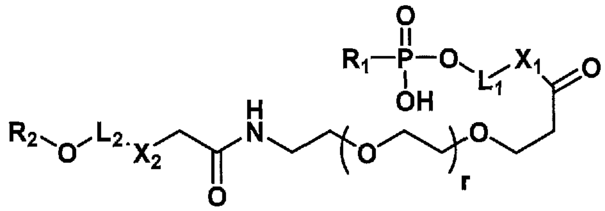

(I'h)

Examples of nucleic acid (I'h) include the following nucleic acids.

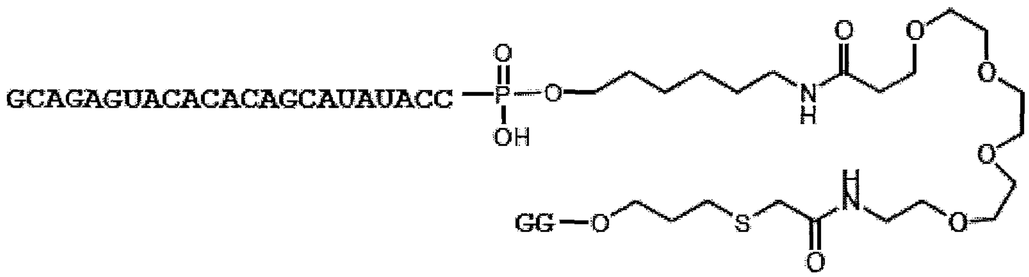

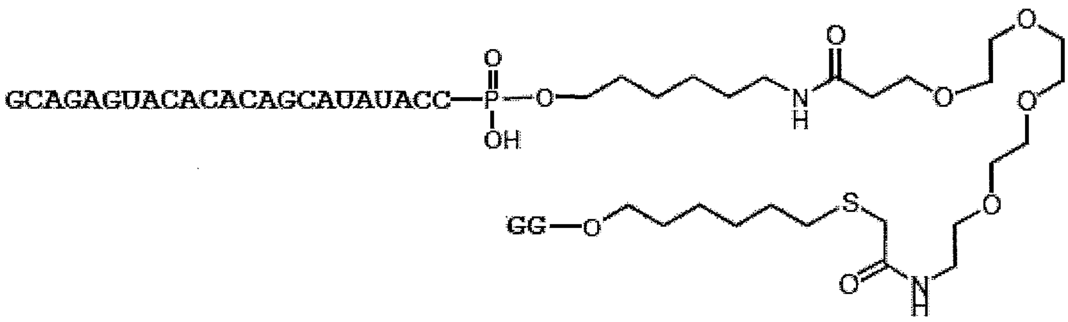

1. Single-Stranded Nucleic Acid Molecule

As described above, the single-stranded nucleic acid molecule (I) of the present invention, which is produced by the above-metioned production method, is a single-stranded nucleic acid molecule containing an expression inhibitory sequence that inhibits expression of a target gene, and is characterized as follows. The single-stranded nucleic acid molecule contains a region (X), a linker region (Lx) and a region (Xc) ($R_1$ in the formula (I) is one of the region (X) and the region (Xc) (the below-mentioned first single-stranded nucleic acid molecule), or $R_1$ contains one of the region (X) and the region (Xc) (the below-mentioned second single-stranded nucleic acid molecule), and $R_2$ is the other (the below-mentioned first single-stranded acetic acid molecule), or $R_2$ contains the other (the below-mentioned second single-stranded nucleic acid molecule)); and the linker region (Lx) is linked between the region (Xc) and the region (Xc); and at least one of the region (X) and the region (Xc) contains the expression inhibitory sequence; and the linker region (Lx) has a structure represented by the below-mwntioned formula (IL).

In the present invention, "inhibition of expression of a target gene" means disrupting the expression of the target gene, for example. The mechanism by which the inhibition is achieved is not particularly limited, and may be downregulation or silencing, for example. The inhibition of the expression of the target gene can be verified by: a decrease in the amount of a transcription product generated from the target gene; a decrease in the activity of the transcription product; a decrease in the amount of a translation product generated from the target gene; a decrease in the activity of the translation product; or the like, for example. The proteins may be mature proteins, precursor proteins before being subjected to processing or post-translational modification, or the like, for example.

The single-stranded nucleic acid molecule of the present invention can be used to inhibit expression of a target gene in vivo or in vitro, for example, so that it also can be referred to as an "single-stranded nucleic acid molecule for inhibiting expression of a target gene" or "inhibitor of expression of a target gene". Furthermore, the single-stranded nucleic acid molecule of the present invention can inhibit expression of a target gene by, for example, RNA interference, so that it also can be referred to as an "single-stranded nucleic acid molecule for RNA interference", "molecule for inducing RNA interference", or "RNA interference agent" or "RNA interference-inducting agent". Furthermore, the single-stranded nucleic acid molecule of the present invention can also suppress, for example, a side effect such as interferon induction in vivo, and is superior in nuclease resistance.

In the single-stranded nucleic acid molecule of the present invention, the 5' end and the 3' end are not linked to each other. Thus, the single-stranded nucleic acid molecule of the present invention also can be referred to as a "linear single-stranded nucleic acid molecule".

In the single-stranded nucleic acid molecule of the present invention, the expression inhibitory sequence is a sequence that exhibits an activity of inhibiting expression of a target gene when the single-stranded nucleic acid molecule of the present invention is introduced into a cell in vivo or in vitro, for example. The expression inhibitory sequence is not particularly limited, and can be set as appropriate depending on the kind of a target gene whose expression is to be inhibited. As the expression inhibitory sequence, a sequence involved in RNA interference caused by siRNA can be used as appropriate, for example. Generally, RNA interference is a phenomenon in which a long double-stranded RNA (dsRNA) is cleaved in a cell by Dicer to produce a double-stranded RNA (siRNA: small interfering RNA) composed of about 19 to 21 base pairs and having a protruding 3' end, and one of the single-stranded RNAs composing the siRNA binds to a target mRNA to degrade the mRNA, whereby the translation of the mRNA is inhibited. As the sequence of the single-stranded RNA of the siRNA binding to the target mRNA, various kinds of sequences for various kinds of target genes have been reported, for example. In the present invention, for example, the sequence of the single-stranded RNA of the siRNA can be used as the expression inhibitory sequence. In the present invention, not only the sequences of the single-stranded RNA of the siRNA known at the time of the filing of the present application but also sequences that would be identified in the future can be used as the expression inhibitory sequence, for example.

The expression inhibitory sequence is preferably at least 90% complementary, more preferably 95% complementary, still more preferably 98% complementary, and particularly preferably 100% complementary to a predetermined region of the target gene, for example. When the expression inhibitory sequence satisfies the above-described complementarity, an off-target effect can be reduced sufficiently, for example.

As specific examples of the expression inhibitory sequence, when the target gene is TGF-β1, a 19-mer sequence shown in SEQ ID NO: 1 can be used, for example. 5'-UAUGCUGUGUGUACUCUGC-3'(SEQ ID NO: 1)

In the single-stranded nucleic acid molecule of the present invention, the linker region is, for example, represented by the following formula (IL).

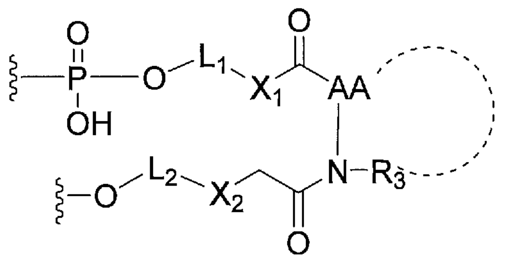 (IL)

wherein each symbol is as defined above.

The 3'-terminal nucleotide residue of the region (Xc) may be bonded to -P(=O)(OH)-O- of the linker region (Lx), and the 5'-terminal nucleotide residue of the region (X) may be bonded to -O- of the linker region (Lx), or the 3'-terminal nucleotide residue of the region (X) may be bonded to -P(=O)(OH)-O- of the linker region (Lx), and the 5'-terminal nucleotide residue of the region (Xc) may be bonded to -O- of the linker region (Lx).

In the single-stranded nucleic acid molecule of the present invention, the region (Xc) is complementary to the region (X). Thus, in the single-stranded nucleic acid molecule of the present invention, a double strand can be formed by fold-back of the region (Xc) toward the region (X) and self-annealing of the region (Xc) and the region (X). The single-stranded nucleic acid molecule of the present invention can form a double strand intramolecularly as described above. Thus, the structure of the single-stranded nucleic acid molecule of the present invention is totally different from the structre of a double-stranded RNA obtained through annealing of two separate single-stranded RNAs, such as siRNA conventionally used in RNA interference, for example.

In the single-stranded nucleic acid molecule of the present invention, for example, only the region (Xc) may fold back to form a double strand with the region (X), or another double strand may be formed in another region. Hereinafter, the former single-stranded nucleic acid molecule, i.e., the single-stranded nucleic acid molecule in which double strand formation occurs at one location is referred to as a "first single-stranded nucleic acid molecule", and the latter single-stranded nucleic acid molecule, i.e., the single-stranded nucleic acid molecule in which double strand formation occurs at two locations is referred to as a "second single-stranded nucleic acid molecule". Examples of the first and second single-stranded nucleic acid molecules are given below. It should be noted, however, that the present invention is not limited to these illustrative examples.

(1) First Single-Stranded Nucleic Acid Molecule

The first single-stranded nucleic acid molecule is a molecule containing the region (X), the region (Xc) and the linker region (Lx), for example.

The first single-stranded nucleic acid molecule may contain the region (Xc), the linker region (Lx) and the region (X) in this order from the 5' side to the 3' side, or may contains the region (Xc), the linker region (Lx) and the region (X) in this order from the 3' side to the 5' side, for example.

In the first single-stranded nucleic acid molecule, the region (Xc) is complementary to the region (X). It is only necessary that the region (Xc) has a sequence complementary to the entire region or part of the region (X). Preferably, the region (Xc) contains or is composed of a sequence complementary to the entire region or part of the region (X). The region (Xc) may be perfectly complementary to the entire region or part of the region (X), or one or a few bases in the region (Xc) may be non-complementary to the same, for example. Preferably, the region (Xc) is perfectly complementary to the same. The expression "one or a few bases" means, for example, 1 to 3 bases, preferably 1 base or 2 bases.

In the first single-stranded nucleic acid molecule, the expression inhibitory sequence is contained in at least one of the region (Xc) and the region (X), as described above. The first single-stranded nucleic acid molecule may contain one expression inhibitory sequence, or two or more expression inhibitory sequences, for example.

In the latter case, the first single-stranded nucleic acid molecule may contain, for example: two or more identical expression inhibitory sequences for the same target gene; two or more different expression inhibitory sequences for the same target gene; or two or more different expression inhibitory sequences for different target genes. When the first single-stranded nucleic acid molecule contains two or more expression inhibitory sequences, the positions of the respective expression inhibitory sequences are not particularly limited, and they may be in one region or different regions selected from the region (X) and the region (Xc). When the first single-stranded nucleic acid molecule contains two or more expression inhibitory sequences for different target genes, the first single-stranded nucleic acid molecule can inhibit the expressions of two or more kinds of different target genes, for example.

Fig. 7 shows schematic views illustrating an example of the first single-stranded nucleic acid molecule. Fig. 7(A) is a schematic view showing the order of the respective regions in the single-stranded nucleic acid molecule, as an illustrative example. Fig. 7(B) is a schematic view showing the state where a double strand is formed in the single-stranded nucleic acid molecule. As shown in Fig. 7(B), in the single-stranded nucleic acid molecule, a double strand is formed between the region (Xc) and the region (X), and therefore, a short hairpin structure is formed. The schematic views shown in Fig. 7 merely illustrate the order in which the respective regions are linked and the positional relationship of the respective regions forming the double strand, and they do not limit the lengths of the respective regions, the shape of the linker region (Lx), and the like, for example.

In the first single-stranded nucleic acid molecule, the number of bases in each of the region (X) and the region (Xc) is not particularly limited. Examples of the lengths of the respective regions are given below. However, it is to be noted that the present invention is by no means limited thereto. In the present invention, "the number of bases" means the "length", for example, and it also can be referred to as the "base length". In the present invention, for example, the numerical range regarding the number of bases discloses all the positive integers falling within that range. For example, the description "1 to 4 bases" disclosed all of "1, 2, 3, and 4 bases" (the same applies hereinafter).

The region (Xc) may be perfectly complementary to the entire region of the region (X), for example. In this case, it means that, for example, the region (Xc) is composed of a base sequence complementary to the entire region extending from the 5' end to the 3' end of the region (X). In other words, it means that the region (Xc) has the same base length as the region (X), and all the bases in the region (Xc) are complementary to all the bases in the region (X).

Furthermore, the region (Xc) may be perfectly complementary to part of the region (X), for example. In this case, it means that, for example, the region (Xc) is composed of a base sequence complementary to the part of the region (X). In other words, it means that the region (Xc) is composed of a base sequence whose base length is shorter than the base length of the region (X) by one or more bases, and all the bases in the region (Xc) are complementary to all the bases in the part of the region (X). The part of the region (X) is preferably a region having a base sequence composed of successive bases starting from the base at the end (the 1st base) on the region (Xc) side in the region (X), for example.

In the first single-stranded nucleic acid molecule, the relationship between the number of bases (X) in the region (X) and the number of bases (Xc) in the region (Xc) satisfy the condition (3) or (5), for example. In the former case, specifically, the following condition (11) is satisfied, for example.

$$X > Xc \quad \cdots (3)$$

$$X-Xc = 1 - 10, \text{ preferably } 1, 2 \text{ or } 3, \text{ more preferably } 1 \text{ or } 2 \quad \cdots(11)$$

$$X=Xc \quad \cdots(5)$$

When the region (X) and/or the region (Xc) contains the expression inhibitory sequence, the region(s) may be a region composed of the expression inhibitory sequence only or a region containing the expression inhibitory sequence, for example. The number of bases in the expression inhibitory sequence is, for example, 19 to 30, preferably 19, 20, or 21. In the region(s) containing the expression inhibitory sequence, for example, the expression inhibitory sequence may further contain an additional sequence on its 5' side and/or 3' side. The number of bases in the additional sequence is, for example, 1 to 31, preferably 1 to 21, and more preferably 1 to 11.

The number of bases in the region (X) is not particularly limited. When the region (X) contains the expression inhibitory sequence, the lower limit of the number of bases in the region (X) is, for example, 19, and the upper limit of the same is, for example, 50, preferably 30, and more preferably 25. Specifically, the number of bases in the region (X) is, for example, 19 to 50, preferably 19 to 30, and more preferably 19 to 25.

The number of bases in the region (Xc) is not particularly limited. The lower limit of the number of bases in the region (Xc) is, for example, 19, preferably 20, and more preferably 21, and the upper limit of the same is, for example, 50, more preferably 40, and still more preferably 30.

In the single-stranded nucleic acid molecule, the length of the linker region (Lx) is not particularly limited. The length of the linker region (Lx) is preferably a length such that, for example, the region (X) and the region (Xc) can form a double strand.

The full length of the first single-stranded nucleic acid molecule is not particularly limited. In the first single-stranded nucleic acid molecule, the lower limit of the total number of bases (the number of bases in the full length single-stranded nucleic acid molecule) is, for example, 38, preferably 42, more preferably 50, still more preferably 51, and particularly preferably 52, and the upper limit of the same is, for example, 300, preferably 200, more preferably 150, still more preferably 100, and particularly preferably 80. In the first single-stranded nucleic acid molecule, the lower limit of the total number of bases excluding that in the linker region (Lx) is, for example, 38, preferably 42, more preferably 50, still more preferably 51, and particularly preferably 52, and the upper limit of the same is, for example, 300, preferably 200, more preferably 150, still more preferably 100, and particularly preferably 80.

(2) Second Single-Stranded Nucleic Acid Molecule

The second single-stranded nucleic acid molecule is a molecule that further contains a region (Y) and a region (Yc) that is complementary to the region (Y), in addition to the region (X), the linker region (Lx) and the region (Xc), for example. In the second single-stranded nucleic acid molecule, an inner region (Z) is composed of the region (X) and the region (Y) that are linked to each other. The description regarding the first single-stranded nucleic acid molecule also applies to the second single-stranded nucleic acid molecule, unless otherwise stated.

The second single-stranded nucleic acid molecule may contain, for example, the region (Xc), the linker region (Lx), the region (X), the region (Y) and the region (Yc) in this order from the 5' side to the 3' side. In this case, the region (Xc) also is referred to as a "5' side region (Xc)"; the region (X) in the inner region (Z) also is referred to as an "inner 5' side region (X)"; the region (Y) in the inner region (Z) also is referred to as an "inner 3' region (Y)"; and the region (Yc) also is referred to as a "3' side region (Yc)". Alternatively, the second single-stranded nucleic acid molecule may contain, for example, the region (Xc), the linker region (Lx), the region (X), the region (Y) and the region (Yc) in this order from the 3' side to the 5' side. In this case, the region (Xc) also is referred to as a "3' side region (Xc)"; the region (X) in the inner region (Z) also is referred to as an "inner 3' side region (X)"; the region (Y) in the inner region (Z) also is referred to as an "inner 5' region (Y)"; and the region (Yc) also is referred to as a "5' side region (Yc)".

As described above, the inner region (Z) is composed of the region (X) and the region (Y) that are linked to each other, for example. The region (X) and the region (Y) are linked directly to each other with no intervening sequence therebetween, for example. The inner region (Z) is defined as being "composed of the region (X) and the region (Y) that are linked to each other" merely to indicate the sequence context relative to the region (Xc) and the region (Yc). This definition does not intend to limit that, in the use of the single-stranded nucleic acid molecule, the region (X) and the region (Y) in the inner region (Z) are discrete independent regions. That is, for example, when the expression inhibitory sequence is contained in the inner region (Z), the expression inhibitory sequence may be arranged so as to extend across the region (X) and the region (Y) in the inner region (Z).

In the second single-stranded nucleic acid molecule, the region (Xc) is complementary to the region (X). It is

only necessary that the region (Xc) has a sequence complementary to the entire region or part of the region (X). Preferably, the region (Xc) contains or is composed of a sequence complementary to the entire region or part of the region (X). The region (Xc) may be perfectly complementary to the entire region or part of the region (X), or one or a few bases in the region (Xc) may be non-complementary to the same, for example. Preferably, the region (Xc) is perfectly complementary to the same. The expression "one or a few bases" means, for example, 1 to 3 bases, preferably 1 base or 2 bases.

In the second single-stranded nucleic acid molecule, the region (Yc) is complementary to the region (Y). It is only necessary that the region (Yc) has a sequence complementary to the entire region or part of the region (Y). Preferably, the region (Yc) contains or is composed of a sequence complementary to the entire region or part of the region (Y). The region (Yc) may be perfectly complementary to the entire region or part of the region (Y), or one or a few bases in the region (Yc) may be non-complementary to the same, for example. Preferably, the region (Yc) is perfectly complementary to the same. The expression "one or a few bases" means, for example, 1 to 3 bases, preferably 1 base or 2 bases.

In the second single-stranded nucleic acid molecule, the expression inhibitory sequence is contained in at least one of the inner region (Z), which is composed of the region (X) and the region (Y), and the region (Xc), for example. Furthermore, the expression inhibitory sequence may also be contained in the region (Yc). When the inner region (Z) contains the expression inhibitory sequence, the expression inhibitory sequence may be contained in either of the region (X) and the region (Y), or the expression inhibitory sequence may be contained so as to extend across the region (X) and the region (Y), for example. The second single-stranded nucleic acid molecule may contain one expression inhibitory sequence, or two or more expression inhibitory sequences, for example.

When the second single-stranded nucleic acid molecule contains two or more expression inhibitory sequences, the positions of the respective expression inhibitory sequences are not particularly limited. They may be in either one of the inner region (Z) and the region (Xc), or may be in one of the inner region (Z) and the region (Xc), and any region other than these regions.

In the second single-stranded nucleic acid molecule, the region (Yc) and the region (Y) may be linked to each other either directly or indirectly, for example. In the former case, the region (Yc) and the region (Y) may be linked directly by phosphodiester linkage or the like, for example. In the latter case, the second single-stranded nucleic acid molecule may be configured so that it contains a linker region (Ly) between the region (Yc) and the region (Y), and the region (Yc) and the region (Y) are linked via the linker region (Ly), for example.

When the second single-stranded nucleic acid molecule contains the linker region (Ly), the building block of the linker region (Ly) is not prticularly limited, and may be a linker composed of the nucleotide residue(s), or a linker having a structure represented by the above-mentioned formula (IL), for example. In the latter case, all the descriptions regarding the formula (IL) stated above in connection with the linker region (Lx) also apply to the linker region (Ly).

Furthermore, the linker region (Ly) may contain at least one selected from the group consisting of an amino acid residue, a polyamine residue and a polycarboxylic acid residue. The linker region may or may not contain a residue other than the amino acid residue, polyamine residue and polycarboxylic acid residue. For example, the linker region may contain any of a polycarboxylic acid residue, a terephthalic acid residue and an amino acid residue.

In the present invention, the "polyamine" means any compound containing a plurality of (two, three or more) amino groups. The "amino group" is not limited to an $-NH_2$ group and also includes an imino group (-NH-). In the present invention, the polyamine is not particularly limited, and examples thereof include 1,4-diaminobenzene, 1,3-diaminobenzene, 1,2-diaminobenzene and the like. In the present invention, moreover, the "polycarboxylic acid" means any compound containing a plurality of (two, three or more) carboxy groups. In the present invention, the polycarboxylic acid is not particularly limited, and examples thereof include 1,4-dicarboxybenzene (terephthalic acid), 1,3-dicarboxybenzene (isophthalic acid), 1,2-dicarboxybenzene (phthalic acid) and the like. In the present invention, moreover, the "amino acid" means any organic compound containing one or more amino groups and one or more carboxy groups in a molecule. The "amino group" is not limited to an $-NH_2$ group and also includes an imino group (-NH-). For example, proline, hydroxyproline and the like contain an imino group (-NH-), but not contain an $-NH_2$ group in the molecule, which are included in the definition of the "amino acid" in the present invention. In the present invention, the "amino acid" may be a natural amino acid or an artificial amino acid.

In the single-stranded nucleic acid molecule, the amino acid residue may be a plurality of interlinked amino acid residues. In the present invention, the amino acid residue that is a plurality of interlinked amino acid residues is, for example, a residue containing a peptide structure, more specifically, for example, a structure such as glycylglycine (Gly-Gly) .

In the single-stranded nucleic acid molecule, the amino acid residue may be a glycine residue, a terephthalamido residue, a proline residue or a lysine residue. Also, the amino acid residue may be a modified amino acid residue or an amino acid derivative.

In preferable embodiment, the linker region (Ly) may be a linker region described in WO 2012/017919 pamphlet, WO 2013/103146 pamphlet, WO 2012/005368 pamphlet, WO 2013/077446 pamphlet, WO 2013/133393 pamphlet, WO 2016/108264 pamphlet, WO 2016/104775 pamphlet and the like, and in the present invention, these doduments can be incorporated herein by reference.

More specific examples thereof include linker regions having the following structure described in WO 2016/104775 pamphlet.

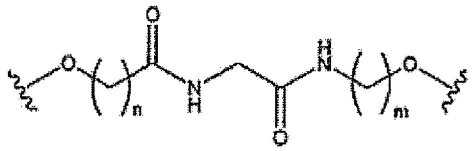

(I-1)

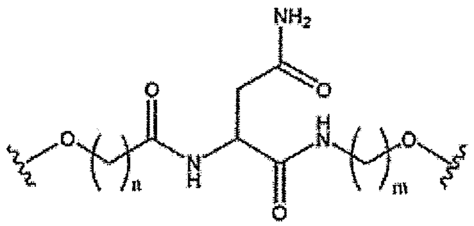

(I-2)

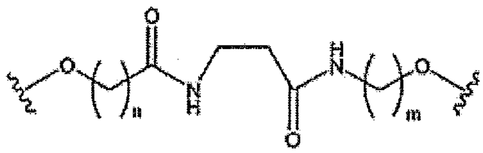

(I-3)

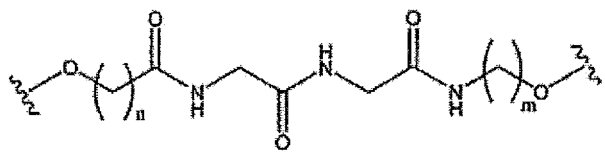

(I-4)

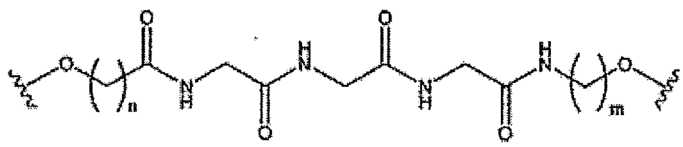

(I-5)

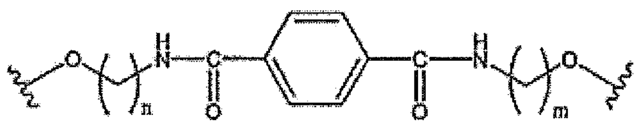

(I-6)

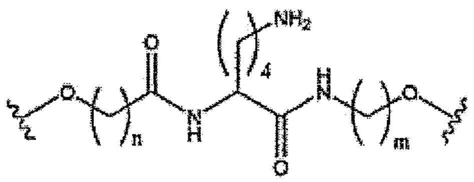

(I−7)

In the chemical formulas (I-1) to (1-7), n and m are not particularly limited, for example, m is an integer of 0 to 30, and n is an integer of 0 to 30. Specific examples thereof include n=11 and m=12 or n=5 and m=4 in the chemical formula (I-1), n=5 and m=4 in the chemical formula (1-4), n=4 and m=4 in the chemical formula (1-6), and n=5 and m=4 in the chemical formula (1-7). The structures are shown by the following chemical formulas (I-1a), (I-1b), (I-4a), (I-6a) and (I-7a).

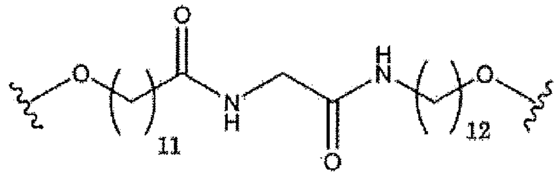

(I−1 a)

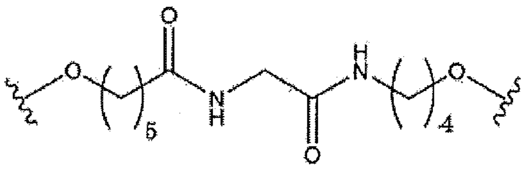

(I−1 b)

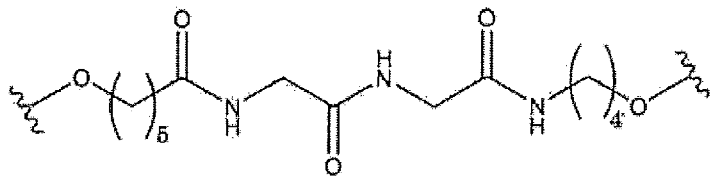

(I−4 a)

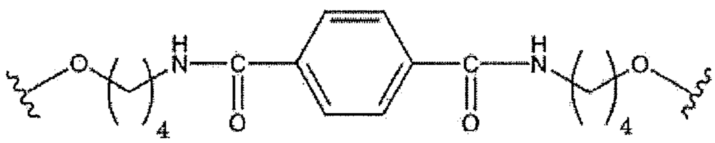

(I−6 a)

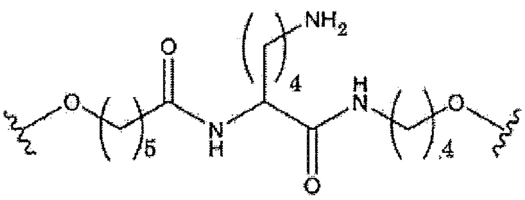

(I−7 a)

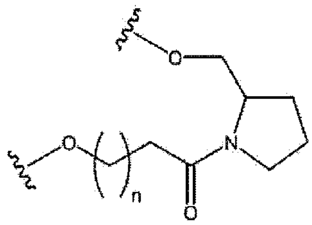

· · · (II-1)

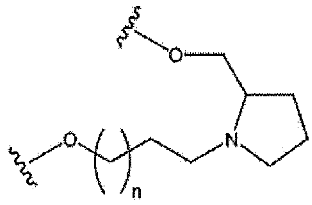

· · · (II-2)

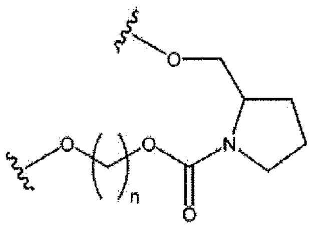

· · · (II-3)

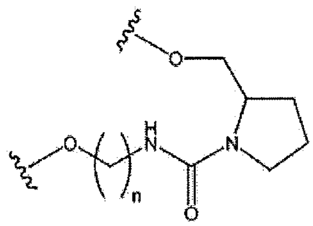

· · · (II-4)

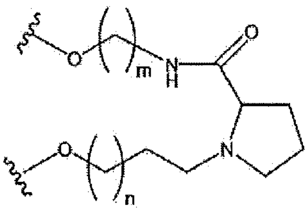

· · · (II-5)

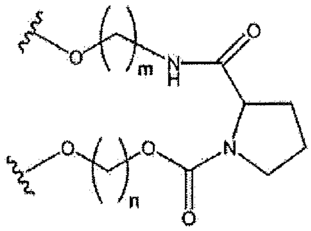

· · · (II-6)

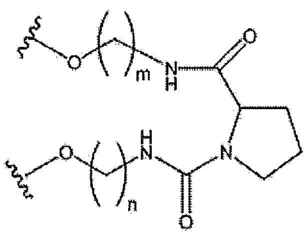

· · · (II-7)

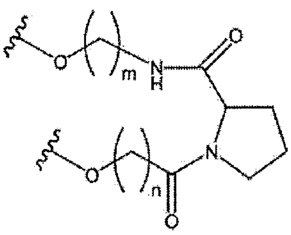

· · · (II-8)

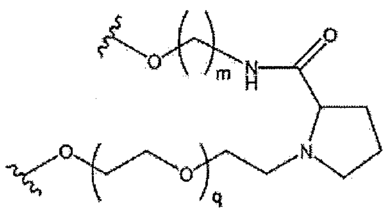

· · · (II-9)

In the formulas (II-1) to (II-9), n, m and q are not particularly limited, for example, m is an integer of 0 to 30, n is an integer of 0 to 30, and q is an integer of 0 to 10. Specific examples thereof include n=8 in the formula (II-1), n=3 in the formula (II-2), n=4 or 8 in the formula (II-3), n=7 or 8 in the formula (II-4), n=3 and m=4 in the formula (II-5), n=8 and m=4 in the formula (II-6), n=8 and m=4 in the formula (II-7), n=5 and m=4 in the formula (11-8), and q=1 and m=4 in the formula (II-9) . The example (n=8) in the formula (II-4) is shown by the following formula (II-4a), and the example (n=5, m=4) in the formula (II-8) is shown by the following formula (II-8a) .

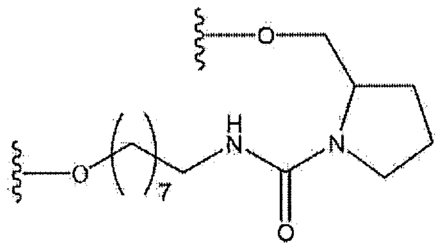

・・・(II-4a)

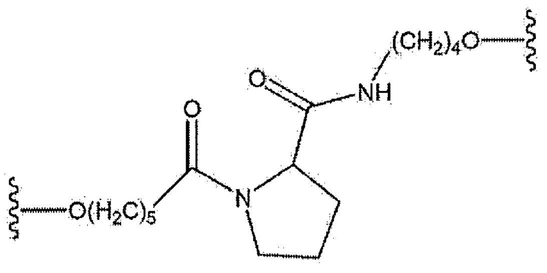

・・・(II-8a)

Either of the terminal nucleotide residues of the region (Yc), and either of the terminal nucleotide residues of the region (Y) are each bonded to, for example, the linker region (Ly) via a phosphodiester bond.

Fig. 8 shows schematic views illustrating an example of the second single-stranded nucleic acid molecule containing the linker region (Ly). Fig. 8(A) is a schematic view showing the order of the respective regions from the 5' side to the 3' side in the single-stranded nucleic acid molecule, as an illustrative example. Fig. 8(B) is a schematic view showing the state where double strands are formed in the single-stranded nucleic acid molecule. As shown in Fig. 8(B), in the single-stranded nucleic acid molecule, double strands are formed between the region (Xc) and the region (X) and between the region (Y) and the region (Yc), and therefore, fold-back structures are formed at both ends. The schematic views shown in Fig. 8 merely illustrates the order in which the respective regions are linked and the positional relationship of the respective regions forming the double strand, and they do not limit the lengths of the respective regions, the shape of the linker region, and the like, for example. Furthermore, although the region (Xc) is shown on the 5' side in Fig. 8, the position of the region (Xc) is not limited thereto, and the region (Xc) may be on the 3' side.

In the second single-stranded nucleic acid molecule, the number of bases in each of the region (Xc), the region (X), the region (Y) and the region (Yc) is not particularly limited. Examples of the lengths of the respective regions are given below. It is to be noted, however, that the present invention is by no means limited thereto.

As described above, the region (Xc) may be complementary to the entire region of the region (X), for example. In this case, it is preferable that, for example, the region (Xc) has the same base length as the region (X), and is composed of a base sequence complementary to the entire region of the region (X). It is more preferable that the region (Xc) has the same base length as the region (X), and all the bases in the region (Xc) are complementary to all the bases in the region (X), i.e., the region (Xc) is perfectly complementary to the region (X), for example. It is to be noted, however, that the configuration of the region (Xc) is not limited thereto, and one or a few bases in the region (Xc) may be non-complementary to the corresponding bases in the region (X), for example, as described above.

Furthermore, as described above, the region (Xc) may be complementary to part of the region (X), for example. In this case, it is preferable that, for example, the region (Xc) has the same base length as the part of the region (X), i.e., the region (Xc) is composed of a base sequence whose base length is shorter than the base length of the region (X) by one or more bases. It is more preferable that the region (Xc) has the same base length as the part of the region (X) and all the bases in the region (Xc) are complementary to all the bases in the part of the region (X), i.e., the region (Xc) is perfectly complementary to the part of the region (X), for example. The part of the region (X) is preferably a region having a base sequence composed of successive bases starting from the base at the end (the 1st base) on the region (Xc) side in the region (X), for example.

As described above, the region (Yc) may be complementary to the entire region of the region (Y), for example. In this case, it is preferable that, for example, the region (Yc) has the same base length as the region (Y), and is composed of a base sequence complementary to the entire region of the region (Y). It is more preferable that the region (Yc) has the same base length as the region (Y), and all the bases in the region (Yc) are complementary to all the bases in the region (Y), i.e., the region (Yc) is perfectly complementary to the region (Y), for example. It is to be noted, however, that the configuration of the region (Yc) is not limited thereto, and one or a few bases in the region (Yc) may be non-complementary to the corresponding bases in the region (Y), for example, as described above.

Furthermore, as described above, the region (Yc) may be complementary to part of the region (Y), for example.

In this case, it is preferable that, for example, the region (Yc) has the same base length as the part of the region (Y), i.e., the region (Yc) is composed of a base sequence whose base length is shorter than the base length of the region (Y) by one or more bases. It is more preferable that the region (Yc) has the same base length as the part of the region (Y) and all the bases in the region (Yc) are complementary to all the bases in the part of the region (Y), i.e., the region (Yc) is perfectly complementary to the part of the region (Y), for example. The part of the region (Y) is preferably a region having a base sequence composed of successive bases starting from the base at the end (the 1st base) on the region (Yc) side in the region (Y), for example.

In the second single-stranded nucleic acid molecule, the relationship of the number of bases (Z) in the inner region (Z) with the number of bases (X) in the region (X) and the number of bases (Y) in the region (Y), and the relationship of the number of bases (Z) in the inner region (Z) with the number of bases (X) in the region (X) and the number of bases (Xc) in the region (Xc) satisfy the conditions of Expressions (1) and (2), for example.

$$Z=X+Y \quad \cdots (1)$$

$$Z \geqq Xc+Yc \quad \cdots (2)$$

In the second single-stranded nucleic acid molecule, the relationship between the number of bases (X) in the region (X) and the number of bases (Y) in the region (Y) is not particularly limited, and satisfy any of the conditions of the following expressions, for example.

$$X=Y \quad \cdots (19)$$

$$X<Y \quad \cdots (20)$$

$$X>Y \quad \cdots (21)$$

In the second single-stranded nucleic acid molecule, the relationship between the number of bases (X) in the region (X) and the number of bases (Xc) in the region (Xc), and the relationship between the number of bases (Y) in the region (Y) and the number of bases (Yc) in the region (Yc) satisfy any of the following conditions (a) to (d), for example.

(a) Conditions of Expressions (3) and (4) are satisfied.

$$X>Xc \quad \cdots (3)$$

$$Y=Yc \quad \cdots (4)$$

(b) Conditions of Expressions (5) and (6) are satisfied.

$$X=Xc \quad \cdots (5)$$

$$Y>Yc \quad \cdots (6)$$

(c) Conditions of Expressions (7) and (8) are satisfied.

$$X>Xc \quad \cdots (7)$$

$$Y>Yc \quad \cdots (8)$$

(d) Conditions of Expressions (9) and (10) are satisfied.

$$X=Xc \quad \cdots(9)$$

$$Y=Yc \quad \cdots(10)$$

In the above-described conditions (a) to (d), the difference between the number of bases (X) in the region (X) and the number of bases (Xc) in the region (Xc), and the difference between the number of bases (Y) in the region (Y) and the number of bases (Yc) in the region (Yc) preferably satisfy the following conditions (a) to (d), for example.

(a) Conditions of Expressions (11) and (12) are satisfied.

$$X-Xc=1 \text{ to } 10, \text{ preferably } 1, 2, 3 \text{ or } 4, \text{ more preferably } 1, 2 \text{ or } 3 \quad \cdots(11)$$

$$Y-Yc=0 \quad \cdots(12)$$

(b) Conditions of Expressions (13) and (14) are satisfied.

$$X-Xc=0 \quad \cdots(13)$$

$$Y-Yc=1 \text{ to } 10, \text{ preferably } 1, 2, 3 \text{ or } 4, \text{ more preferably } 1, 2 \text{ or } 3 \quad \cdots(14)$$

(c) Conditions of Expressions (15) and (16) are satisfied.

$$X-Xc=1 \text{ to } 10, \text{ preferably } 1, 2 \text{ or } 3, \text{ more preferably } 1 \text{ or } 2 \quad \cdots(15)$$

$$Y-Yc=1 \text{ to } 10, \text{ preferably } 1, 2 \text{ or } 3, \text{ more preferably } 1 \text{ or } 2 \quad \cdots(16)$$

(d) Conditions of Expressions (17) and (18) are satisfied.

$$X-Xc=0 \quad \cdots(17)$$

$$Y-Yc=0 \quad \cdots(18)$$

Regarding the second single-stranded nucleic acid molecules satisfying the conditions (a) to (d), examples of their structures are shown respectively in the schematic views of Fig. 9. Fig. 9 shows the single-stranded nucleic acid molecules containing the linker region (Lx) and the linker region (Ly). Fig. 9(A) shows an example of the single-stranded nucleic acid molecule satisfying the condition (a); Fig. 9(B) shows an example of the single-stranded nucleic acid molecule satisfying the condition (b); Fig. 9(C) shows an example of the single-stranded nucleic acid molecule satisfying the condition (c); and Fig. 9(D) shows an example of the single-stranded nucleic acid molecule satisfying the condition (d). In Fig. 9, dotted lines indicate a state where double strands are formed by self-annealing. The single-stranded nucleic

acid molecules shown in Fig. 9 are all directed to examples where the relationship between the number of bases (X) in the region (X) and the number of bases (Y) in the region (Y) satisfy "X < Y" of Expression (20). It is to be noted, however, that the relationship is not limited thereto, and "X = Y" of Expression (19) or "X > Y" of Expression (21) may be satisfied. The schematic views shown in Fig. 9 merely illustrate the relationship between the regions (X) and (Xc) and the relationship between the region (Y) and the region (Yc), and they do not limit the length and the shape of each region, and the presence or absence of the linker region (Ly), for example.

Each of the single-stranded nucleic acid molecules satisfying the conditions (a) to (c) is configured so that, for example, when the double strands are formed by the region (Xc) and the region (X) and by the region (Yc) and the region (Y), respectively, the inner region (Z) contains a base that cannot be aligned with either of the regions (Xc) and (Yc), i.e., a base that cannot form the double strand. In the inner region (Z), the base that is not aligned (a base that does not form the double strand) hereinafter also is referred to as a "unpaired base". In Fig. 9, a region composed of the unpaired base(s) is shown as "F". The number of bases in the region (F) is not particularly limited. The number of bases (F) in the region (F) is as follows, for example: the number of bases represented by "Xc - X" in the case of the single-stranded nucleic acid molecule satisfying the condition (a); the number of bases represented by "Y -Yc" in the case of the the single-stranded nucleic acid molecule satisfying the condition (b); and the total of the number of bases represented by "Xc - X" and the number of bases represented by "Y - Yc" in the case of the single-stranded nucleic acid molecule satisfying the condition (c).

On the other hand, the single-stranded nucleic acid molecule satisfying the condition (d) is configured so that, for example, the entire region of the inner region (Z) is aligned with the regions (Xc) and (Yc), in other words, the entire region of the inner region (Z) forms a double strand. In the single-stranded nucleic acid molecule satisfying the condition (d), the 5' end of the region (Xc) and the 3' end of the region (Yc) are not linked to each other.

The total number of the bases in the region (Xc), the bases in the region (Yc), and the number of the unpaired bases (F) in the inner region (Z) is equal to the number of the bases in the inner region (Z). Thus, the length of the region (Xc) and the length of the region (Yc) can be determined as appropriate depending on the length of the inner region (Z), the number of the unpaired bases, and the positions of the unpaired bases, for example.

The number of the bases in the inner region (Z) is 19 or more, for example. The lower limit of the number of the bases is, for example, 19, preferably 20, and more preferably 21. The upper limit of the number of the bases is, for example, 50, preferably 40, and more preferably 30. A specific example of the number of the bases in the inner region (Z) is 19, 20, 21, 22, 23, 24, 25, 26, 27, 28, 29, or 30. When the inner region (Z) contains the expression inhibitory sequence, the above condition is preferable, for example.

When the inner region (Z) contains the expression inhibitory sequence, the inner region (Z) may be a region composed of the expression inhibitory sequence only or a region containing the expression inhibitory sequence, for example. The number of bases of the expression inhibitory sequence is, for example, 19 to 30, preferably 19, 20, or 21. When the inner region (Z) contains the expression inhibitory sequence, the expression inhibitory sequence may further contain an additional sequence on its 5' side and/or 3' side. The number of bases in the additional sequence is, for example, 1 to 31, preferably 1 to 21, more preferably 1 to 11, and still more preferably 1 to 7.

The number of bases in the region (Xc) is, for example, 1 to 29, preferably 1 to 11, more preferably 1 to 7, still more preferably 1 to 4, and particularly preferably 1, 2, or 3. When the inner region (Z) or the region (Yc) contains the expression inhibitory sequence, the number of bases as described above is preferable, for example. A specific example is as follows: when the number of bases in the inner region (Z) is 19 to 30 (e.g., 19), the number of bases in the region (Xc) is, for example, 1 to 11, preferably 1 to 7, more preferably 1 to 4, and still more preferably 1, 2, or 3.

When the region (Xc) contains the expression inhibitory sequence, the region (Xc) may be a region composed of the expression inhibitory sequence only or a region containing the expression inhibitory sequence, for example. The length of the expression inhibitory sequence is as described above, for example. When the region (Xc) contains the expression inhibitory sequence, the expression inhibitory sequence may further contain an additional sequence on its 5' side and/or 3' side. The number of bases in the additional sequence is, for example, 1 to 11, preferably 1 to 7.

The number of bases in the region (Yc) is, for example, 1 to 29, preferably 1 to 11, more preferably 1 to 7, still more preferably 1 to 4, and particularly preferably 1, 2, or 3. When the inner region (Z) or the region (Xc) contains the expression inhibitory sequence, the number of bases as described above is preferable, for example. A specific example is as follows: when the number of bases in the inner region (Z) is 19 to 30 (e.g., 19), the number of bases in the region (Yc) is, for example, 1 to 11, preferably 1 to 7, more preferably 1, 2, 3, or 4, and still more preferably 1, 2, or 3.

When the region (Yc) contains the expression inhibitory sequence, the region (Yc) may be a region composed of the expression inhibitory sequence only or a region containing the expression inhibitory sequence, for example. The length of the expression inhibitory sequence is as described above, for example. When the region (Yc) contains the expression inhibitory sequence, the expression inhibitory sequence may further contain an additional sequence on its 5' side and/or 3' side. The number of bases in the additional sequence is, for example, 1 to 11, preferably 1 to 7.

As described above, the relationship among the number of bases in the inner region (Z), the number of bases in the region (Xc), and the number of bases in the region (Yc) can be expressed by Expression (2): "$Z \geq Xc + Yc$", for

example. Specifically, the number of bases represented by "Xc + Yc" is equal to the number of bases in the inner region (Z), or lower than the number of bases in the inner region (Z), for example. In the latter case, "Z - (Xc + Yc)" is, for example, 1 to 10, preferably 1 to 4, and more preferably 1, 2, or 3. The "Z - (Xc + Yc)" corresponds to the number of bases (F) in the unpaired base region (F) in the inner region (Z), for example.

In the second single-stranded nucleic acid molecule, the lengths of the linker region (Lx) and the linker region (Ly) are not particularly limited. The linker region (Lx) is as described above. When the building blocks of the linker region (Ly) contain a base(s), the lower limit of the number of bases in the linker region (Ly) is, for example, 1, preferably 2, and more preferably 3, and the upper limit of the same is, for example, 100, preferably 80, and more preferably 50. The number of bases in each of the linker regions is specifically 1 to 50, 1 to 30, 1 to 20, 1 to 10, 1 to 7, or 1 to 4, for example, but it is not limited to these illustrative examples.

The linker region (Lx) may be the same as or different from the linker region (Ly), for example.

The full length of the second single-stranded nucleic acid molecule is not particularly limited. In the second single-stranded nucleic acid molecule, the lower limit of the total number of bases (the number of bases in the full length single-stranded nucleic acid molecule), is, for example, 38, preferably 42, more preferably 50, still more preferably 51, and particularly preferably 52, and the upper limit of the same is, for example, 300, preferably 200, more preferably 150, still more preferably 100, and particularly preferably 80. In the second single-stranded nucleic acid molecule, the lower limit of the total number of bases excluding those in the linker region (Lx) and the linker region (Ly) is, for example, 38, preferably 42, more preferably 50, still more preferably 51, and particularly preferably 52, and the upper limit of the same is, for example, 300, preferably 200, more preferably 150, still more preferably 100, and particularly preferably 80.

In the single-stranded nucleic acid molecule of the present invention, it is only necessary that the linker region (Lx) has the structure represented by the formula (IL), as described above, and other building blocks are not particularly limited. Examples of the building blocks include nucleotide residues. Examples of the nucleotide residues include a ribonucleotide residue and a deoxyribonucleotide residue. The nucleotide residue may be the one that is not modified (unmodified nucleotide residue) or the one that is modified (modified nucleotide residue), for example. By configuring the single-stranded nucleic acid molecule of the present invention so as to contain a modified nucleotide residue, for example, the resistance of the single-stranded nucleic acid molecule to nuclease can be improved, thereby allowing the stability of the single-stranded nucleic acid molecule to be improved. Furthermore, the single-stranded nucleic acid molecule of the present invention may further contain, for example, a non-nucleotide residue in addition to the nucleotide residue.

The nucleotide residue is preferable as the building block of each of the regions (Xc), (X), (Y), and (Yc). Each of the regions is composed of any of the following residues (1) to (3), for example.

(1) an unmodified nucleotide residue(s)
(2) a modified nucleotide residue(s)
(3) an unmodified nucleotide residue(s) and a modified nucleotide residue(s)

The linker region (Lx) is composed of the non-nucleotide residue(s) only.

The building blocks of the linker region (Ly) are not particularly limited, and examples thereof include the nucleotide residues and the non-nucleotide residues, as described above. Each of the linker regions may be composed of the nucleotide residue(s) only, the non-nucleotide residue(s) only, or both the nucleotide residue(s) and the non-nucleotide residue(s). The linker region (Ly) is composed of any of the following residues (1) to (7), for example.

(1) an unmodified nucleotide residue(s)
(2) a modified nucleotide residue(s)
(3) an unmodified nucleotide residue(s) and a modified nucleotide residue(s)
(4) a non-nudeotide residue(s)
(5) a non-nucleotide residue(s) and an unmodified nucleotide residue(s)
(6) a non-nucleotide residue(s) and a modified nucleotide residue(s)
(7) a non-nucleoticle residue(s), an unmodified nucleotide residue(s), and a modified nucleotide residue

The single-stranded nucleic acid molecule of the present invention may be, for example: a molecule composed of the nucleotide residues only except for its linker region (Lx); a molecule containing the non-nucleotide residue(s) in addition to the nucleotide residues; or the like. In the single-stranded nucleic acid molecule of the present invention, the nucleotide residues may be the unmodified nucleotide residues only; the modified nucleotide residues only; or both the unmodified nucleotide residue(s) and the modified nucleotide residue(s), as described above, for example. When the single-stranded nucleic acid molecule contains both the unmodified nucleotide residue(s) and the modified nucleotide residue(s), the number of the modified nucleotide residue(s) is not particularly limited, and is, for example, "one or more", specifically, for example, 1 to 5, preferably 1 to 4, more preferably 1 to 3, and most preferably 1 or 2. When the single-stranded nucleic acid molecule of the present invention contains the non-nucleotide residue(s), the number of the non-nucleotide residue(s) is not particularly limited, and is, for example, "one or more", specifically, for example, 1 or 2.

In the single-stranded nucleic acid molecule of the present invention, the nucleotide residue is preferably a ribonucleotide residue, for example. In this case, the single-stranded nucleic acid molecule of the present invention is referred to as an "single-stranded RNA molecule", for example. The ssRNA molecule may be, for example: a molecule composed of the ribonucleotide residues only except for its linker region (Lx); a molecule containing the non-nucleotide residue(s) in addition to the ribonucleotide residues; or the like. As described above, as the ribonucleotide residues, the ssRNA molecule may contain: the unmodified ribonucleotide residues only; modified ribonucleotide residues only; or both the unmodified ribonucleotide residue(s) and the modified ribonucleotide residue(s), for example.

When the ssRNA molecule contains the modified ribonucleotide residue(s) in addition to the unmodified ribonucleotide residues, for example, the number of the modified ribonucleotide residue(s) is not particularly limited, and is, for example, "one or more", specifically, for example, 1 to 5, preferably 1 to 4, more preferably 1 to 3, and most preferably 1 or 2. Examples of the modified ribonucleotide residue as contrasted to the unmodified ribonucleotide residue include the deoxyribonucleotide residue obtained by substituting a ribose residue with a deoxyribose residue. When the ssRNA molecule contains the deoxyribonucleotide residue(s) in addition to the unmodified ribonucleotide residue(s), for example, the number of the deoxyribonucleotide residue(s) is not particularly limited, and is, for example, "one or more", specifically, for example, 1 to 5, preferably 1 to 4, more preferably 1 to 3, and most preferably 1 or 2.

The single-stranded nucleic acid molecule of the present invention may contains a labeling substance (marker), and may be labeled with the labeling substance, for example. The labeling substance is not particularly limited, and may be a fluorescent substance, a dye, an isotope, or the like, for example. Examples of the fluorescent substance include: fluorophores such as pyrene, TAMRA, fluorescein, a Cy3 dye, and a Cy5 dye. Examples of the dye include Alexa dyes such as Alexa 488. Examples of the isotope include stable isotopes and radioisotopes. Among them, stable isotopes are preferable. Stable isotopes have a low risk of radiation exposure, and they require no dedicated facilities, for example. Thus, stable isotopes are excellent in handleability and can contribute to cost reduction. Moreover, a stable isotope does not change the physical properties of a compound labeled therewith, for example, and thus has an excellent property as a tracer. The stable isotope is not particularly limited, and examples thereof include $^{2}H$, $^{13}C$, $^{15}N$, $^{17}O$, $^{18}O$, $^{33}S$, $^{34}S$ and $^{36}S$ and the like.

In the single-stranded nucleic acid molecule of the present invention, as described above, it is preferable to introduce the labeling substance into the non-nucleotide structure, more preferably to the non-nucleotide residue(s) in the linker region (Lx), for example. Introduction of the labeling substance to the non-nucleotide residue(s) can be carried out easily and at low cost, for example.

As described above, the single-stranded nucleic acid molecule of the present invention can inhibit expression of a target gene. Thus, the single-stranded nucleic acid molecule of the present invention can be used as a therapeutic agent for treating a disease caused by a gene, for example. According to the single-stranded nucleic acid molecule containing, as the expression inhibitory sequence, a sequence that inhibits expression of a gene causing the disease, for example, it is possible to treat the disease by inhibiting the expression of the target gene. In the present invention, the term "treatment" encompasses: prevention of diseases; improvement of diseases; and improvement in prognosis, for example, and it can mean any of them.

The method of using the single-stranded nucleic acid molecule of the present invention is not particularly limited. For example, the single-stranded nucleic acid molecule may be administered to a subject having the target gene.

Examples of the subject to which the single-stranded nucleic acid molecule of the present invention is administered include cells, tissues, and organs. Examples of the subject also include humans and nonhuman animals such as nonhuman mammals, i.e., mammals excluding humans. The administration may be performed in vivo or in vitro, for example. The cells are not particularly limited, and examples thereof include: various cultured cells such as HeLa cells, 293 cells, NIH3T3 cells, and COS cells; stem cells such as ES cells and hematopoietic stem cells; and cells isolated from living organisms, such as primary cultured cells.

In the present invention, the target gene whose expression is to be inhibited is not particularly limited, and any desired gene can be set as the target gene. The expression inhibitory sequence may be designed as appropriate depending on the kind of the target gene.

Specific examples of the single-stranded nucleic acid molecule of the present invention will be given below. It is to be noted, however, that the present invention is by no means limited thereto. When the target gene is TGF-β1, examples of the base sequence of the single-stranded nucleic acid molecule include the base sequence of SEQ ID NO: 1. When the target gene is a prorenin receptor, examples of the base sequence of the single-stranded nucleic acid molecule include the base sequence of SEQ ID NO: 2. When the target gene is mir-29b, examples of the base sequence of the single-stranded nucleic acid molecule include the base sequence of SEQ ID NO: 3. Furthermore, the base sequence may be, for example, base sequences obtained by, for example, deletion, substitution, and/or addition of one or more bases in these base sequences.

prorenin receptor 5'- UUAUAUGCAAGGUUAUAGGGA -3'(SEQ ID NO: 2)
miR-29b 5'-UAGCACCAUUUGAAAUCAGUGUU-3'(EQ ID NO: 3)

As to the use of the single-stranded nucleic acid molecule of the present invention, the following descriptions regarding the composition, the expression inhibitory method, the treatment method, and the like according to the present invention can be referred to.

Since the single-stranded nucleic acid molecule of the present invention can inhibit expression of a target gene as described above, it is useful as a pharmaceutical, a diagnostic agent, an agricultural chemical, and a tool for conducting research on agricultural chemicals, medical science, life science, and the like, for example.

2. Nucleotide Residue

The nucleotide residue composing the single-stranded nucleic acid molecule of the present invention is as described above, and it may be, for example, a ribonucleotide residue or a deoxyribonucleotide residue. The ribonucleotide residue has, for example: a ribose residue as the sugar; and adenine (A), guanine (G), cytosine (C), or uracil (U) as the base. The deoxyribose residue has, for example: a deoxyribose residue as the sugar; and adenine (A), guanine (G), cytosine (C), or thymine (T) as the base.

The nucleotide residue may be, for example, an unmodified nucleotide residue or a modified nucleotide residue. The building blocks of the unmodified nucleotide residue are the same or substantially the same as the building blocks of a naturally-occurring nucleotide residue, for example. Preferably, the building blocks are the same or substantially the same as the building blocks of a nucleotide residue occurring naturally in a human body.

The modified nucleotide residue is a nucleotide residue obtained by modifying the unmodified nucleotide residue, for example. The modified nucleotide may be such that any of the building blocks of the unmodified nucleotide residue is modified, for example. In the present invention, "modification" means, for example: substitution, addition, and/or deletion of any of the building blocks; and substitution, addition, and/or deletion of an atom(s) and/or a functional group(s) in the building block(s). It also can be referred to as "alteration". Examples of the modified nucleotide residue include naturally-occurring nucleotide residues and artificially-modified nucleotide residues. Regarding the naturally-derived modified nucleotide residues, Limbach et al. (1994, Summary: the modified nucleosides of RNA, Nucleic Acids Res. 22: pp. 2183 to 2196) can be referred to, for example. The modified nucleotide residue may be a residue of an alternative of the nucleotide, for example.

Examples of the modification of the nucleotide residue include modification of a ribose-phosphate backbone (hereinafter referred to as a "ribophosphate backbone").

In the ribophosphate backbone, a ribose residue may be modified, for example. In the ribose residue, for example, the 2'-position carbon can be modified. Specifically, a hydroxyl group bonded to the 2'-position carbon can be substituted with hydrogen, fluoro, or the like, for example. By substituting the hydroxyl group bonded to the 2'-position carbon with hydrogen, it is possible to substitute the ribose residue with deoxyribose. The ribose residue can be replaced with its stereoisomer, for example, and may be replaced with an arabinose residue, for example.

The ribophosphate backbone may be replaced with a non-ribophosphate backbone having a non-ribose residue and/or a non-phosphate, for example. The non-ribophosphate backbone may be, for example, the ribophosphate backbone modified so as to be uncharged, or the like. Examples of an alternative obtained by replacing the ribophosphate backbone with the non-ribophosphate backbone in the nucleotide include morpholino, cyclobutyl, and pyrrolidine. Other examples of the alternative include artificial nucleic acid monomer residues. Specific examples thereof include PNA (Peptide Nucleic Acid), LNA (Locked Nucleic Acid), and ENAs (2'-O,4'-C-Ethylenebridged Nucleic Acids). Among them, PNA is preferable.

In the ribophosphate backbone, a phosphate group can be modified, for example. In the ribophosphate backbone, a phosphate group in the closest proximity to the sugar residue is called an "a-phosphate group". The α-phosphate group is charged negatively, and the electric charges are distributed evenly over two oxygen atoms that are not linked to the sugar residue. Among the four oxygen atoms in the α-phosphate group, the two oxygen atoms not linked to the sugar residue in the phosphodiester linkage between the nucleotide residues hereinafter are referred to as "non-linking oxygens". On the other hand, two oxygen atoms that are linked to the sugar residue in the phosphodiester linkage between the nucleotide residues hereinafter are referred to as "linking oxygens". The α-phosphate group is preferably modified so as to be uncharged, or so as to render the charge distribution between the non-linking atoms asymmetric, for example.

In the phosphate group, the non-linking oxygen(s) may be replaced, for example. The oxygen(s) can be replaced with any atom selected from S (sulfur), Se (selenium), B (boron), C (carbon), H (hydrogen), N (nitrogen), and OR (R is an alkyl group or an aryl group, for example), for example, and replacement with S is preferable. It is preferable that both the non-linking oxygens are replaced, for example, and it is more preferable that both the non-linking oxygens are replaced with S. Examples of the thus-modified phosphate group include phosphorothioates, phosphorodithioates, phosphoroselenates, boranophosphates, boranophosphate esters, hydrogenphosphonates, phosphoroamidates, alkyl or aryl phosphonates, and phosphotriesters. In particular, phosphorodithioate in which both of the two non-linking oxygens are replaced with S is preferable.

In the phosphate group, the linking oxygen(s) may be replaced, for example. The oxygen(s) can be replaced with any atom selected from S (sulfur), C (carbon), and N (nitrogen), for example. Examples of the thus-modified phosphate group include: bridged phosphoroamidates resulting from the replacement with N; bridged phosphorothioates resulting from the replacement S; and bridged methylenephosphonates resulting from the replacement C. Preferably, replacement of the linking oxygen(s) is performed in at least one of the 5'-terminal nucleotide residue and the 3'-terminal nucleotide residue of the single-stranded nucleic acid molecule of the present invention, for example. When the replacement is performed on the 5' side, replacement with C is preferable. When the replacement is performed on the 3' side, replacement with N is preferable.

The phosphate group may be replaced with the phosphate-free linker, for example. The linker may contain siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo, methyleneoxymethylimino, or the like. Preferably, the linker may contain a methylenecarbonylamino group and a methylenemethylimino group.

In the single-stranded nucleic acid molecule of the present invention, for example, at least one of a nucleotide residue at the 3' end and a nucleotide residue at the 5' end may be modified. The nucleotide residue at either one of the 3' end and the 5' end may be modified, or the nucleotide residues at both the 3' end and the 5' end may be modified, for example. The modification may be as described above, for example, and it is preferable to modify a phosphate group(s) at the end(s). The entire phosphate group may be modified, or one or more atoms in the phosphate group may be modified, for example. In the former case, for example, the entire phosphate group may be replaced or deleted.

Modification of the terminal nucleotide residue(s) may be addition of any other molecule, or the like, for example. Examples of the other molecule include functional molecules such as labeling substances as described above and protecting groups. Examples of the protecting groups include S (sulfur), Si (silicon), B (boron), and ester-containing groups. The functional molecules such as the labeling substances can be used in the detection and the like of the single-stranded nucleic acid molecule of the present invention, for example.

The other molecule may be added to the phosphate group of the nucleotide residue, or may be added to the phosphate group or the sugar residue via a spacer, for example. The terminal atom of the spacer can be added to or replaced with either one of the linking oxygens of the phosphate group, or O, N, S, or C of the sugar residue, for example. The bonding site in the sugar residue is preferably, for example, C at the 3'-position, C at the 5'-position, or any atom bonded thereto. The spacer also can be added to or replaced with a terminal atom of the nucleotide alternative such as PNA, for example.

The spacer is not particularly limited, and examples thereof include $-(CH_2)n-$, $-(CH_2)nN-$, $-(CH_2)nO-$, $-(CH_2)nS-$, $O(CH_2CH_2O)$ $nCH_2CH_2OH$, abasic sugars, amide, carboxy, amine, oxyamine, oxyimine, thioether, disulfide, thiourea, sulfonamide, and morpholino, and also biotin reagents and fluorescein reagents. In the above formulae, n is a positive integer, and n = 3 or 6 is preferable.

Other examples of the molecule to be added to the end include dyes, intercalating agents (e.g., acridines), crosslinking agents (e.g., psoralen, mitomycin C), porphyrins (TPPC4, texaphyrin, Sapphyrin), polycyclic aromatic hydrocarbons (e.g., phenazine, dihydrophenazine), artificial endonucleases (e.g., EDTA), lipophilic carriers (e.g., cholesterol, cholic acid, adamantane acetic acid, 1-pyrene butyric acid, dihydrotestosterone, 1,3-bis-O (hexadecyl)glycerol, a geranyloxyhexyl group, hexadecylglycerol, borneol, menthol, 1,3-propanediol, a heptadecyl group, palmitic acid, myristic acid, 03-(oleoyl)lithocholic acid, O3-(oleoyl)cholic acid, dimethoxytrityl, or phenoxazine), peptide complexes (e.g., Antennapedia peptide, Tat peptide), alkylating agents, phosphate, amino, mercapto, PEG (e.g., PEG-40K), MPEG, [MPEG]2, polyamino, alkyl, substituted alkyl, radiolabeled markers, enzymes, haptens (e.g., biotin), transport/absorption facilitators (e.g., aspirin, vitamin E, folic acid), and synthetic ribonucleases (e.g., imidazole, bisimidazole, histamine, imidazole clusters, acridine-imidazole complexes, Eu3+ complexes of tetraazamacrocycles).

In the single-stranded nucleic acid molecule of the present invention, the 5' end may be modified with a phosphate group or a phosphate group analog, for example. Examples of the phosphate group include:

5'-monophosphate ($(HO)_2(O)P$-O-5');
5'-diphosphate ($(HO)_2(O)P$-O-P(HO)(O)-O-5');
5'-triphosphate $(HO)_2(O)P$-O-(HO)(O)P-O-P(HO)(O)-O-5');
5'-guanosine cap (7-methylated or non-methylated, 7m-G-O-5'-(HO)(O)P-O-(HO)(O)P-O-P(HO)(O)-O-5');
5'-adenosine cap (Appp);
any modified or unmodified nucleotide cap structure (N-O-5'-(HO)(O)P-O-(HO)(O)P-O-P(HO)(O)-O-5');
5'-monothiophosphate (phosphorothioate: $(HO)_2(S)P$-O-5');
5'-monodithiophosphate (phosphorodithioate: (HO)(HS)(S)P-O-5');

5'-phosphorothiolate ($(HO)_2(O)P-S-5'$);
sulfur substituted monophosphate, diphosphate, and
triphosphates (e.g., 5'-α-thiotriphosphate, 5'-y-thiotriphosphate, and the like);
5 '-phosphoramidates ($(HO)_2(O)P-NH-5'$, $(HO)(NH_2)(O)P-O-5'$);
5'-alkylphosphonates (e.g., RP(OH)(O)-O-5', $(OH)_2O)P-5'-CH_2$, where R is alkyl (e.g., methyl, ethyl, isopropyl, propyl, or the like)); and
5'-alkyl etherphosphonates (e.g., RP(OH)(O)-O-5', where R is an alkyl ether (e.g., methoxymethyl, ethoxymethyl, or the like)).

In the nucleotide residue, the base is not particularly limited. The base may be a natural base or a non-natural base, for example. The base may be a naturally-derived base or a synthetic base, for example. As the base, a common (universal) base, a modified analog thereof, and the like can be used, for example.
Examples of the base include: purine bases such as adenine and guanine; and pyrimidine bases such as cytosine, uracil, and thymine. Other examples of the base include inosine, thymine, xanthine, hypoxanthine, purine, nubularine, isoguanisine, tubercidine, and 7-deazazaadenine. Examples of the base also include: alkyl derivatives such as 2-aminoadenine, 6-methylated purine, and 2-propylated purine; 5-halouracil and 5-halocytosine; 5-propynyl uracil and 5-propynyl cytosine; 6-azo uracil, 6-azo cytosine, and 6-azo thymine; 5-uracil (pseudouracil), 4-thiouracil, 5-halouracil, 5-(2-aminopropyl)uracil, 5-amino allyl uracil; 8-halogenated, aminated, thiolated, thioalkylated, hydroxylated, and other 8-substituted purines; 5-trifluoromethylated and other 5-substituted pyrimidines; 7-methylguanine; 5-substituted pyrimidines; 6-azapyrimidines; N-2, N-6, and 0-6 substituted purines (including 2-aminopropyladenine); 5-propynyluracil and 5-propynylcytosine; dihydrouracil; 3-deaza-5-azacytosine; 2-aminopurine; 5-alkyluracil; 7-alkylguanine; 5-alkylcytosine; 7-deazaadenine; N6,N6-dimethyladenine; 2,6-diaminopurine; 5-amino-allyl-uracil; N3-methyluracil; substituted 1,2,4-triazoles; 2-pyridinone; 5-nitroindole; 3-nitropyrrole; 5-methoxyuracil; uracil-5-oxyacetic acid; 5-methoxycarbonylmethyluracil; 5-methyl-2-thiouracil; 5-methoxycarbonylmethyl-2-thiouracil; 5-methylaminomethyl-2-thiouracil; 3-(3-amino-3-carboxypropyl)uracil; 3-methylcytosine; 5-methylcytosine; N4-acetylcytosine; 2-thiocytosine; N6-methyladenine; N6-isopentyladenine; 2-methylthio-N6-isopentenyladenine; N-methylguanine; and 0-alkylated bases. Examples of the purines and pyrimidines include those disclosed in U.S. Patent No. 3,687,808, "Concise Encyclopedia of Polymer Science and Engineering", pp. 858 to 859, edited by Kroschwitz J. I, John Wiley & Sons, 1990, and Englisch et al, Angewandte Chemie, International Edition, 1991, vol. 30, p. 613.
Other examples of the modified nucleotide residue include those having no base, i.e., those having an abasic ribophosphate backbone. Furthermore, as the modified nucleotide residue, those described in U.S. provisional Application 60/465,665 (filing date: April 25, 2003) and International Application No. PCT/US04/07070 (filing date: March 8, 2004) can be used, for example, and these documents are incorporated herein by reference.

3. Non-nucleotide residue

The non-nucleotide residue is not particularly limited. The single-stranded nucleic acid molecule of the present invention may have, as the non-nucleotide residue, for example, a non-nucleotide structure containing an amino acid residue or a peptide residue. Examples of the amino acid composing the amino acid residue or peptide residue include basic amino acids, acidic amino acids and the like. Examples of the basic amino acid include lysine, arginine, histidine and the like. Examples of the acidic amino acid include aspartic acid, glutamic acid and the like.

4. Composition

The expression inhibitory composition according to the present invention is, as described above, a composition for inhibiting expression of a target gene, containing the single-stranded nucleic acid molecule of the present invention. The composition of the present invention is characterized in that it contains the single-stranded nucleic acid molecule of the present invention, and other configurations are by no means limited. The expression inhibitory composition of the present invention also can be referred to as an expression inhibitory reagent, for example.
According to the present invention, for example, by administering the composition to a subject in which the target gene is present, it is possible to inhibit the expression of the target gene.
Furthermore, as described above, the pharmaceutical composition according to the present invention contains the single-stranded nucleic acid molecule of the present invention. The pharmaceutical composition of the present invention is characterized in that it contains the single-stranded nucleic acid molecule of the present invention, and other configurations are by no means limited. The pharmaceutical composition of the present invention also can be referred to as a pharmaceutical, for example.
According to the present invention, for example, by administering the pharmaceutical composition to a patient with a disease caused by a gene, it is possible to inhibit the expression of the gene, thereby treating the disease. In the

present invention, the term "treatment" encompasses: prevention of diseases; improvement of diseases; and improvement in prognosis, for example, and it can mean any of them.

In the present invention, a disease to be treated is not particularly limited, and examples thereof include diseases caused by the expression of genes. Depending on the kind of the disease, a gene that causes the disease may be set as the target gene, and further, depending on the target gene, the expression inhibitory sequence may be set as appropriate.

A specific example is as follows. By setting TGF-β1 gene as the target gene and incorporating an expression inhibitory sequence for this gene into the single-stranded nucleic acid molecule, the single-stranded nucleic acid molecule can be used for the treatment of disease or pathological condition wherein the therapeutic effect is expected due to inhibition of TGF-β1, for example, inflammatory diseases, specifically, acute lung injury and the like, for example.

The method of using the expression inhibitory composition and the pharmaceutical composition according to the present invention (hereinafter, both the compositions simply are referred to as "the compositions") are not particularly limited, and examples thereof include administering the single-stranded nucleic acid molecule to a subject having the target gene.

Examples of the subject to which the single-stranded nucleic acid molecule of the present invention is administered include cells, tissues, and organs. Examples of the subject also include humans and nonhuman animals such as nonhuman mammals, i.e., mammals excluding humans. The administration may be performed in vivo or in vitro, for example. The cells are not particularly limited, and examples thereof include: various cultured cells such as HeLa cells, 293 cells, NIH3T3 cells, and COS cells; stem cells such as ES cells and hematopoietic stem cells; and cells isolated from living organisms, such as primary cultured cells.

The administration method is not particularly limited, and can be determined as appropriate depending on the subject, for example. When the subject is a cultured cell, the administration method may be a method using a transfection reagent, an electroporation method, or the like, for example. When the administration is performed in vivo, the administration may be either oral administration or parenteral administration, for example. Examples of the parenteral administration include injection, subcutaneous administration, and local administration.

The compositions of the present invention may contain only the single-stranded nucleic acid molecule of the present invention or may further contain an additive(s) in addition to the single-stranded nucleic acid molecule, for example. The additive is not particularly limited, and is preferably a pharmaceutically acceptable additive, for example. The kind of the additive is not particularly limited, and can be selected as appropriate depending on the kind of the subject, for example.

In the composition of the present invention, the single-stranded nucleic acid molecule may form a complex with the additive, for example. The additive also can be referred to as a complexing agent, for example. The complex allows the single-stranded nucleic acid molecule to be delivered efficiently, for example. The bond between the single-stranded nucleic acid molecule and the complexing agent is not particularly limited; and examples thereof include noncovalent bond. The complex may be an inclusion complex or the like, for example.

The complexing agent is not particularly limited, and examples thereof include polymers, cyclodextrins, and adamantine. Examples of the cyclodextrins include linear cyclodextrin copolymers and linear oxidized cyclodextrin copolymers.

Other examples of the additive include a carrier, a binding substance that binds to a target cell, a condensing agent, and a fusogenic agent.

The carrier is preferably a polymer, more preferably a biopolymer, for example. Preferably, the carrier is biodegradable, for example. Examples of the carrier include: proteins such as human serum albumin (HSA), low-density lipoprotein (LDL), and globulin; carbohydrates such as, for example, dextran, pullulan, chitin, chitosan, inulin, cyclodextrin, and hyaluronic acid; and lipids. As the carrier, a synthetic polymer such as a synthetic polyamino acid also can be used, for example. Examples of the polyamino acid include polylysine (PLL), poly L-aspartic acid, poly L-glutamic acid, styrene-maleic anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacryllic acid), N-isopropylacrylamide polymer, and polyphosphazine.

Examples of the binding substance include thyroid-stimulating hormone, melanocyte-stimulating hormone, lectin, glycoproteins, surfactant protein A, Mucin carbohydrate, multivalent lactose, multivalent galactose, N-acetylgalactosamine, N-acetyl-gulucosamine, multivalent mannose, multivalent fucose, glycosylated polyamino acid, multivalent galactose, transferrin, bisphosphonate, polyglutamic acid, polyaspartic acid, lipids, cholesterol, steroids, bile acid, folate, vitamin B12, biotin, Neproxin, RGD peptide, and RGD peptide mimetic.

Examples of the fusogenic agent and the condensing agent include polyamino chains such as polyethyleneimine (PEI). PEI may be either linear or branched, and also, may be either synthetic or naturally occurring, for example. The PEI may be substituted with an alkyl or a lipid, for example. As the fusogenic agent, it is also possible to use polyhistidine, polyimidazole, polypyridine, polypropyleneimine, mellitin, a polyacetal substance (e.g., cationic polyacetal or the like), or the like, for example. The fusogenic agent may have an α-helix structure, for example. The fusogenic agent may be

a membrane disruptive agent such as mellitin, for example.

As to the compositions according to the present invention, for example, the descriptions regarding the formation of the complex and the like in U.S. Patent No. 6,509,323 , U.S. Patent Publication No. 2003/0008818 , PCT/US04/07070 , and the like are incorporated herein by reference.

Other examples of the additive include amphiphilic molecules. The amphiphilic molecule is a molecule having a hydrophobic region and a hydrophilic region, for example. The molecule is preferably a polymer, for example. The polymer may have, for example, a secondary structure, preferably a repeating secondary structure. Specifically, polypeptide is preferable, and $\alpha$-helix polypeptide and the like are more preferable, for example.

The amphiphilic polymer may be a polymer having two or more amphiphilic subunits, for example. Examples of the subunit include subunits with a cyclic structure having at least one hydrophilic group and one hydrophobic group. The subunit may contain steroid such as cholic acid, an aromatic structure, and the like, for example. The polymer may contain, for example, both a cyclic structure subunit, such as an aromatic subunit, and an amino acid.

5. Expression Inhibitory Method

The expression inhibitory method according to the present invention is, as described above, a method for inhibiting expression of a target gene, in which the single-stranded nucleic acid molecule of the present invention is used. The expression inhibitory method of the present invention is characterized in that the single-stranded nucleic acid molecule of the present invention is used therein, and other steps and conditions are by no means limited.

In the expression inhibitory method of the present invention, the mechanism by which the gene expression is inhibited is not particularly limited, and examples thereof include inhibition of the expression by RNA interference. The expression inhibitory method of the present invention is, for example, a method for inducing RNA interference that inhibits expression of a target gene, and it also can be referred to an expression induction method that is characterized in that the single-stranded nucleic acid molecule of the present invention is used therein.

The expression inhibitory method of the present invention contains the step of administering the single-stranded nucleic acid molecule to a subject in which the target gene is present, for example. By the administration step, the single-stranded nucleic acid molecule is bought into contact with the subject to which the single-stranded nucleic acid molecule is administered, for example. Examples of the subject include cells, tissues, and organs. Examples of the subject also include humans and nonhuman animals such as nonhuman mammals, i.e., mammals excluding humans. The administration may be performed in vivo or in vitro, for example.

In the expression inhibitory method of the present invention, the single-stranded nucleic acid molecule may be administered alone, or the composition of the present invention containing the single-stranded nucleic acid molecule may be administered, for example. The administration method is not particularly limited, and can be selected as appropriate depending on the kind of the subject, for example.

6. Treatment Method

As described above, the method for treating a disease according to the present invention contains the step of administering the single-stranded nucleic acid molecule of the present invention to a patient, and the single-stranded nucleic acid molecule contains, as the expression inhibitory sequence, a sequence that inhibits expression of a gene causing the disease. The treatment method of the present invention is characterized in that the single-stranded nucleic acid molecule of the present invention is used therein, and other steps and conditions are by no means limited. The description regarding the expression inhibitory method of the present invention also applies to the treatment method of the present invention, for example.

7. Use of Single-Stranded Nucleic Acid Molecule

The use according to the present invention is the use of the single-stranded nucleic acid molecule of the present invention for inhibiting expression of a target gene. Also, the use according to the present invention is the use of the single-stranded nucleic acid molecule of the present invention for inducing RNA interference.

The nucleic acid molecule according to the present invention is a nucleic acid molecule for use in treatment of a disease. The nucleic acid molecule is the single-stranded nucleic acid molecule of the present invention, and the single-stranded nucleic acid molecule contains, as the expression inhibitory sequence, a sequence that inhibits expression of a gene causing the disease.

**Examples**

The present invention is explained in detail in the following by referring to Examples, Experimental Examples

and Formulation Examples, which are not to be construed as limitative, and the invention may be changed within the scope of the present invention.

Example 1: Synthesis of N-2-chloroacetyl-L-proline-succinimidyl ester (3)

N-2-Chloroacetyl-L-proline-succinimidyl ester (3) was synthesized according to the following scheme.

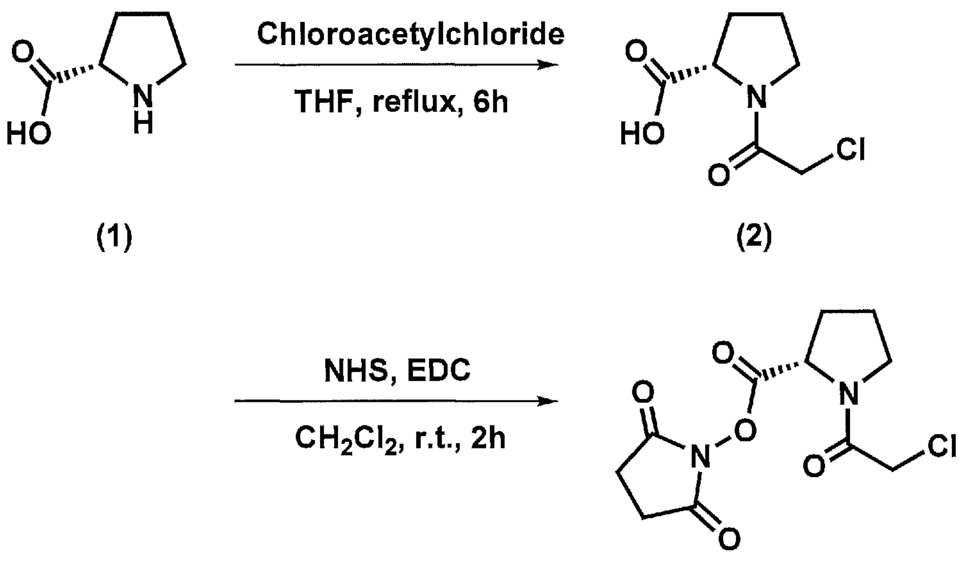


(1) (2)

(3)

(1) Synthesis of N-2-chloroacetyl-L-proline (2)

To a supension of L-proline (1) (1.0 g, 8.7 mmol) in anhydrous THF (10 mL) was added dropwise a solution of chloroacetyl chloride (1.05 mL, 13.2 mmol) in anhydrous THF (1.5 mL) with stirring under argon atmosphere. The reaction mixture was heated with reflux for 6 hr, and allowed to cool to room temperature. Deionized water (2 mL) was added thereto, and the mixture was stirred for 10 min. The mixture was subjected to separation operation with saturated brine and ethyl acetate to give an organic layer. After checking whether the pH of the remaining aqueous layer was 2 or lower, the aqueous layer was extracted twice with ethyl acetate. The organic layers were combined, and dried over magnesium sulfate, and the solvent was evaporated under reduced pressure. The obtained residue was triturated with diisopropyl ether to give compound (2) (0.97 g, yield 58.4%) as a white powder. The instrumental analysis value of compound (2) are shown below. compound (2):
$^1$H-NMR (500 MHz, $CDCl_3$) δ: 8.35 (1H, br.s), 4.56-4.63 (1H, m), 4.00-4.14 (2H, m), 3.57-3.73 (2H, m), 1.93-2.35 (4H, m). melting point: 110-111°C
ESI-MS:214.03[M+Na]$^+$

(2) Synthesis of N-2-chloroacetyl-L-proline-succinimidyl ester (3)

N-2-Chloroacetyl-L-proline (2) (0.1 g, 0.5 mmol) was dissolved in dichloromethane (3 mL), and 1-ethyl-3- (3-dimethylaminopropyl)carbodiimide hydrochloride (0.12 g, 0.6 mmol) and N-hydroxysuccinimide (0.07 g, 0.6 mmol) were added thereto, and the mixture was stirred at room temperature for 2 hr. After the completion of the reaction, dichloromethane was added thereto, and the mixture was washed with water (twice) and saturated brine (twice). The organic layer was dried over anhydrous sodium sulfate, and concentrated under reduced pressure to give compound (3) (0.11 g, yield 73%) as a white foam. The instrumental analysis value of compound (3) are shown below.
compound (3):
$^1$H-NMR (500 MHz, $CDCl_2$) δ: 4. 83-4. 96 (1H, m), 4.04-4.11 (2H, m), 3.61-3.78 (2H, m), 2.83-2.87 (4H, m), 2.02-2.50 (4H, m).
ESI-MS:311.05[M+Na]$^+$

Example 2: Synthesis of new single-stranded nucleic acid molecule (PS-0001-C3)

(1) Synthesis of nucleic acid

The nucleic acid shown below was synthesized using a nucleic acid synthesizer (trade name ABI Expedite (trademark registration) 8909 Nucleic Acid Synthesis System, Applied Biosystems), according to phosphoramidite method. Solid-phase synthesis was performed using TBDMS amidite as RNA amidite. For synthesis of sense strand, Phthalamido Amino C6 lcaa CPG (ChemGenes) (the structure was shown below) was used as the 3'-end. For synthesis of antisense strand, 5'-Thiol C-3 Disulfide Modifier CED phosphoramidite (ChemGenes) (the structure was shown below) was used as the 5'-end. The release the resulting nucleic acid from the solid-phase and deprotection were performed according to a conventional method. The synthesized nucleic acid was pufiried by HPLC. The underlined part in the following antisense strand nucleic acid is expression inhibitory sequence of humans TGF-β1 gene.

Phthalamido Amino C6 lcaa CPG

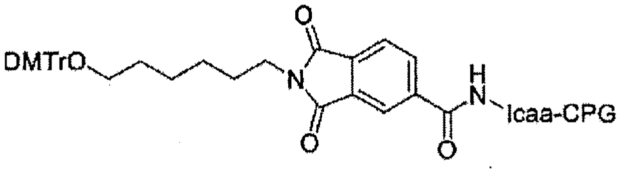

5'-Thiol C-3 Disulfide Modifier CED phosphoramidite

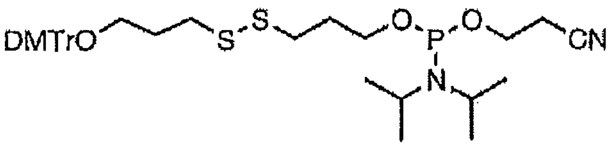

Sense Strand

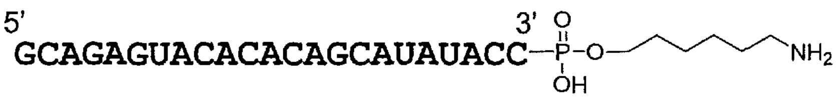


Antisense Strand

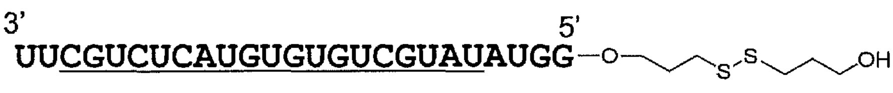


(2) Synthesis of new single-stranded nucleic acid molecule

The structure of the single-stranded nucleic acid molecule of the present invention, PS-0001-C3 are shown below.

PS-0001-C3 is the single-stranded nucleic acid molecule obtained by bonding the 3'-end of the sense strand and the 5'-end of the antisense strand, via N-2-chloroacetyl-L-proline-succinimidyl ester (3) obtained in Example 1, according to the method mentioned below.

PS-0001-C3

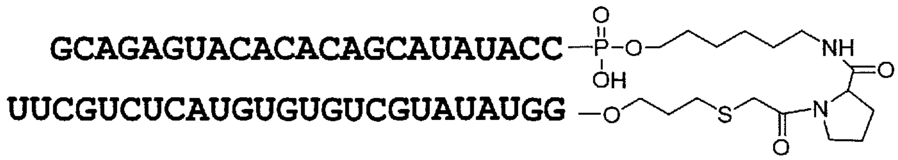

Synthesis of sense strand + P

Sense strand (1 mmol/L, 40 μL), phosphate buffer solution (pH 8.5, 1 mol/L, 20 pL), N-2-chloroacetyl-L-proline-succinimidyl ester (3)/DMF (693 nmol, 2.0 mg/100 μL, 10 μL) and distilled water for injection (30 μL) were mixed, and the mixture was stirred at 25°C for 50 min. Then, N-2-chloroacetyl-L-proline-succinimidyl ester (3)/DMF (2.0 mg/100 μL, 5 μL) was added again thereto, and the mixture was left to stand for 70 min. This reaction solution was purified by illustra MicroSpin G-25 Columns (GE Healthcare) with distilled water for injection (Otsuka Pharmaceutical) as an eluent to give the sense strand + P. mass analysis: 7689.00 (calculated value: 7689.29). The HPLC analysis result is shown in Fig.1. The analysis conditions are as follows; XBridge OST C18 (4.6×50 mM), mobile phase A: 50 mM TEAA (pH7), 5% $CH_3CN$, mobile phase B: 50 mM TEAA (pH7), 50% $CH_3CN$, gradient B: 0-30%/20 min, flow rate: 1 mL/min, column temperature: 60°C, and detection: UV 260 nm.

Sense Strand + P

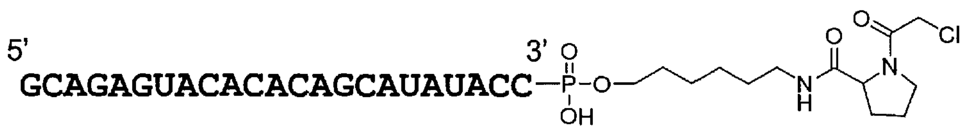

Synthesis of antisense strand (SH form)

Antisense strand (1 mmol/L, 40 μL), phosphate buffer solution (pH 8.0, 1 mol/L, 20 μL), aqueous dithiothreitol solution (10 mg/mL, 20 μL) and distilled water for injection (20 μL) were mixed, and the mixture was stirred at 25°C for 45 min. Then, aqueous dithiothreitol solution (10 mg/mL, 5 μL) was added again thereto, and the mixture was stirred for 1.5 hr. This reaction solution was purified by illustra MicroSpin G-25 Columns (GE Healthcare) with distilled water for injection (Otsuka Pharmaceutical) as an eluent to give the antisense strand (SH form). mass analysis: 8084.30 (calculated value: 8084.85). The HPLC analysis result is shown in Fig.2. The analysis conditions are as follows; XBridge OST C18 (4.6×50 mM), mobile phase A: 50 mM TEAA (pH7), 5% $CH_3CN$, mobile phase B: 50 mM TEAA (pH7), 50% $CH_3CN$, gradient B: 0-30%/20 min, flow rate: 1 mL/min, column temperature: 60°C, and detection: UV 260 nm.

Antisense Strand (SH form)

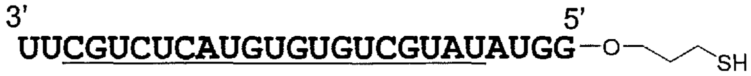

Synthesis of PS-0001-C3

Sense strand + P (170 μL), antisense strand (SH form, 170 μL), phosphate buffer solution (pH 8.5, 1 mol/L, 100 μL) and distilled water for injection (60 μL) were mixed, and the mixture was stirred at 25°C for 2 hr. The reaction solution was purified by HPLC (column: XBridge Oligonucleotide BEH C18, 2.5 μm, 10×50 mM; flow rate: 4 mL/min; detection: UV 260 nm; column temperature: 40°C; mobile phase A: 50 mMol/L TEAA (pH7.0), 5% $CH_3CN$; mobile phase

B: 50 mMol/L TEAA (pH7.0), 50% $CH_3CN$), and the objective fractions were collected. The collected fraction was subjected to ethanol precipitation to give PS-0001-C3. mass analysis: 15737.80 (calculated value: 15737.67). The HPLC analysis result after purification is shown in Fig.3. The analysis conditions are as follows; DNAPac PA-100 (4×250 mM), mobile phase A: 25 mM Tris-HCl (pH 8), 10% $CH_3CN$, 8M Urea, mobile phase B: 25 mM Tris-HCl (pH 8), 10% $CH_3CN$, 8M Urea, 700 mM $NaClO_4$, gradient B: 10-40%/20 min, flow rate: 1 mL/min, column temperature: 80°C, and detection: UV 260 nm.

Example 3: Synthesis of $N^{\alpha}$-2-bromoacetyl-$N^{\varepsilon}$-tert-butoxycarbonyl-L-lysine-succinimidyl ester (6)

According to the following scheme, $N^{\alpha}$-2-bromoacetyl-$N^{\varepsilon}$-tert-butoxycarbonyl-L-lysine-succinimidyl ester (6) was synthesized.

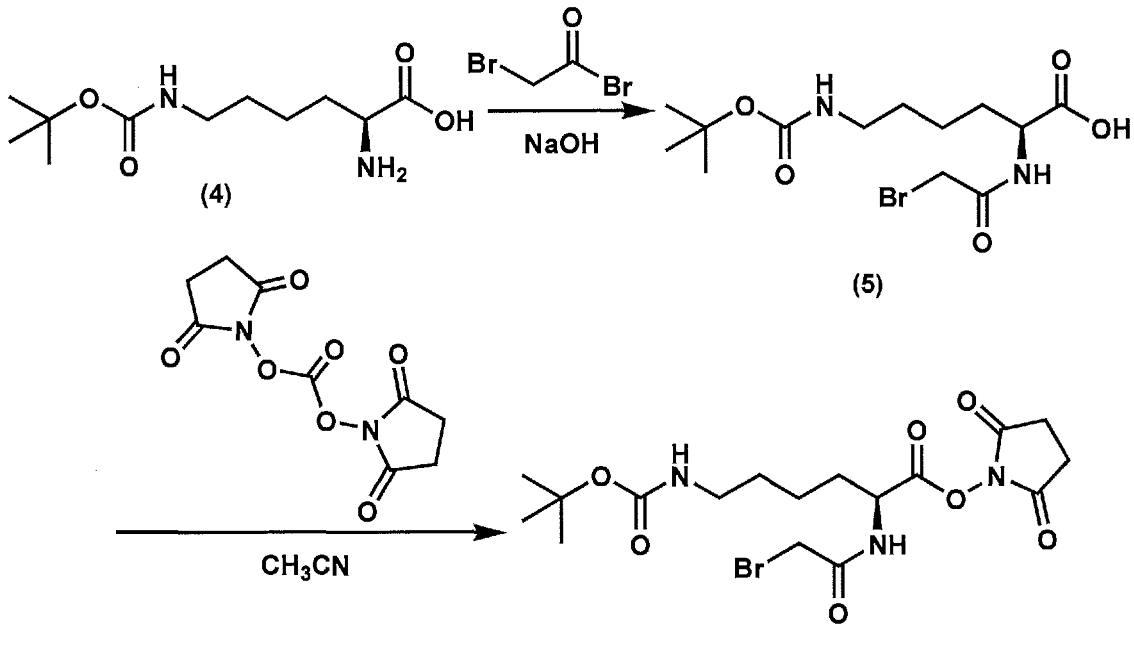

(4)
(5)

(6)

(1) Synthesis of $N^{\alpha}$-2-bromoacetyl-$N^{\varepsilon}$-tert-butoxycarbonyl-L-lysine (5)

To $N^{\varepsilon}$-tert-butoxycarbonyl-L-lysine (4) (1.00 g, 4.06 mmol) were added water (4 mL), THF (4 mL) and 4N aqueous NaOH solution (880 μL, 3.52 mmol) at room temperature to dissolve the compound. This solution was cooled to 0°C, and 4 mol/L aqueous sodium hydroxide solution (1.2 mL, 4.8 mmol) and bromoacetyl bromide (400 μL, 4.62 mmol) were added thereto. To the reaction solution was added 3 mol/L hydrobromic acid (2 mL), and the mixture was extracted three times with methyl tert-butyl ether (10 mL). The combined organic layers were washed with aqueous sodium hydrogencarbonate solution, and concentrated under reduced pressure to give compound (5) (307 mg, yield 21%) as a colorless oil. The instrumental analysis value of compound (5) are shown below.
compound (5):
$^1$H-NMR (400 MHz, DMSO-d6) δ: 8.54 (1H, br.s), 6.76 (1H, br.s), 4.10-4.15 (1H, m), 3.85-3.91 (2H, m), 2.84-2.89 (2H, m), 1.51-1.71 (2H, m),1.19-1.39 (4H, m), 1.35 (9H, s).
ESI-MS:389.10$[M+Na]^+$

(2) Synthesis of $N^{\alpha}$-2-bromoacetyl-$N^{\varepsilon}$-tert-butoxycarbonyl-L-lysine-succinimidyl ester (6)

To a suspension of $N^{\alpha}$-2-bromoacetyl-$N^{\varepsilon}$-tert-butoxycarbonyl-L-lysine (5) (439.4 mg, 1.20 mmol) and di(N-succinimidylcarbonate) (322.0 mg, 1.26 mmol) in acetonitrile (4.4 mL) was added N,N-dimethylaminopyridine (29.6 mg, 0.24 mmol) at room temperature. The mixture was stirred for 20 min, and the reaction solution was concentrated under reduced pressure. The residue was dilued with chloroform (11 mL), and washed four times with water (8.8 mL), and the organic layer was dried over anhydrous magnesium sulfate, and concentrated under reduced pressure to give compound (6) (507.1 mg, yield 91%) as colorless foamy solid. The instrumental analysis value of compound (6) are shown below.

compound (6):
$^1$H-NMR (400 MHz, DMSO-d6) δ: 8.98 (1H, br.s), 6.79 (1H, br.s), 4.59-4.64 (1H, m), 3.89-3.95 (2H, m), 2.89-2.91 (2H, m), 2.81-2.83 (4H, m), 1.73-1.90 (2H, m), 1.34-1.40 (4H, m), 1.37 (9H, s) .
ESI-MS:486.08[M+Na]$^+$

Example 4: Synthesis of new single-stranded nucleic acid molecule (KS-0001)

The following sense strand and antisense strand prepared in Example 2 were used.

Sense Strand

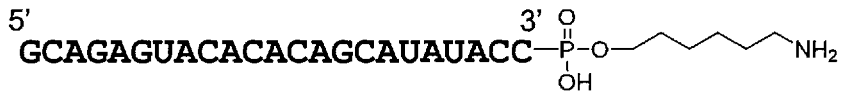

Antisense Strand

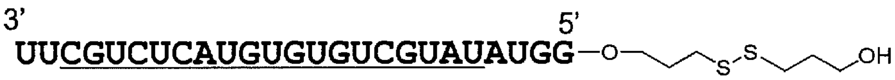

The structure of the single-stranded nucleic acid molecule of the present invention, KS-0001 are shown below.

KS-0001 is the single-stranded nucleic acid molecule obtained by bonding the 3'-end of the sense strand and the 5'-end of the antisense strand, via N$^\alpha$-2-bromoacetyl-N$^\varepsilon$-tert-butoxycarbonyl-L-lysine-succinimidyl ester (6), according to the method mentioned below.

KS-0001

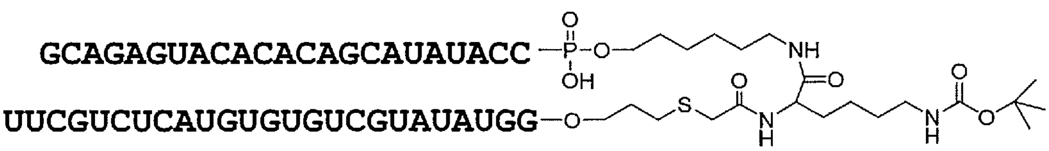

Synthesis of KS-0001

A mixed solution of sense strand (844 μmol/L, 48 pL), distilled water for injection (438 μL) and diisopropylethylamine (14 μL) was added to N$^\alpha$-2-bromoacetyl-N$^\varepsilon$-tert-butoxycarbonyl-L-lysine-succinimidyl ester (6) (obtained in Example 3)/DMF (17.2 mg/200 μL, 200 μL) in 5 parts, and the mixture was stirred at 25°C for 20 min.

Antisense strand (1 mmol/L, 20 μL), phosphate buffer solution (pH 8.0, 1 mol/L, 20 μL), aqueous dithiothreitol solution (10 mg/mL, 20 μL) and distilled water for injection (40 μL) were mixed, and the mixture was stirred at 25°C for 2 hr. This reaction solution was added to the reaction solution prepared above, and the mixture was attired at 25°C for 20 min. The reaction solution was subjected to ethanol precipitation, and purified by HPLC (column: Develosil C8-UG-5, 5 μm, 10×50 mM; flow rate: 4.7 mL/min; detection: UV 260 nm; column temperature: 50°C; mobile phase A: 50 mMol/L TEAA (pH7.0), 5% $CH_3CN$; mobile phase B: 50 mMol/L TEAA (pH7.0), 50% $CH_3CN$), and the objective fractions were collected. The collected fraction was concentrated under reduced pressure to give KS-0001. mass analysis: 15869.50 (calculated value: 15868.85). The HPLC analysis result after purification is shown in Fig.4. The analysis conditions are as follows; DNAPac PA-100 (4×250 mM), mobile phase A: 25 mM Tris-HCl (pH 8), 10% $CH_3CN$, 8M Urea, mobile phase B: 25 mM Tris-HCl (pH 8), 10% $CH_3CN$, 8M Urea, 700 mM $NaClO_4$, gradient B: 10-40%/20 min, flow rate: 1 mL/min, column temperature: 80°C, and detection: UV 260 nm.

Reference Example: Synthesis of new single-stranded nucleic acid molecule (AS-0001)

The following sense strand and antisense strand prepared in Example 2 were used.

Sense Strand

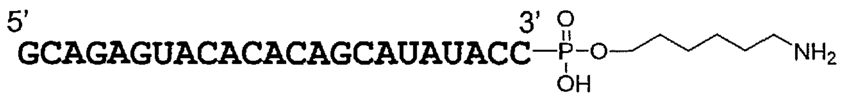

Antisense Strand

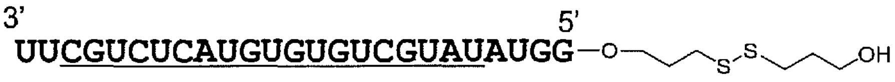

The structure of the single-stranded nucleic acid molecule of the present invention, AS-0001 are are shown below.

AS-0001 is the single-stranded nucleic acid molecule obtained by bonding the 3'-end of the sense strand and the 5'-end of the antisense strand, via succinimidyl 2-bromoacetate, according to the method mentioned below.

AS-0001

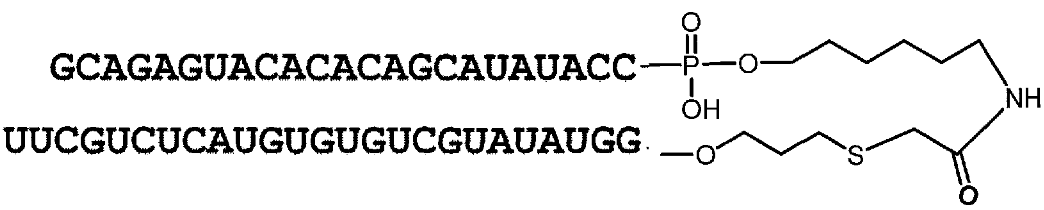

Synthesis of AS-0001

A mixed solution of sense strand (844 μmol/L, 48 μL), distilled water for injection (438 pL), and diisopropylethylamine (14 μL) were added to succinimidyl 2-bromoacetate/DMF (7.5 μmol/L, 200 μL) in 5 parts, and the mixture was stirred at 25°C for 20 min. The reaction solution was subjected to ethanol precipitation, and purified by HPLC (column: Develosil C8-UG-5, 5 μm, 10×50 mM; flow rate: 4.7 mL/min; detection: UV 260 nm; column temperature: 50°C; mobile phase A: 50 mMol/L TEAA (pH7.0), 5% $CH_3CN$; mobile phase B: 50 mMol/L TEAA (pH7.0), 50% $CH_3CN$), and the objective fractions were collected. The collected fraction was concentrated under reduced pressure to give sense strand + bromoacetyl form. mass analysis: 7636.90 (calculated value: 7636.62).

Antisense strand (1 mmol/L, 4 μL), phosphate buffer solution (pH 8.0, 1 mol/L, 4 μL), aqueous dithiothreitol solution (10 mg/mL, 4 μL) and distilled water for injection (8 μL) were mixed, and the mixture was stirred at 25°C for 30 min. The reaction solution (17.5 μL), the sense strand + bromoacetyl form (750 μL, 3.45 nmol) prepared above, and phosphate buffer solution (pH 8.5, 1mol/L, 85 μL) were mixed, and the mixture was stirred at 25°C for 30 min. The reaction solution was subjected to ethanol precipitation, and purified by illustra MicroSpin G-25 Columns (GE Healthcare) with distilled water for injection (Otsuka Pharmaceutical) as an eluent to give AS-0001. mass analysis: 15641.40(calculated value: 15640.56). The HPLC analysis result after purification is shown in Fig.5. The analysis conditions are as follows; DNAPac PA-100 (4×250 mM), mobile phase A: 25 mM Tris-HCl (pH 8), 10% $CH_3CN$, 8M Urea, mobile phase B: 25 mM Tris-HCl (pH 8), 10% $CH_3CN$, 8M Urea, 700 mM $NaClO_4$, gradient B: 10-40%/20 min, flow rate: 1 mL/min, column temperature: 80°C, and detection: UV 260 nm.

Experimental Example 1 (Measurement Of Gene Expression Inhibitory Activity)

A549 cells (DS Pharma Biomedical Co., Ltd.) were used as cells. A 10% FBS-containing DMEM (Invitrogen) was used as a medium. The culture conditions were set to 37°C and 5% $CO_2$.

First, the cells were cultured in the medium, and the cell suspension were dispensed to a 24-well plate by 400 μL per well at $4\times10^4$ cells/well. Next, the cells in the well were transfected with the nucleic acid molecule using a transfection reagent Lipofectamine RNAiMAX Transfection Reagent 2000 (Invitrogen) according to the protocol supplied therewith. Specifically, the transfection was carried out by setting the composition per well as follows. In the following composition, (B) is Opti-MEM (Invitrogen), (C) is a nucleic acid molecule solution, and 98.5 μL of the total of (B) and (C) was used. The final concentration of the nucleic acid molecule in the well was set to 0.01 nmol/L, 0.1 nmol/L or 1 nmol/L.

Table 1

| (Composition per well: μL) | |
|---|---|
| Medium | 400 |
| Transfection reagent | 1.5 |
| (B) and (C) | 98.5 |
| Total | 500 |

After the transfection, the cells were cultured for 24 hours, and then, the RNA was collected using NucleoSpin RNA Plus (Takara Bio) according to the protocol supplied therewith. Subsequently, cDNA was synthesized from the RNA using a reverse transcriptase (Transcriptor First Strand cDNA Synthesis Kit, Roche) according to the protocol supplied therewith. Then, as described below, PCR was carried out using the thus-synthesized cDNA as a template, and the expression level of the TGF-β1 gene and that of the β-actin gene as an internal standard were measured. The expression level of the TGF-β1 gene was corrected with reference to that of the β-actin gene.

The PCR was carried out using LightCycler 480 SYBR Green I Master (Roche) as a reagent and Light Cycler 480 Instrument II (Roche) as an instrument. The TGF-β1 gene and the β-actin gene were amplified using the following primer sets, respectively.

PCR Primer set for TGF-β1 gene

5'-ttgtgcggcagtggttgagccg-3' (SEQ ID NO: 6)
5'-gaagcaggaaaggccggttcatgc-3' (SEQ ID NO: 7)

PCR Primer set for β-actin gene

5'-gccacggctgcttccagctcctc-3' (SEQ ID NO: 8)
5'-aggtctttgcggatgtccacgtcac-3' (SEQ ID NO: 9)

As control, transfection was performed by the same procedures as in the above except that the nucleic acid molecule was not added and that (B) and 1.5 μL of the transfection reagent (A) were added so that the total amount of (A) and (B) would be 100 μL, and the expression level of the gene also was measured (mock).

Then, the corrected expression level of the TGF-β1 gene in the control (mock) was set as 1, and that in the cells transfected with the nucleic acid molecule at each concentration was presented as the relative value to that in the control (mock).

The results are shown in Fig.6. As shown in Fig.6, it is demonstrated that the nucleic acid molecule of the example exhibits a strong inhibitory activity of gene expression.

## Industrial Applicability

According to the new production method of single-stranded nucleic acid molecules of the present invention, long-chain oligomer synthesis with difficulty can be carried out by employing short-chain oligomer techniques, and new single-stranded nucleic acid molecules produced by the method can be provided.

This application is based on patent application No. 2017-199945 filed on October 13, 2017 in Japan, the contents of which are encompassed in full herein.

## Claims

**1.** A method of producing a single-stranded nucleic acid molecule represented by the following general formula (I):

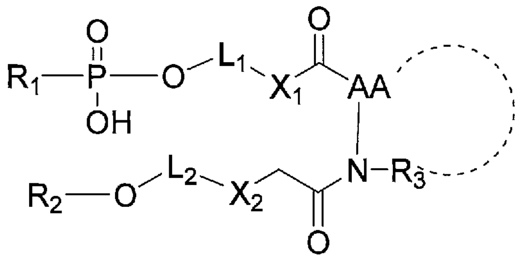 (I)

wherein

$R_1$ is a region comprising an oligonucleotide or a polynucleotide, the 3'-terminal nucleotide residue of which is bonded to -P(=O)(OH)-O-;
$R_2$ is a region comprising an oligonucleotide or a polynucleotide, the 5'-terminal nucleotide residue of which is bonded to -O-;
$X_1$ and $X_2$ are each independently NH, S or 0;
$L_1$ and $L_2$ are each independently a $C_{1-30}$ alkylene chain, or-$(CH_2)_l$-$(O-(CH_2)_m-)_n$- wherein 1 is an integer of 1 to 3, m is an integer of 1 to 3, and n is an integer of 1 to 10;
-C(=O)-AA-$NR_3$- is an amino acid residue; and
$R_3$ is a hydrogen atom or a $C_{1-6}$ alkyl group, or $R_3$ is bonded to AA to form a nitrogen-containing heterocycle, which comprises

(Step 1) a step of reacting a nucleic acid represented by the following general formula (II):

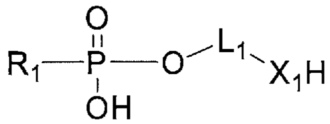 (II)

wherein each symbol is as defined above,
with a compound represented by the following general formula (IV) :

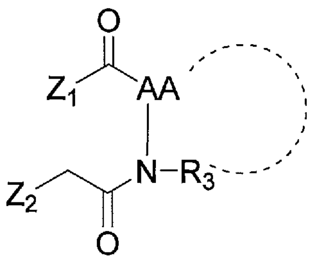 (IV)

wherein

$Z_1$ is a succinimidyloxy group, a benzotriazolyloxy group, a pentafluorophenoxy group or a halogen atom;
$Z_2$ is a halogen atom; and
the other symbols are as defined above,
to obtain a nucleic acid represented by the following general formula (V):

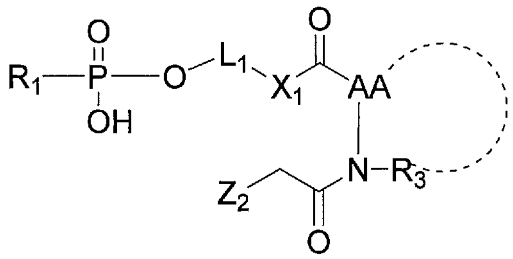

(V)

wherein each symbol is as defined above; and

(Step 2) a step of reacting the nucleic acid represented by the general formula (V) with a nucleic acid represented by the following general formula (III):

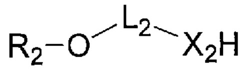

(III)

wherein each symbol is as defined above,
to obtain the single-stranded nucleic acid molecule represented by the general formula (I).

2. The method according to claim 1, wherein the amino acid residue is a proline residue, a glycine residue, or a lysine residue with the side chain amino group being optionally protected.

3. The method according to claim 1, wherein $X_2$ is S.

4. The method according to claim 1, wherein $X_1$ is NH.

5. The method according to claim 1, wherein $L_1$ is a $C_{2-11}$ alkylene chain.

6. The method according to claim 1, wherein $L_1$ is - $CH_2CH_2(OCH_2CH_2)_n$- wherein n is an integer of 1 to 5.

7. The method according to claim 1, wherein $L_2$ is a $C_{2-11}$ alkylene chain.

8. The method according to claim 1, wherein $L_2$ is-$CH_2CH_2$ $(OCH_2CH_2)_n$- wherein n is an integer of 1 to 5.

9. The method according to claim 1, wherein $Z_1$ is a succinimidyloxy group.

10. The method according to any of claims 1 to 9, wherein the single-stranded nucleic acid molecule comprises a sequence that inhibits expression of a target gene.

11. A single-stranded nucleic acid molecule represented by the following general formula (Ia) :

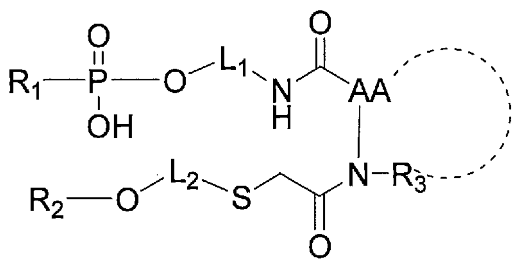

(Ia)

wherein

$R_1$ is a region comprising an oligonucleotide or a polynucleotide, the 3'-terminal nucleotide residue of which is bonded to -P(=O)(OH)-O-;

$R_2$ is a region comprising an oligonucleotide or a polynucleotide, the 5'-terminal nucleotide residue of which is bonded to -O-;
$L_1$ and $L_2$ are each independently a $C_{1-30}$ alkylene chain, or-$(CH_2)_1$-(O-$(CH_2)_m$-$)_n$- wherein 1 is an integer of 1 to 3, m is an integer of 1 to 3, and n is an integer of 1 to 10;
-C(=O)-AA-$NR_3$- is an amino acid residue; and
$R_3$ is a hydrogen atom or a $C_{1-6}$ alkyl group, or $R_3$ is bonded to AA to form a nitrogen-containing heterocycle.

**12.** The single-stranded nucleic acid molecule according to claim 11, wherein the amino acid residue is a proline residue, a glycine residue, or a lysine residue with the side chain amino group being optionally protected.

**13.** The single-stranded nucleic acid molecule according to claim 11, wherein $L_1$ is a $C_{2-11}$ alkylene chain.

**14.** The single-stranded nucleic acid molecule according to claim 11, wherein $L_1$ is $-CH_2CH_2(OCH_2CH_2)_n-$ wherein n is an integer of 1 to 5.

**15.** The single-stranded nucleic acid molecule according to claim 11, wherein $L_2$ is a $C_{2-11}$ alkylene chain.

**16.** The single-stranded nucleic acid molecule according to claim 11, wherein $L_2$ is $-CH_2CH_2(OCH_2CH_2)_n-$ wherein n is an integer of 1 to 5.

**17.** The single-stranded nucleic acid molecule according to any of claims 11 to 16, which comprises a sequence that inhibits expression of a target gene.

**18.** A pharmaceutical composition comprising the single-stranded nucleic acid molecule according to any of claims 11 to 17.

**19.** An inhibitor of expression of a target gene, comprising the single-stranded nucleic acid molecule according to claim 17.

**20.** A compound represented by the following general formula (IVa) :

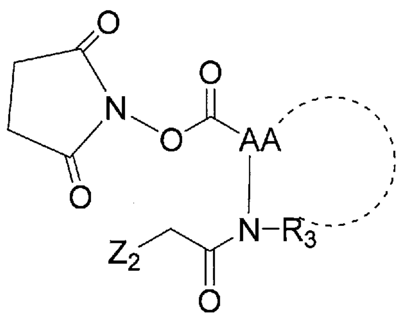

(IVa)

wherein

$Z_2$ is a halogen atom;
-C(=O)-AA-$NR_3$- is an amino acid residue; and
$R_3$ is a hydrogen atom or a $C_{1-6}$ alkyl group, or $R_3$ is bonded to AA to form a nitrogen-containing heterocycle.

**21.** The compound according to claim 20, wherein the amino acid residue is a proline residue, a glycine residue, or a lysine residue with the side chain amino group being optionally protected.

**22.** The compound according to claim 20, wherein $Z_2$ is a chlorine atom or a bromine atom.

# Fig.1

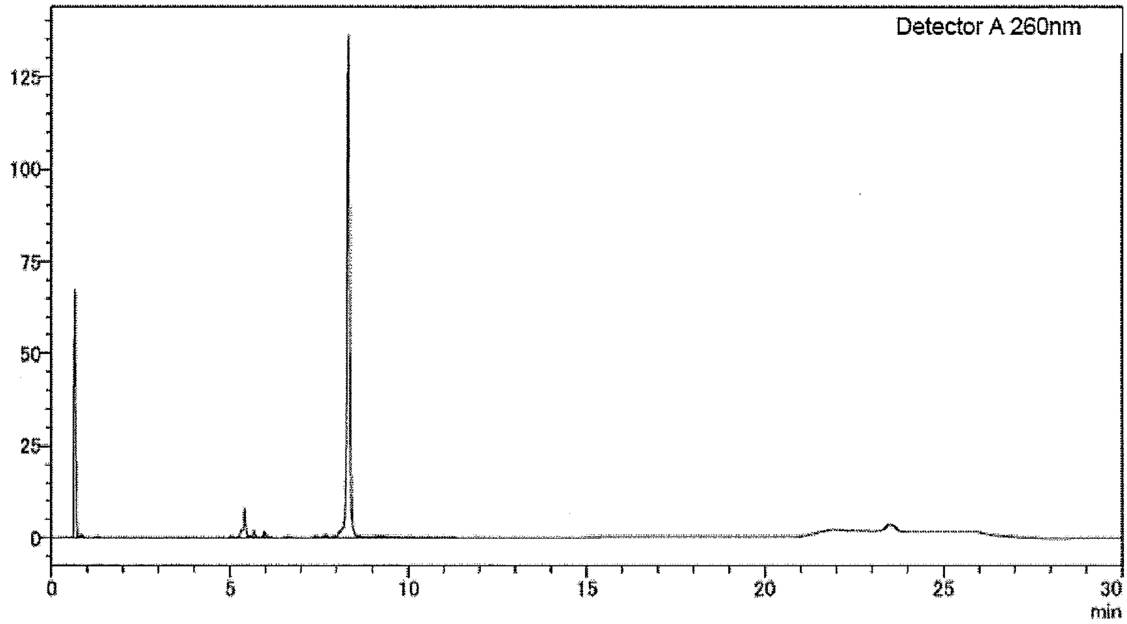


Fig.1 HPLC chart of sense strand + P

# Fig.2

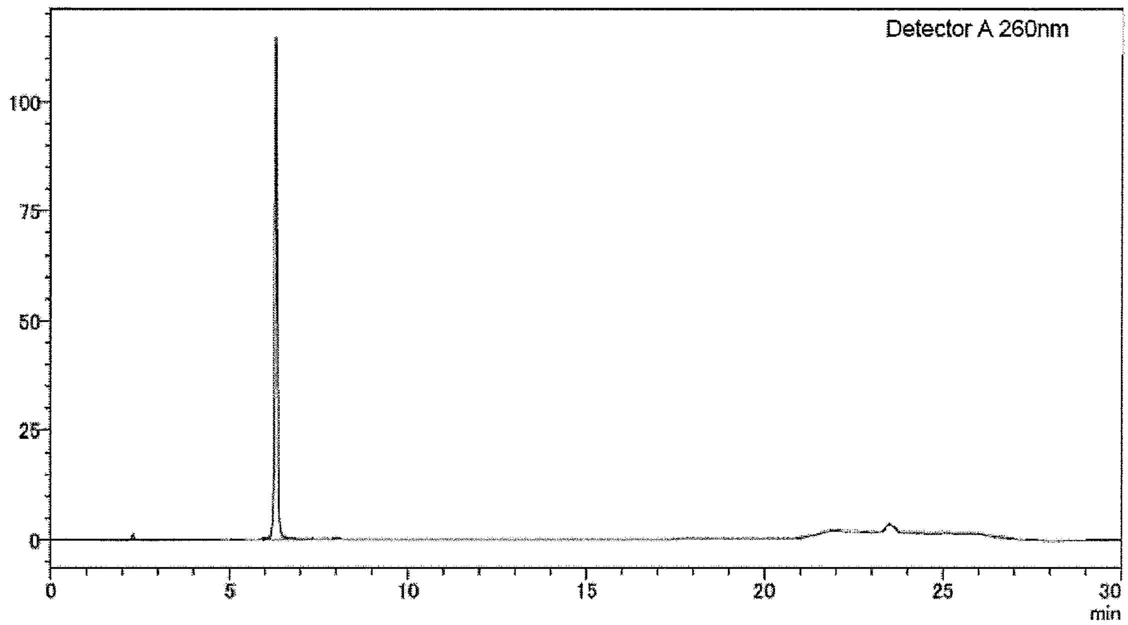


Fig.2 HPLC chart of antisense strand (SH form)

# Fig.3

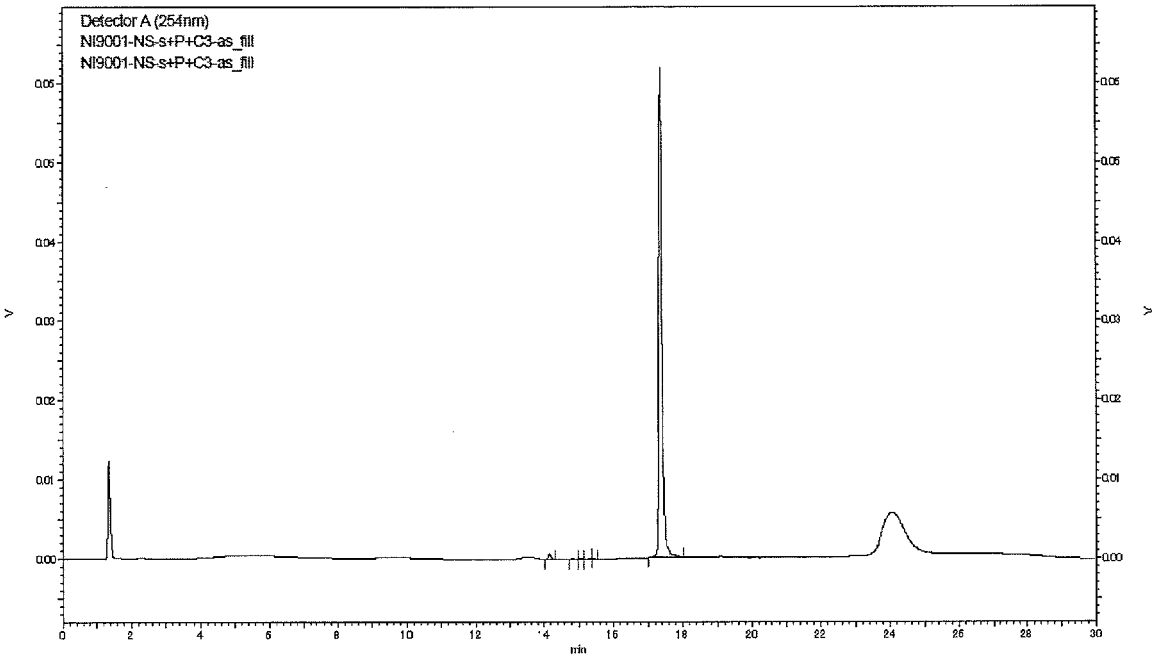


Fig.3 HPLC chart of PS-0001-C3

# Fig.4

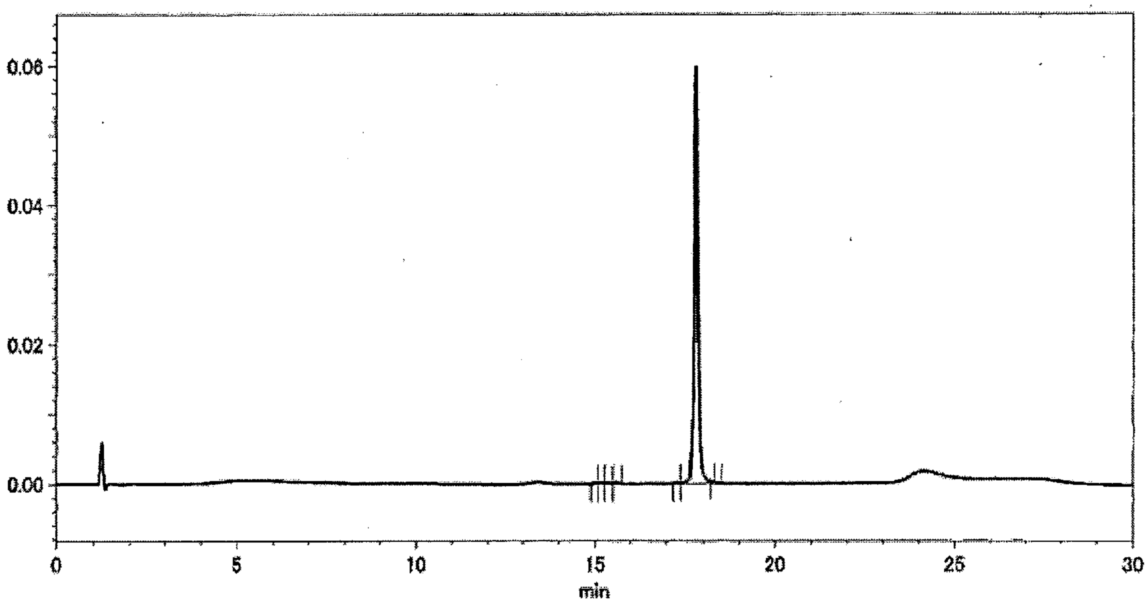


Fig.4 HPLC chart of KS-0001

# Fig.5

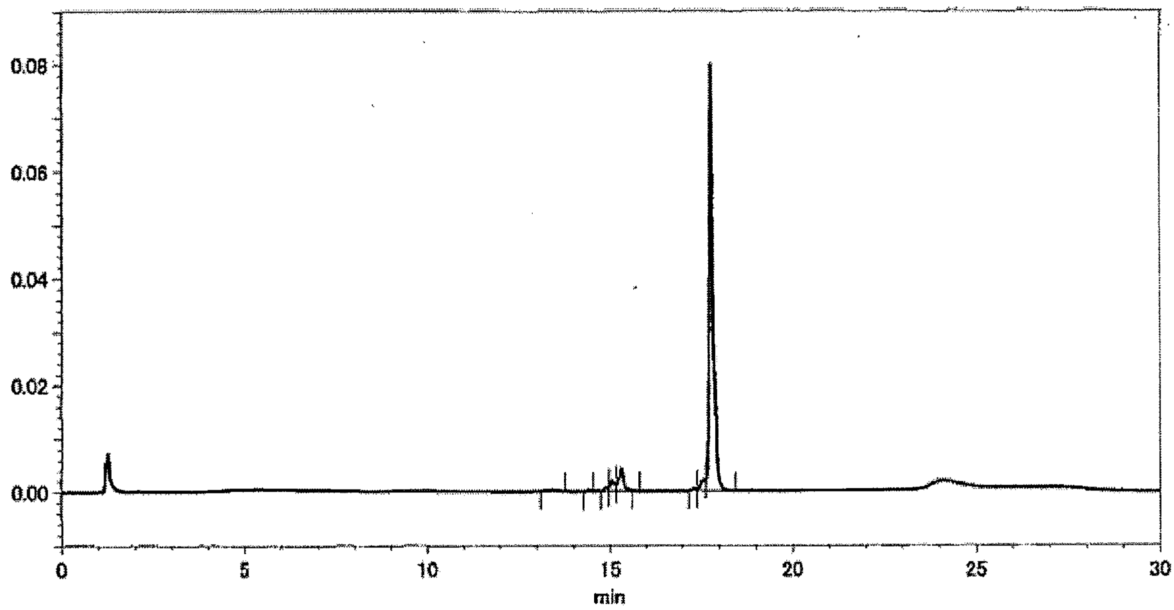


Fig.5 HPLC chart of AS-0001

# Fig.6

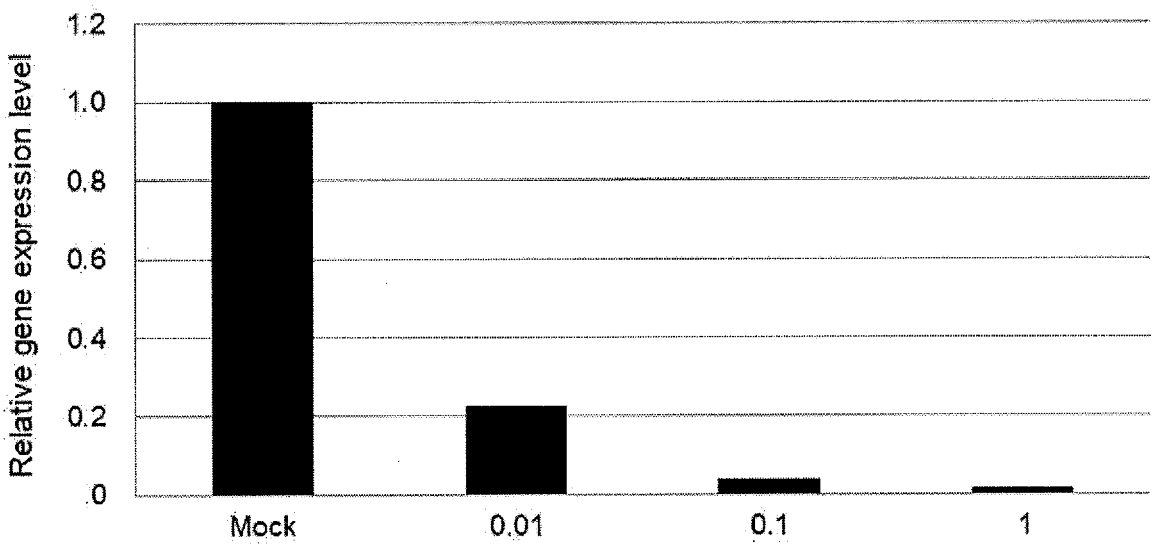

Fig.7

(A)

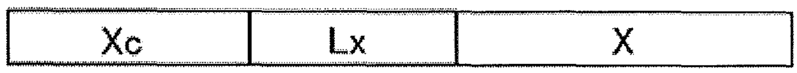


(B)

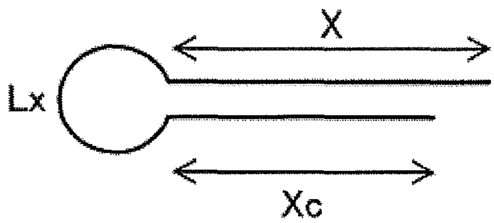


Fig.8

(A)

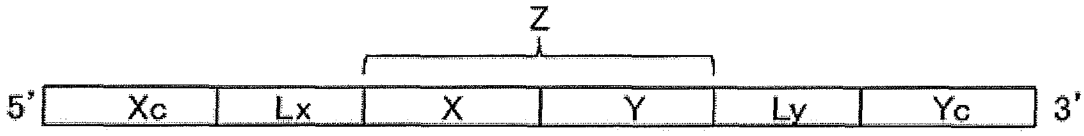


(B)

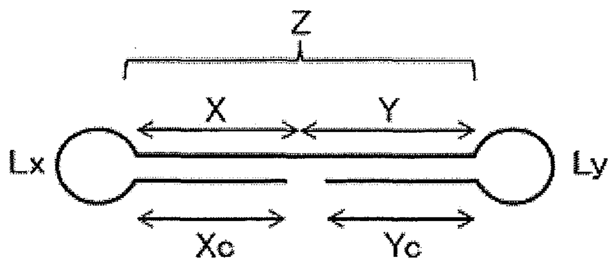

# Fig.9

(A)

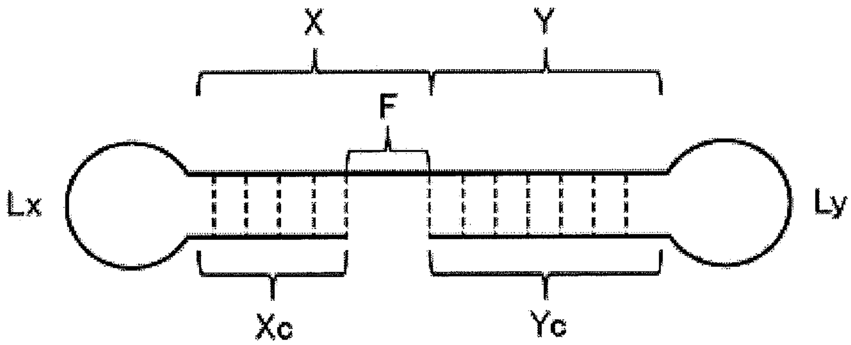


(B)

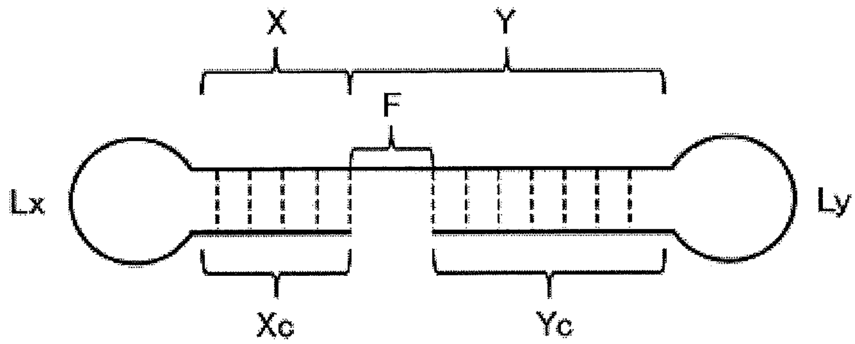


(C)

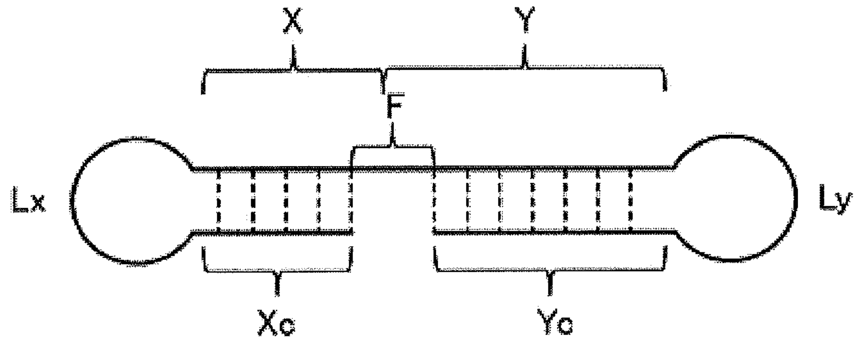


(D)

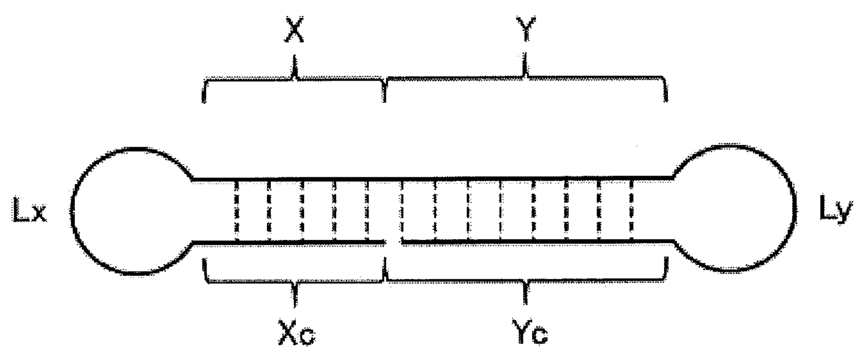

## INTERNATIONAL SEARCH REPORT

International application No. PCT/JP2018/038174

A. CLASSIFICATION OF SUBJECT MATTER
Int.Cl. C12N15/113(2010.01)i, A61K31/7088(2006.01)i, A61K48/00(2006.01)i

According to International Patent Classification (IPC) or to both national classification and IPC

B. FIELDS SEARCHED

Minimum documentation searched (classification system followed by classification symbols)
Int.Cl. C12N15/00-15/90, C07H21/00-21/04, A61K31/7088, A61K48/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY/MEDLINE/EMBASE/BIOSIS/WPIDS (STN), JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X<br>Y | ZAITSU, Kiyoshi et al., "New Heterobifunctional Cross-Linking Reagents for Protein Conjugation, N-(Bromoacetamido-n-alkanoyloxy)succinimides", Chem. Pharm. Bull., 1987, vol. 35, pp. 1991-1997, abstract, page 1994, lines 48-49, page 1995, lines 12-13, charts 1, 2 | 20-22<br>1-19 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 December 2018 (20.12.2018) | 08 January 2019 (08.01.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/038174

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO 2016/100113 A2 (SIEMENS HEALTHCARE DIAGNOSTICS INC.) 23 June 2016, paragraph [0063], fig. 7, 8 & US 2017/0362579 A1 & EP 3234604 A2 & CN 107003304 A | 20-22<br>1-19 |
| X | US 2005/0130244 A1 (ZHENG, Yifeng et al.) 16 June 2005, paragraph [0389], fig. 4A & WO 2005/058864 A1 & EP 1701948 A1 | 20-22 |
| X | WO 2005/103697 A2 (DADE BEHRING INC.) 03 November 2005, paragraph [0089], fig. 12 & US 2005/0221405 A1 & EP 1756570 A2 | 20-22 |
| X | SUCHY, Mojmir et al., "Analogs of Eu3+ DOTAM-Gly-Phe-OH and Tm3+ DOTAM-Gly-Lys-OH: Synthesis and magnetic properties of potential PARACEST MRI contrast agents", Bioorg. Med. Chem., 2008, vol. 16, pp. 6156-6166, page 6161, left column, lines 36-38 | 20-22 |
| X | WO 2016/061562 A2 (KODIAK SCIENCES INC.) 21 April 2016, example 28 & JP 2017-536105 A & US 2016/0184445 A & EP 3207128 A2 & KR 10-2017-0086036 A & CN 107208076 A | 20-21 |
| Y | WO 2016/104775 A1 (BONAC CORPORATION) 30 June 2016, claims & EP 3239297 A1, claims & CN 107109415 A & KR 10-2017-0098914 A | 1-19 |
| Y | JP 2002-504096 A (HENRY M. JACKSON FOUNDATION FOR THE ADVANCEMENT OF MILITARY MEDICINE) 05 February 2002, claims, fig. 1 & US 6087328 A, claims, fig. 1A & WO 1998/047530 A2 & EP 977588 A2 | 1-19 |
| P,X | WO 2018/185131 A2 (NOVO NORDISK A/S) 11 October 2018, example 3.3 (Family: none) | 20-21 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012005368 A
- WO 2012017919 A
- WO 2013103146 A
- WO 2013077446 A
- WO 2013133393 A
- WO 2016108264 A
- WO 2016104775 A
- US 3687808 A
- US 46566503
- US 0407070 W
- US 6509323 B
- US 20030008818 A
- JP 2017199945 A


### Non-patent literature cited in the description


- **LIMBACH et al.** Summary: the modified nucleosides of RNA. *Nucleic Acids Res.,* 1994, vol. 22, 2183-2196
- **KROSCHWITZ J. I.** Concise Encyclopedia of Polymer Science and Engineering. John Wiley & Sons, 1990, 858-859
- **ENGLISCH et al.** Angewandte Chemie. International Edition, 1991, vol. 30, 613